# EUROPEAN PATENT APPLICATION

(11) **EP 4 060 038 A1**
(43) Date of publication of application: **21.09.2022**
(21) Application number: 20887397.6
(22) Date of filing: 13.11.2020
(51) Int. Cl.: C12N 15/09, A61K 35/76, A61K 38/46, A61K 48/00, A61P 35/00, A61P 37/06, C12N 5/0786, C12N 5/10, C12N 15/12, C12N 15/63

(54) **METHOD FOR INTRODUCING ANTIGEN-SPECIFIC RECEPTOR GENE INTO T CELL GENOME USING CYCLIC DNA**

(30) Priority: 14.11.2019 JP 2019206448
(71) Applicant: Hiroshima University, Higashi-Hiroshima-shi Hiroshima 739-8511 (JP); Repertoire Genesis Incorporation, Ibaraki-shi, Osaka 567-0085 (JP)
(72) Inventor: ICHINOHE, Tatsuo, Hiroshima-shi, Hiroshima 739-8511 (JP); YAMAMOTO, Takashi, Hiroshima-shi, Hiroshima 739-8511 (JP); SAKUMA, Tetsushi, Hiroshima-shi, Hiroshima 739-8511 (JP); HONJO, Yasuko, Hiroshima-shi, Hiroshima 739-8511 (JP); KAWASE, Takakazu, Hiroshima-shi, Hiroshima 739-8511 (JP); NISHIZAWA, Masatoshi, Hiroshima-shi, Hiroshima 739-8511 (JP); SUZUKI, Ryuji, Ibaraki-shi, Osaka 567-0085 (JP); MAGOORI, Kenta, Ibaraki-shi, Osaka 567-0085 (JP); SATO, Hiroyuki, Ibaraki-shi, Osaka 567-0085 (JP)
(74) Representative: Uexküll & Stolberg
(86) International application number: PCT/JP2020/042429
(87) International publication number: WO 2021/095847

(57) **Abstract**

The present disclosure provides a technique for introducing an antigen receptor-encoding nucleic acid into a definite site in a nucleic acid that is contained in an immune cell of a target organism. In the technique according to the present disclosure, a vector, which contains an antigen receptor-encoding nucleic acid, and a nuclease are introduced into an immune cell. The nuclease cleaves the vector and the genome sequence of the immune cell. The vector can be configured so that, when cleaved with the nuclease, a sequence, which is an antigen receptor-encoding sequence and which is introduced by microhomology-mediated end-joining (MMEJ) at the nuclease cleavage site of the genome sequence of the immune cell, is formed.

SEQUENCE LISTING

<110>  Hiroshima University
       Repertoire Genesis Incorporation

<120>  Method for introducing antigen-specific receptor gene into T cell
genome using circular DNA

<130>  EC010PCT

<160>  39

<170>  PatentIn version 3.5

<210>  1
<211>  6207
<212>  DNA
<213>  artificial sequence

<220>
<223>  PITCh_1G4 vector

<400>  1
tcgcgcgttt cggtgatgac ggtgaaaacc tctgacacat gcagctcccg gagacggtca      60

cagcttgtct gtaagcggat gccgggagca gacaagcccg tcagggcgcg tcagcgggtg     120

ttggcgggtg tcggggctgg cttaactatg cggcatcaga gcagattgta ctgagagtgc     180

accatatgcg gtgtgaaata ccgcacagat gcgtaaggag aaaataccgc atcaggcgcc     240

attcgccatt caggctgcgc aactgttggg aagggcgatc ggtgcgggcc tcttcgctat     300

tacgccagct ggcgaaaggg ggatgtgctg caaggcgatt aagttgggta acgccagggt     360

tttcccagtc acgacgttgt aaaacgacgg ccagtgagct agtgtaatac gactcactat     420

agggcgcggc cgcagaattc gagctcggta cccgggatct cgaggccaga tctgtctgtc     480

tgcctattca ccgattgaag attttgattc aatgtgtcac aaagtaagga ttcgatgcaa     540

tttcctcatt ttattaggaa aggacagtgg gagtggcacc ttccagggtc aaggaaggca     600

cgggggaggg gcaaacaaca gatggctggc aactagaagg cacagtcgag gctgattttt     660

acgtagcggc cgctcagctg ctccacagtc tcagggtcat cagcaggttg aagccggcca     720

ccttcagcag caggattctc agcacgatca ccagcaggtt ctggaagttc aggttggtgt     780

cggtctcgaa gctcttctcc accagcttca cgtcgcagct gctctcgggg ctggggaaga     840

aggtgtcctc ggggatgatg ctgttgttga aggcgttggc gcaggcgaag tcgctcttgt     900

tgctccaggc cacggcgctg ttgctcttga agtccatgct tctcatgtcc agcacgcact     960

tgtcggtgat gtacacgtcg ctgtccttgc tctggctcac gttggtctgg ctgtcgaagt    1020

cggtgaatag gcagacagac ttgtcactgg atttagagtc tctcagctgg tacacggcgg    1080

ggtcggggtt ctggatgtag gggtgcacga tcaggctggt gcctctgcca aaggtgggga    1140

tatagctgcc gccgctggtt ggtctcacgg cacacaggta ggtggcgcta tcgcctggct    1200

ggctggcggc gatatacagg gtgcttctgc cgctgctctt gtccaggctg gcgttcagtc 1260

tgccgctggt ctgctctctc tggctgctct ggatcagcag caggctggtc aggcccttgc 1320

cggggtcctg tctgaaccac tgcaggttgt agatggcgct gtcggtgaag ctgcagttca 1380

gcaccagatt ctcgccttca ggcacgctca gggcggcagg aatctgggtc acctcctgct 1440

tgctgctcac ccactgcagc tgcagccaca ggatcagcag gcccaagagg gtctccatag 1500

gtccagggtt ctcctccacg tctccagcct gcttcagcag gctgaagtta gtagctccgc 1560

ttccgaagtc ctttctcttc accatggcca tcagcaccag ggcgctcacc agcacggcgt 1620

acagggtggc cttgcccagc aggatctcgt acaggatggt ggcgctcagc acgccctgct 1680

ggtagctcac gctggtgaag ccgcagtcgg ctctgcccca ggcctcggcg ctcacgatct 1740

gggtcacggg cttggctctg tcctgggtcc actcgtcgtt ctcgctcagg ccgtagaact 1800

gcacctggca tctgaagtgg tttctggggt tctgccagaa ggtggcgctc actctcagtc 1860

tgctgctcag gcagtatctg ctgtcgttca gggcgggctg ctccttcagg ggctgggggt 1920

cggtgcacac gccgctgtgc acctccttgc cgttcaccca ccagctcagc tccacgtggt 1980

cggggaagaa gcctgtggcc aggcacacca gtgtggcctt ttgggtgtgg ctgatctcgg 2040

cctcgcttgg ctcaaacacg gccacctcag gagggaacac gttcttcagg tcctccagca 2100

cggtcagtct gctgccctcg ccgaagaaca gctcgccggt gttgcccacg tagctgctgg 2160

cgcaaaagta cacgctggtc tggcttgggg cggcagacag cagtctcagt gggaaatcct 2220

cggtggtgct tctgctcacg ttgtagccgt ttggcacctc gccctggtct gtgattccgg 2280

cgcccacgct gtagtggatc agtctcaggc ccatgccggg gtcctgtctg taccagctca 2340

tgtactcgtg gttcatgtcc tgggcgcact gcagggtcat gctctggccg gtcttcagca 2400

cctggaactt aggggtctgt gtcacgccgg cattcactgg tccagcccac agcagagaca 2460

gggcggcaca acacagcagg ccgatgctca tggtggcctc gaggccaaag ttgagcgttt 2520

attctgagct tctgcaaaaa gaacaagaag cttccctata gtgagtcgta ttaatttcga 2580

taagccagta agcagtgggt tctctagtta gccagagggc cgcttcacga cacctgaaat 2640

ggaagaaaaa aactttgaac cactgtctga ggcttgagaa tgaaccaaga tccaaactca 2700

aaaagggcaa attccaagga gaattacatc aagtgccaag ctggcctaac ttcagtctcc 2760

acccactcag tgtggggaaa ctccatcgca taaaacccct ccccccaacc taaagacgac 2820

gtactccaaa agctcgagaa ctaatcgagg tgcctggacg gcgcccggta ctccgtggag 2880

tcacatgaag cgacggctga ggacggaaag gcccttttcc tttgtgtggg tgactcaccc 2940

gcccgctctc ccgagcgccg cgtcctccat tttgagctcc ctgcagcagg gccgggaagc 3000

ggccatcttt ccgctcacgc aactggtgcc gaccgggcca gccttgccgc ccagggcggg 3060

gcgatacacg gcggcgcgag gccaggcacc agagcaggcc ggccagcttg agactacccc 3120

cgtccgattc tcggtggccg cgctcgcagg ccccgcctcg ccgaacatgt gcgctgggac 3180

gcacgggccc cgtcgccgcc cgcggcccca aaaaccgaaa taccagtgtg cagatcttgg 3240

cccgcattta caagactatc ttgccagaaa aaaagcgtcg cagcaggtca tcaaaaattt 3300

taaatggcta gagacttatc gaaagcagcg agacaggcgc gaaggtgcca ccagattcgc 3360

acgcggcggc cccagcgccc aggccaggcc tcaactcaag cacgaggcga aggggctcct 3420

taagcgcaag gcctcgaact ctcccaccca cttccaaccc gaagctcggg atcaagaatc 3480

acgtactgca gccaggggcg tggaagtaat tcaaggcacg caagggccat aacccgtaaa 3540

gaggccaggc ccgcgggaac cacacacggc acttacctgt gttctggcgg caaacccgtt 3600

gcgaaaaaga acgttcacgg cgactactgc acttatatac ggttctcccc caccctcggg 3660

aaaaaggcgg agccagtaca cgacatcact ttcccagttt accccgcgcc accttctcta 3720

ggcaccggtt caattgccga cccctccccc caacttctcg gggactgtgg gcgatgtgcg 3780

ctctgcccac tgacgggcac cggagcctca cgactagtca ataatcaatg tcaacgtctg 3840

tctgcctatt caccgattca aacaaatgtg ttaatgtgtc acaaagtaag gattctgtgg 3900

atccgctcta gagtcgacct gcaggcatgc aagcttgcgg ccgcgtattc tatagtgtca 3960

cctaaatagc atggcgtaat catggtcata gctgtttcct gtgtgaaatt gttatccgct 4020

cacaattcca cacaacatac gagccggaag cataaagtgt aaagcctggg gtgcctaatg 4080

agtgagctaa ctcacattaa ttgcgttgcg ctcactgccc gctttccagt cgggaaacct 4140

gtcgtgccag ctgcattaat gaatcggcca acgcgcgggg agaggcggtt tgcgtattgg 4200

gcgctcttcc gcttcctcgc tcactgactc gctgcgctcg gtcgttcggc tgcggcgagc 4260

ggtatcagct cactcaaagg cggtaatacg gttatccaca gaatcagggg ataacgcagg 4320

aaagaacatg tgagcaaaag gccagcaaaa ggccaggaac cgtaaaaagg ccgcgttgct 4380

ggcgtttttc cataggctcc gcccccctga cgagcatcac aaaaatcgac gctcaagtca 4440

gaggtggcga aacccgacag gactataaag ataccaggcg tttccccctg gaagctccct 4500

cgtgcgctct cctgttccga ccctgccgct taccggatac ctgtccgcct ttctcccttc 4560

gggaagcgtg gcgctttctc atagctcacg ctgtaggtat ctcagttcgg tgtaggtcgt 4620

tcgctccaag ctgggctgtg tgcacgaacc ccccgttcag cccgaccgct gcgccttatc 4680

cggtaactat cgtcttgagt ccaacccggt aagacacgac ttatcgccac tggcagcagc 4740

cactggtaac aggattagca gagcgaggta tgtaggcggt gctacagagt tcttgaagtg 4800

gtggcctaac tacggctaca ctagaagaac agtatttggt atctgcgctc tgctgaagcc 4860

agttaccttc ggaaaaagag ttggtagctc ttgatccggc aaacaaacca ccgctggtag 4920

cggtggtttt tttgtttgca agcagcagat tacgcgcaga aaaaaaggat ctcaagaaga 4980

tcctttgatc ttttctacgg ggtctgacgc tcagtggaac gaaaactcac gttaagggat 5040

tttggtcatg agattatcaa aaaggatctt cacctagatc cttttaaatt aaaaatgaag 5100

ttttaaatca atctaaagta tatatgttta aacttggtct gacagttacc aatgcttaat 5160

cagtgaggca cctatctcag cgatctgtct atttcgttca tccatagttg cctgactccc 5220

cgtcgtgtag ataactacga tacgggaggg cttaccatct ggccccagtg ctgcaatgat 5280

accgcgagac ccacgctcac cggctccaga tttatcagca ataaaccagc cagccggaag 5340

ggccgagcgc agaagtggtc ctgcaacttt atccgcctcc atccagtcta ttaattgttg 5400

ccgggaagct agagtaagta gttcgccagt taatagtttg cgcaacgttg ttgccattgc 5460

tacaggcatc gtggtgtcac gctcgtcgtt tggtatggct tcattcagct ccggttccca 5520

acgatcaagg cgagttacat gatcccccat gttgtgcaaa aaagcggtta gctccttcgg 5580

tcctccgatc gttgtcagaa gtaagttggc cgcagtgtta tcactcatgg ttatggcagc 5640

actgcataat tctcttactg tcatgccatc cgtaagatgc ttttctgtga ctggtgagta 5700

ctcaaccaag tcattctgag aatagtgtat gcggcgaccg agttgctctt gcccggcgtc 5760

aatacgggat aataccgcgc cacatagcag aactttaaaa gtgctcatca ttggaaaacg 5820

ttcttcgggg cgaaaactct caaggatctt accgctgttg agatccagtt cgatgtaacc 5880

cactcgtgca cccaactgat cttcagcatc ttttactttc accagcgttt ctgggtgagc 5940

aaaaacagga aggcaaaatg ccgcaaaaaa gggaataagg gcgacacgga aatgttgaat 6000

actcatactc ttcctttttc aatattatta taagcattta tcagggttat tgtctcatga 6060

gcggatacat atttgaatgt atttagaaaa ataaacaaat aggggttccg cgcacatttc 6120

cccgaaaagt gccacctgac gtctaagaaa ccattattat catgacatta acctataaaa 6180

ataggcgtat cacgaggccc tttcgtc 6207

<210> 2
<211> 6085
<212> DNA
<213> artificial sequence

<220>
<223> PITCh_1G4 modified vector

<400> 2
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgagct agtgtaatac gactcactat 420

agggcgcggc cgcagatctc gaggccagat ctgtctgtct gcctattcac cgattgaaga 480

ttttgattca atgtgtcaca aagtaaggag atgcaatttc ctcattttat taggaaagga 540

cagtgggagt ggcaccttcc agggtcaagg aaggcacggg ggaggggcaa acaacagatg 600

gctggcaact agaaggcaca gcccgggtac cgagctcggc tgctccacag tctcagggtc 660

atcagcaggt tgaagccggc caccttcagc agcaggattc tcagcacgat caccagcagg 720

ttctggaagt tcaggttggt gtcggtctcg aagctcttct ccaccagctt cacgtcgcag 780

ctgctctcgg ggctggggaa gaaggtgtcc tcggggatga tgctgttgtt gaaggcgttg 840

gcgcaggcga agtcgctctt gttgctccag gccacggcgc tgttgctctt gaagtccatg 900

cttctcatgt ccagcacgca cttgtcggtg atgtacacgt cgctgtcctt gctctggctc 960

acgttggtct ggctgtcgaa gtcggtgaat aggcagacag acttgtcact ggatttagag 1020

tctctcagct ggtacacggc ggggtcgggg ttctggatgt aggggtgcac gatcaggctg 1080

gtgcctctgc caaaggtggg gatatagctg ccgccgctgg ttggtctcac ggcacacagg 1140

taggtggcgc tatcgcctgg ctggctggcg gcgatataca gggtgcttct gccgctgctc 1200

ttgtccaggc tggcgttcag tctgccgctg gtctgctctc tctggctgct ctggatcagc 1260

agcaggctgg tcaggccctt gccggggtcc tgtctgaacc actgcaggtt gtagatggcg 1320

ctgtcggtga agctgcagtt cagcaccaga ttctcgcctt caggcacgct cagggcggca 1380

ggaatctggg tcacctcctg cttgctgctc acccactgca gctgcagcca caggatcagc 1440

aggcccaaga gggtctccat aggtccaggg ttctcctcca cgtctccagc ctgcttcagc 1500

aggctgaagt tagtagctcc gcttccgaag tcctttctct tcaccatggc catcagcacc 1560

agggcgctca ccagcacggc gtacagggtg gccttgccca gcaggatctc gtacaggatg 1620

gtggcgctca gcacgccctg ctggtagctc acgctggtga agccgcagtc ggctctgccc 1680

caggcctcgg cgctcacgat ctgggtcacg ggcttggctc tgtcctgggt ccactcgtcg 1740

ttctcgctca ggccgtagaa ctgcacctgg catctgaagt ggtttctggg gttctgccag 1800

aaggtggcgc tcactctcag tctgctgctc aggcagtatc tgctgtcgtt cagggcgggc 1860

tgctccttca ggggctgggg gtcggtgcac acgccgctgt gcacctcctt gccgttcacc 1920

caccagctca gctccacgtg gtcggggaag aagcctgtgg ccaggcacac cagtgtggcc 1980

ttttgggtgt ggctgatctc ggcctcgctt ggctcaaaca cggccacctc aggagggaac 2040

acgttcttca ggtcctccag cacggtcagt ctgctgccct cgccgaagaa cagctcgccg 2100

gtgttgccca cgtagctgct ggcgcaaaag tacacgctgg tctggcttgg ggcggcagac 2160

agcagtctca gtgggaaatc ctcggtggtg cttctgctca cgttgtagcc gtttggcacc 2220

tcgccctggt ctgtgattcc ggcgcccacg ctgtagtgga tcagtctcag gcccatgccg 2280

gggtcctgtc tgtaccagct catgtactcg tggttcatgt cctgggcgca ctgcagggtc 2340

atgctctggc cggtcttcag cacctggaac ttaggggtct gtgtcacgcc ggcattcact 2400

ggtccagccc acagcagaga cagggcggca caacacagca ggccgatgct catgaattcc 2460

caaagttgag cgtttattct gagcttctgc aaaaagaaca agtcacgaca cctgaaatgg 2520

aagaaaaaaa ctttgaacca ctgtctgagg cttgagaatg aaccaagatc caaactcaaa 2580

aagggcaaat tccaaggaga attacatcaa gtgccaagct ggcctaactt cagtctccac 2640

ccactcagtg tggggaaact ccatcgcata aaacccctcc ccccaaccta aagacgacgt 2700

actccaaaag ctcgagaact aatcgaggtg cctggacggc gcccggtact ccgtggagtc 2760

acatgaagcg acggctgagg acggaaaggc ccttttcctt tgtgtgggtg actcacccgc 2820

ccgctctccc gagcgccgcg tcctccattt tgagctccct gcagcagggc cgggaagcgg 2880

ccatctttcc gctcacgcaa ctggtgccga ccgggccagc cttgccgccc agggcggggc 2940

gatacacggc ggcgcgaggc caggcaccag agcaggccgg ccagcttgag actacccccg 3000

tccgattctc ggtggccgcg ctcgcaggcc ccgcctcgcc gaacatgtgc gctgggacgc 3060

acgggccccg tcgccgcccg cggccccaaa aaccgaaata ccagtgtgca gatcttggcc 3120

cgcatttaca agactatctt gccagaaaaa aagcgtcgca gcaggtcatc aaaaatttta 3180

aatggctaga gacttatcga aagcagcgag acaggcgcga aggtgccacc agattcgcac 3240

gcggcggccc cagcgcccag gccaggcctc aactcaagca cgaggcgaag gggctcctta 3300

agcgcaaggc ctcgaactct cccacccact tccaacccga agctcgggat caagaatcac 3360

gtactgcagc caggggcgtg gaagtaattc aaggcacgca agggccataa cccgtaaaga 3420

ggccaggccc gcgggaacca cacacggcac ttacctgtgt tctggcggca aacccgttgc 3480

gaaaaagaac gttcacggcg actactgcac ttatatacgg ttctccccca ccctcgggaa 3540

aaaggcggag ccagtacacg acatcacttt cccagtttac cccgcgccac cttctctagg 3600

caccggttca attgccgacc cctcccccca acttctcggg gactgtgggc gatgtgcgct 3660

ctgcccactg acgggcaccg gagcctcacg actagtcaat aatcaatgtc aacgtctgtc 3720

tgcctattca ccgattcaaa caaatgtgtt aatgtgtcac aaagtaagga ttctgtggat 3780

ccgctctaga gtcgacctgc aggcatgcaa gcttgcggcc gcgtattcta tagtgtcacc 3840

taaatagcat ggcgtaatca tggtcatagc tgtttcctgt gtgaaattgt tatccgctca 3900

caattccaca caacatacga gccggaagca taaagtgtaa agcctggggt gcctaatgag 3960

tgagctaact cacattaatt gcgttgcgct cactgcccgc tttccagtcg ggaaacctgt 4020

cgtgccagct gcattaatga atcggccaac gcgcggggag aggcggtttg cgtattgggc 4080

gctcttccgc ttcctcgctc actgactcgc tgcgctcggt cgttcggctg cggcgagcgg 4140

tatcagctca ctcaaaggcg gtaatacggt tatccacaga atcaggggat aacgcaggaa 4200

agaacatgtg agcaaaaggc cagcaaaagg ccaggaaccg taaaaaggcc gcgttgctgg 4260

cgtttttcca taggctccgc ccccctgacg agcatcacaa aaatcgacgc tcaagtcaga 4320

ggtggcgaaa cccgacagga ctataaagat accaggcgtt tccccctgga agctccctcg 4380

tgcgctctcc tgttccgacc ctgccgctta ccggatacct gtccgccttt ctcccttcgg 4440

gaagcgtggc gctttctcat agctcacgct gtaggtatct cagttcggtg taggtcgttc 4500

gctccaagct gggctgtgtg cacgaacccc ccgttcagcc cgaccgctgc gccttatccg 4560

gtaactatcg tcttgagtcc aacccggtaa gacacgactt atcgccactg gcagcagcca 4620

ctggtaacag gattagcaga gcgaggtatg taggcggtgc tacagagttc ttgaagtggt 4680

ggcctaacta cggctacact agaagaacag tatttggtat ctgcgctctg ctgaagccag 4740

ttaccttcgg aaaaagagtt ggtagctctt gatccggcaa acaaaccacc gctggtagcg 4800

gtggtttttt tgtttgcaag cagcagatta cgcgcagaaa aaaaggatct caagaagatc 4860

ctttgatctt ttctacgggg tctgacgctc agtggaacga aaactcacgt taagggattt 4920

tggtcatgag attatcaaaa aggatcttca cctagatcct tttaaattaa aaatgaagtt 4980

ttaaatcaat ctaaagtata tatgtttaaa cttggtctga cagttaccaa tgcttaatca 5040

gtgaggcacc tatctcagcg atctgtctat ttcgttcatc catagttgcc tgactccccg 5100

tcgtgtagat aactacgata cgggagggct taccatctgg ccccagtgct gcaatgatac 5160

cgcgagaccc acgctcaccg gctccagatt tatcagcaat aaaccagcca gccggaaggg 5220

ccgagcgcag aagtggtcct gcaactttat ccgcctccat ccagtctatt aattgttgcc 5280

gggaagctag agtaagtagt tcgccagtta atagtttgcg caacgttgtt gccattgcta 5340

caggcatcgt ggtgtcacgc tcgtcgtttg gtatggcttc attcagctcc ggttcccaac 5400

gatcaaggcg agttacatga tcccccatgt tgtgcaaaaa agcggttagc tccttcggtc 5460

ctccgatcgt tgtcagaagt aagttggccg cagtgttatc actcatggtt atggcagcac 5520

tgcataattc tcttactgtc atgccatccg taagatgctt ttctgtgact ggtgagtact 5580

caaccaagtc attctgagaa tagtgtatgc ggcgaccgag ttgctcttgc ccggcgtcaa 5640

tacgggataa taccgcgcca catagcagaa ctttaaaagt gctcatcatt ggaaaacgtt 5700

cttcggggcg aaaactctca aggatcttac cgctgttgag atccagttcg atgtaaccca 5760

ctcgtgcacc caactgatct tcagcatctt ttactttcac cagcgtttct gggtgagcaa 5820

aaacaggaag gcaaaatgcc gcaaaaaagg gaataagggc gacacggaaa tgttgaatac 5880

tcatactctt cctttttcaa tattattata agcatttatc agggttattg tctcatgagc 5940

ggatacatat ttgaatgtat ttagaaaaat aaacaaatag gggttccgcg cacatttccc 6000

cgaaaagtgc cacctgacgt ctaagaaacc attattatca tgacattaac ctataaaaat 6060

aggcgtatca cgaggccctt tcgtc 6085

<210> 3
<211> 6075
<212> DNA
<213> artificial sequence

<220>
<223> PITCh_NLV48 modified vector

<220>
<221> misc_feature
<222> (1427)..(1427)
<223> n is a, c, g, t or u

<220>
<221> misc_feature
<222> (1991)..(1991)
<223> n is a, c, g, t or u

<220>
<221> misc_feature
<222> (2021)..(2021)
<223> n is a, c, g, t or u

<400> 3
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgagct agtgtaatac gactcactat 420

agggcgcggc cgcagatctc gaggccagat ctgtctgtct gcctattcac cgattgaaga 480

ttttgattca atgtgtcaca aagtaaggag atgcaatttc ctcattttat taggaaagga 540

cagtgggagt ggcaccttcc agggtcaagg aaggcacggg ggaggggcaa acaacagatg 600

gctggcaact agaaggcaca gcccgggtac cgagctcgca gctgctccac agtctcaggg 660

tcatcagcag gttgaagccg gccaccttca gcagcaggat tctcagcacg atcaccagca 720

ggttctggaa gttcaggttg gtgtcggtct cgaagctctt ctccaccagc ttcacgtcgc 780

agctgctctc ggggctgggg aagaaggtgt cctcggggat gatgctgttg ttgaaggcgt 840

tggcgcaggc gaagtcgctc ttgttgctcc aggccacggc gctgttgctc ttgaagtcca 900

tgcttctcat gtccagcacg cacttgtcgg tgatgtacac gtcgctgtcc ttgctctggc 960

tcacgttggt ctggctgtcg aagtcggtga ataggcagac agacttgtca ctggatttag 1020

agtctctcag ctggtacacg gcagggtcag ggttctggat atagggcagc acggacaatc 1080

tggttccggg accaaagaca aaattctgac cataatagtc agcacagaag tacaaagcgg 1140

agtcgctcac aagggcagat ggtttcttca ggtggaagga ggtttggctc ttgttaaatt 1200

cagcttcaaa gccatagctg cctttaacca ggttatcccc tgtgatgtat ttcagaagga 1260

actggaggcc tcggttgggg tattgaacat accaaaaaag ataaggtttt ccagagactg 1320

aataggtgca tttcacagtc agaggattcc cttcagcaac gttgacctga tcttccggct 1380

gagccactga ctgagctctc agcccactca atgtgaagag catcgcnagc atcgagatgg 1440

gtgcagaggc cataggtcca gggttctcct ccacgtctcc agcctgcttc agcaggctga 1500

agttagtagc tccgcttccg aagtcctttc tcttcaccat ggccatcagc accagggcgc 1560

tcaccagcac ggcgtacagg gtggccttgc ccagcaggat ctcgtacagg atggtggcgc 1620

tcagcacgcc ctgctggtag ctcacgctgg tgaagccgca gtcggctctg ccccaggcct 1680

cggcgctcac gatctgggtc acgggcttgg ctctgtcctg ggtccactcg tcgttctcgc 1740

tcaggccgta gaactgcacc tggcatctga agtggtttct ggggttctgc cagaaggtgg 1800

cgctcactct cagtctgctg ctcaggcagt atctgctgtc gttcagggcg ggctgctcct 1860

tcaggggctg ggggtcggtg cacacgccgc tgtgcacctc cttgccgttc acccaccagc 1920

tcagctccac gtggtcaggg aagaagcctg tggccaggca caccagtgtg gccttttggg 1980

tgtgggagat ntctgcttct gatggctcaa acacagcgac ntcgggtggg aacaccttgt 2040

tcaggtcctc tacaactgtg agtctggtgc cttgtccaaa gaaagcttca gtgaacccct 2100

ggaaactgct ggcacagagg tacatagatg tctggttggt gctggcggac tccagaatca 2160

gggagaagcg ctccttcttc tctctagaga cactgtaccc ctcaggaata tctccttttt 2220

ctttcatttt aacatcatat gagaaataga tccgccgtag ccccagacct gggtcttgtc 2280

gataccagaa catattttca tggtccatat cctggacaca ttccagaaaa actttctctc 2340

ccgtcctttt gactagatat ctcgagctct gggttacttt cacatctacg aggcctacag 2400

ccaggaaaca aaaggccaca cgacagagga gcctggtgcc catgaattcc caaagttgag 2460

cgtttattct gagcttctgc aaaaagaaca agtcacgaca cctgaaatgg aagaaaaaaa 2520

ctttgaacca ctgtctgagg cttgagaatg aaccaagatc caaactcaaa aagggcaaat 2580

tccaaggaga attacatcaa gtgccaagct ggcctaactt cagtctccac ccactcagtg 2640

tggggaaact ccatcgcata aaacccctcc ccccaaccta aagacgacgt actccaaaag 2700

ctcgagaact aatcgaggtg cctggacggc gcccggtact ccgtggagtc acatgaagcg 2760

acggctgagg acggaaaggc ccttttcctt tgtgtgggtg actcacccgc ccgctctccc 2820

gagcgccgcg tcctccattt tgagctccct gcagcagggc cgggaagcgg ccatctttcc 2880

gctcacgcaa ctggtgccga ccgggccagc cttgccgccc agggcggggc gatacacggc 2940

ggcgcgaggc caggcaccag agcaggccgg ccagcttgag actacccccg tccgattctc 3000

ggtggccgcg ctcgcaggcc ccgcctcgcc gaacatgtgc gctgggacgc acgggccccg 3060

tcgccgcccg cggccccaaa aaccgaaata ccagtgtgca gatcttggcc cgcatttaca 3120

agactatctt gccagaaaaa aagcgtcgca gcaggtcatc aaaaatttta aatggctaga 3180

gacttatcga aagcagcgag acaggcgcga aggtgccacc agattcgcac gcggcggccc 3240

cagcgcccag gccaggcctc aactcaagca cgaggcgaag gggctcctta agcgcaaggc 3300

ctcgaactct cccacccact tccaacccga agctcgggat caagaatcac gtactgcagc 3360

caggggcgtg gaagtaattc aaggcacgca agggccataa cccgtaaaga ggccaggccc 3420

gcgggaacca cacacggcac ttacctgtgt tctggcggca aacccgttgc gaaaaagaac 3480

gttcacggcg actactgcac ttatatacgg ttctccccca ccctcgggaa aaaggcggag 3540

ccagtacacg acatcacttt cccagtttac cccgcgccac cttctctagg caccggttca 3600

attgccgacc cctcccccca acttctcggg gactgtgggc gatgtgcgct ctgcccactg 3660

acgggcaccg gagcctcacg actagtcaat aatcaatgtc aacgtctgtc tgcctattca 3720

ccgattcaaa caaatgtgtt aatgtgtcac aaagtaagga ttctgtggat ccgctctaga 3780

gtcgacctgc aggcatgcaa gcttgcggcc gcgtattcta tagtgtcacc taaatagcat 3840

ggcgtaatca tggtcatagc tgtttcctgt gtgaaattgt tatccgctca caattccaca 3900

caacatacga gccggaagca taaagtgtaa agcctggggt gcctaatgag tgagctaact 3960

cacattaatt gcgttgcgct cactgcccgc tttccagtcg ggaaacctgt cgtgccagct 4020

gcattaatga atcggccaac gcgcggggag aggcggtttg cgtattgggc gctcttccgc 4080

ttcctcgctc actgactcgc tgcgctcggt cgttcggctg cggcgagcgg tatcagctca 4140

ctcaaaggcg gtaatacggt tatccacaga atcaggggat aacgcaggaa agaacatgtg 4200

agcaaaaggc cagcaaaagg ccaggaaccg taaaaaggcc gcgttgctgg cgtttttcca 4260

taggctccgc ccccctgacg agcatcacaa aaatcgacgc tcaagtcaga ggtggcgaaa 4320

cccgacagga ctataaagat accaggcgtt tccccctgga agctccctcg tgcgctctcc 4380

tgttccgacc ctgccgctta ccggatacct gtccgccttt ctcccttcgg gaagcgtggc 4440

gctttctcat agctcacgct gtaggtatct cagttcggtg taggtcgttc gctccaagct 4500

gggctgtgtg cacgaacccc ccgttcagcc cgaccgctgc gccttatccg gtaactatcg 4560

tcttgagtcc aacccggtaa gacacgactt atcgccactg gcagcagcca ctggtaacag 4620

gattagcaga gcgaggtatg taggcggtgc tacagagttc ttgaagtggt ggcctaacta 4680

cggctacact agaagaacag tatttggtat ctgcgctctg ctgaagccag ttaccttcgg 4740

aaaaagagtt ggtagctctt gatccggcaa acaaaccacc gctggtagcg gtggtttttt 4800

tgtttgcaag cagcagatta cgcgcagaaa aaaaggatct caagaagatc ctttgatctt 4860

ttctacgggg tctgacgctc agtggaacga aaactcacgt taagggattt tggtcatgag 4920

attatcaaaa aggatcttca cctagatcct tttaaattaa aaatgaagtt ttaaatcaat 4980

ctaaagtata tatgtttaaa cttggtctga cagttaccaa tgcttaatca gtgaggcacc 5040

tatctcagcg atctgtctat ttcgttcatc catagttgcc tgactccccg tcgtgtagat 5100

aactacgata cgggagggct taccatctgg ccccagtgct gcaatgatac cgcgagaccc 5160

acgctcaccg gctccagatt tatcagcaat aaaccagcca gccggaaggg ccgagcgcag 5220

aagtggtcct gcaactttat ccgcctccat ccagtctatt aattgttgcc gggaagctag 5280

agtaagtagt tcgccagtta atagtttgcg caacgttgtt gccattgcta caggcatcgt 5340

ggtgtcacgc tcgtcgtttg gtatggcttc attcagctcc ggttcccaac gatcaaggcg 5400

agttacatga tcccccatgt tgtgcaaaaa agcggttagc tccttcggtc ctccgatcgt 5460

tgtcagaagt aagttggccg cagtgttatc actcatggtt atggcagcac tgcataattc 5520

tcttactgtc atgccatccg taagatgctt ttctgtgact ggtgagtact caaccaagtc 5580

attctgagaa tagtgtatgc ggcgaccgag ttgctcttgc ccggcgtcaa tacgggataa 5640

taccgcgcca catagcagaa ctttaaaagt gctcatcatt ggaaaacgtt cttcggggcg 5700

aaaactctca aggatcttac cgctgttgag atccagttcg atgtaaccca ctcgtgcacc 5760

caactgatct tcagcatctt ttactttcac cagcgtttct gggtgagcaa aaacaggaag 5820

gcaaaatgcc gcaaaaaagg gaataagggc gacacggaaa tgttgaatac tcatactctt 5880

cctttttcaa tattattata agcatttatc agggttattg tctcatgagc ggatacatat 5940

ttgaatgtat ttagaaaaat aaacaaatag gggttccgcg cacatttccc cgaaaagtgc 6000

cacctgacgt ctaagaaacc attattatca tgacattaac ctataaaaat aggcgtatca 6060

cgaggccctt tcgtc 6075

<210> 4
<211> 477
<212> DNA
<213> artificial sequence

<220>
<223> PITCh_1G4 vector backbone

<400> 4
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgagct agtgtaatac gactcactat 420

agggcgcggc cgcagaattc gagctcggta cccgggatct cgaggccaga tctgtct 477

<210> 5
<211> 2312
<212> DNA
<213> artificial sequence

<220>
<223> PITCh_1G4 vector backbone

<400> 5
   tgtggatccg ctctagagtc gacctgcagg catgcaagct tgcggccgcg tattctatag 60
   tgtcacctaa atagcatggc gtaatcatgg tcatagctgt ttcctgtgtg aaattgttat 120
   ccgctcacaa ttccacacaa catacgagcc ggaagcataa agtgtaaagc ctggggtgcc 180
   taatgagtga gctaactcac attaattgcg ttgcgctcac tgcccgcttt ccagtcggga 240
   aacctgtcgt gccagctgca ttaatgaatc ggccaacgcg cggggagagg cggtttgcgt 300
   attgggcgct cttccgcttc ctcgctcact gactcgctgc gctcggtcgt tcggctgcgg 360
   cgagcggtat cagctcactc aaaggcggta atacggttat ccacagaatc aggggataac 420
   gcaggaaaga acatgtgagc aaaaggccag caaaaggcca ggaaccgtaa aaaggccgcg 480
   ttgctggcgt ttttccatag gctccgcccc cctgacgagc atcacaaaaa tcgacgctca 540
   agtcagaggt ggcgaaaccc gacaggacta taaagatacc aggcgtttcc ccctggaagc 600
   tccctcgtgc gctctcctgt tccgaccctg ccgcttaccg gatacctgtc cgcctttctc 660
   ccttcgggaa gcgtggcgct ttctcatagc tcacgctgta ggtatctcag ttcggtgtag 720
   gtcgttcgct ccaagctggg ctgtgtgcac gaaccccccg ttcagcccga ccgctgcgcc 780
   ttatccggta actatcgtct tgagtccaac ccggtaagac acgacttatc gccactggca 840
   gcagccactg gtaacaggat tagcagagcg aggtatgtag gcggtgctac agagttcttg 900
   aagtggtggc ctaactacgg ctacactaga agaacagtat ttggtatctg cgctctgctg 960
   aagccagtta ccttcggaaa aagagttggt agctcttgat ccggcaaaca aaccaccgct 1020
   ggtagcggtg gtttttttgt ttgcaagcag cagattacgc gcagaaaaaa aggatctcaa 1080
   gaagatcctt tgatcttttc tacggggtct gacgctcagt ggaacgaaaa ctcacgttaa 1140
   gggattttgg tcatgagatt atcaaaaagg atcttcacct agatcctttt aaattaaaaa 1200
   tgaagtttta aatcaatcta aagtatatat gtttaaactt ggtctgacag ttaccaatgc 1260
   ttaatcagtg aggcacctat ctcagcgatc tgtctatttc gttcatccat agttgcctga 1320
   ctccccgtcg tgtagataac tacgatacgg gagggcttac catctggccc cagtgctgca 1380
   atgataccgc gagacccacg ctcaccggct ccagatttat cagcaataaa ccagccagcc 1440
   ggaagggccg agcgcagaag tggtcctgca actttatccg cctccatcca gtctattaat 1500
   tgttgccggg aagctagagt aagtagttcg ccagttaata gtttgcgcaa cgttgttgcc 1560
   attgctacag gcatcgtggt gtcacgctcg tcgtttggta tggcttcatt cagctccggt 1620
   tcccaacgat caaggcgagt tacatgatcc cccatgttgt gcaaaaaagc ggttagctcc 1680
   ttcggtcctc cgatcgttgt cagaagtaag ttggccgcag tgttatcact catggttatg 1740
   gcagcactgc ataattctct tactgtcatg ccatccgtaa gatgcttttc tgtgactggt 1800
   gagtactcaa ccaagtcatt ctgagaatag tgtatgcggc gaccgagttg ctcttgcccg 1860
   gcgtcaatac gggataatac cgcgccacat agcagaactt taaaagtgct catcattgga 1920
   aaacgttctt cggggcgaaa actctcaagg atcttaccgc tgttgagatc cagttcgatg 1980
   taacccactc gtgcacccaa ctgatcttca gcatctttta ctttcaccag cgtttctggg 2040
   tgagcaaaaa caggaaggca aaatgccgca aaaaagggaa taagggcgac acggaaatgt 2100
   tgaatactca tactcttcct ttttcaatat tattataagc atttatcagg gttattgtct 2160
   catgagcgga tacatatttg aatgtattta gaaaaataaa caaatagggg ttccgcgcac 2220
   atttccccga aaagtgccac ctgacgtcta agaaaccatt attatcatga cattaaccta 2280
   taaaaatagg cgtatcacga ggccctttcg tc 2312

<210> 6
<211> 456
<212> DNA
<213> artificial sequence

<220>
<223> PITCh_1G4 modified vector backbone

<400> 6
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgagct agtgtaatac gactcactat 420

agggcgcggc cgcagatctc gaggccagat ctgtct 456

<210> 7
<211> 2312
<212> DNA

<213>  artificial sequence

<220>
<223>  PITCh_1G4 modified vector backbone

<400>  7
tgtggatccg ctctagagtc gacctgcagg catgcaagct tgcggccgcg tattctatag      60

tgtcacctaa atagcatggc gtaatcatgg tcatagctgt ttcctgtgtg aaattgttat     120

ccgctcacaa ttccacacaa catacgagcc ggaagcataa agtgtaaagc ctggggtgcc     180

taatgagtga gctaactcac attaattgcg ttgcgctcac tgcccgcttt ccagtcggga     240

aacctgtcgt gccagctgca ttaatgaatc ggccaacgcg cggggagagg cggtttgcgt     300

attgggcgct cttccgcttc ctcgctcact gactcgctgc gctcggtcgt tcggctgcgg     360

cgagcggtat cagctcactc aaaggcggta atacggttat ccacagaatc aggggataac     420

gcaggaaaga acatgtgagc aaaaggccag caaaaggcca ggaaccgtaa aaaggccgcg     480

ttgctggcgt ttttccatag gctccgcccc cctgacgagc atcacaaaaa tcgacgctca     540

agtcagaggt ggcgaaaccc gacaggacta taaagatacc aggcgtttcc ccctggaagc     600

tccctcgtgc gctctcctgt tccgaccctg ccgcttaccg gatacctgtc cgcctttctc     660

ccttcgggaa gcgtggcgct ttctcatagc tcacgctgta ggtatctcag ttcggtgtag     720

gtcgttcgct ccaagctggg ctgtgtgcac gaaccccccg ttcagcccga ccgctgcgcc     780

ttatccggta actatcgtct tgagtccaac ccggtaagac acgacttatc gccactggca     840

gcagccactg gtaacaggat tagcagagcg aggtatgtag gcggtgctac agagttcttg     900

aagtggtggc ctaactacgg ctacactaga agaacagtat ttggtatctg cgctctgctg     960

aagccagtta ccttcggaaa aagagttggt agctcttgat ccggcaaaca aaccaccgct    1020

ggtagcggtg gtttttttgt ttgcaagcag cagattacgc gcagaaaaaa aggatctcaa    1080

gaagatcctt tgatcttttc tacggggtct gacgctcagt ggaacgaaaa ctcacgttaa    1140

gggattttgg tcatgagatt atcaaaaagg atcttcacct agatcctttt aaattaaaaa    1200

tgaagtttta aatcaatcta aagtatatat gtttaaactt ggtctgacag ttaccaatgc    1260

ttaatcagtg aggcacctat ctcagcgatc tgtctatttc gttcatccat agttgcctga    1320

ctccccgtcg tgtagataac tacgatacgg gagggcttac catctggccc cagtgctgca    1380

atgataccgc gagacccacg ctcaccggct ccagatttat cagcaataaa ccagccagcc    1440

ggaagggccg agcgcagaag tggtcctgca actttatccg cctccatcca gtctattaat    1500

tgttgccggg aagctagagt aagtagttcg ccagttaata gtttgcgcaa cgttgttgcc    1560

attgctacag gcatcgtggt gtcacgctcg tcgtttggta tggcttcatt cagctccggt    1620

tcccaacgat caaggcgagt tacatgatcc cccatgttgt gcaaaaaagc ggttagctcc    1680

ttcggtcctc cgatcgttgt cagaagtaag ttggccgcag tgttatcact catggttatg    1740

gcagcactgc ataattctct tactgtcatg ccatccgtaa gatgcttttc tgtgactggt 1800

gagtactcaa ccaagtcatt ctgagaatag tgtatgcggc gaccgagttg ctcttgcccg 1860

gcgtcaatac gggataatac cgcgccacat agcagaactt taaaagtgct catcattgga 1920

aaacgttctt cggggcgaaa actctcaagg atcttaccgc tgttgagatc cagttcgatg 1980

taacccactc gtgcacccaa ctgatcttca gcatctttta ctttcaccag cgtttctggg 2040

tgagcaaaaa caggaaggca aaatgccgca aaaaagggaa taagggcgac acggaaatgt 2100

tgaatactca tactcttcct ttttcaatat tattataagc atttatcagg gttattgtct 2160

catgagcgga tacatatttg aatgtattta gaaaaataaa caaatagggg ttccgcgcac 2220

atttccccga aaagtgccac ctgacgtcta agaaaccatt attatcatga cattaaccta 2280

taaaaatagg cgtatcacga ggccctttcg tc 2312

<210> 8
<211> 456
<212> DNA
<213> artificial sequence

<220>
<223> PITCh_NLV48 modified vector backbone

<400> 8
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaatattac tacaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgagct agtgtaatac gactcactat 420

agggcgcggc cgcagatctc gaggccagat ctgtct 456

<210> 9
<211> 2312
<212> DNA
<213> artificial sequence

<220>
<223> PITCh_NLV48 modified vector backbone

<400> 9
tgtggatccg ctctagagtc gacctgcagg catgcaagct tgcggccgcg tattctatag 60

tgtcacctaa atagcatggc gtaatcatgg tcatagctgt ttcctgtgtg aaattgttat 120

ccgctcacaa ttccacacaa catacgagcc ggaagcataa agtgtaaagc ctggggtgcc 180

taatgagtga gctaactcac attaattgcg ttgcgctcac tgcccgcttt ccagtcggga 240

aacctgtcgt gccagctgca ttaatgaatc ggccaacgcg cggggagagg cggtttgcgt 300

attgggcgct cttccgcttc ctcgctcact gactcgctgc gctcggtcgt tcggctgcgg 360

cgagcggtat cagctcactc aaaggcggta atacggttat ccacagaatc aggggataac 420

gcaggaaaga acatgtgagc aaaaggccag caaaaggcca ggaaccgtaa aaaggccgcg 480

ttgctggcgt ttttccatag gctccgcccc cctgacgagc atcacaaaaa tcgacgctca 540

agtcagaggt ggcgaaaccc gacaggacta taaagatacc aggcgtttcc ccctggaagc 600

tccctcgtgc gctctcctgt tccgaccctg ccgcttaccg gatacctgtc cgcctttctc 660

ccttcgggaa gcgtggcgct ttctcatagc tcacgctgta ggtatctcag ttcggtgtag 720

gtcgttcgct ccaagctggg ctgtgtgcac gaaccccccg ttcagcccga ccgctgcgcc 780

ttatccggta actatcgtct tgagtccaac ccggtaagac acgacttatc gccactggca 840

gcagccactg gtaacaggat tagcagagcg aggtatgtag gcggtgctac agagttcttg 900

aagtggtggc ctaactacgg ctacactaga agaacagtat ttggtatctg cgctctgctg 960

aagccagtta ccttcggaaa aagagttggt agctcttgat ccggcaaaca aaccaccgct 1020

ggtagcggtg gtttttttgt ttgcaagcag cagattacgc gcagaaaaaa aggatctcaa 1080

gaagatcctt tgatcttttc tacggggtct gacgctcagt ggaacgaaaa ctcacgttaa 1140

gggattttgg tcatgagatt atcaaaaagg atcttcacct agatcctttt aaattaaaaa 1200

tgaagtttta aatcaatcta aagtatatat gtttaaactt ggtctgacag ttaccaatgc 1260

ttaatcagtg aggcacctat ctcagcgatc tgtctatttc gttcatccat agttgcctga 1320

ctccccgtcg tgtagataac tacgatacgg gagggcttac catctggccc cagtgctgca 1380

atgataccgc gagacccacg ctcaccggct ccagatttat cagcaataaa ccagccagcc 1440

ggaagggccg agcgcagaag tggtcctgca actttatccg cctccatcca gtctattaat 1500

tgttgccggg aagctagagt aagtagttcg ccagttaata gtttgcgcaa cgttgttgcc 1560

attgctacag gcatcgtggt gtcacgctcg tcgtttggta tggcttcatt cagctccggt 1620

tcccaacgat caaggcgagt tacatgatcc cccatgttgt gcaaaaaagc ggttagctcc 1680

ttcggtcctc cgatcgttgt cagaagtaag ttggccgcag tgttatcact catggttatg 1740

gcagcactgc ataattctct tactgtcatg ccatccgtaa gatgcttttc tgtgactggt 1800

gagtactcaa ccaagtcatt ctgagaatag tgtatgcggc gaccgagttg ctcttgcccg 1860

gcgtcaatac gggataatac cgcgccacat agcagaactt taaaagtgct catcattgga 1920

aaacgttctt cggggcgaaa actctcaagg atcttaccgc tgttgagatc cagttcgatg 1980

taacccactc gtgcacccaa ctgatcttca gcatctttta ctttcaccag cgtttctggg 2040

tgagcaaaaa caggaaggca aaatgccgca aaaaagggaa taagggcgac acggaaatgt 2100

tgaatactca tactcttcct ttttcaatat tattataagc atttatcagg gttattgtct 2160

catgagcgga tacatatttg aatgtattta gaaaaataaa caaatagggg ttccgcgcac 2220

atttccccga aaagtgccac ctgacgtcta agaaaccatt attatcatga cattaaccta 2280

taaaaatagg cgtatcacga ggccctttcg tc 2312

<210> 10
<211> 56
<212> DNA
<213> artificial sequence

<220>
<223> Left TALEN site

<400> 10
gtctgcctat tcaccgattg aagattttga ttcaatgtgt cacaaagtaa ggattc 56

<210> 11
<211> 61
<212> DNA
<213> artificial sequence

<220>
<223> Right TALEN site

<400> 11
cgtctgtctg cctattcacc gattcaaaca aatgtgttaa tgtgtcacaa agtaaggatt 60

c 61

<210> 12
<211> 53
<212> DNA
<213> artificial sequence

<220>
<223> Left TALEN site

<400> 12
gtctgcctat tcaccgattg aagattttga ttcaatgtgt cacaaagtaa gga 53

<210> 13
<211> 61
<212> DNA
<213> artificial sequence

<220>
<223> Right TALEN site

<400> 13
cgtctgtctg cctattcacc gattcaaaca aatgtgttaa tgtgtcacaa agtaaggatt 60

c 61

<210> 14

<211>  53
<212>  DNA
<213>  artificial sequence

<220>
<223>  Left TALEN site

<400>  14
gtctgcctat tcaccgattg aagattttga ttcaatgtgt cacaaagtaa gga          53


<210>  15
<211>  61
<212>  DNA
<213>  artificial sequence

<220>
<223>  Right TALEN site

<400>  15
cgtctgtctg cctattcacc gattcaaaca aatgtgttaa tgtgtcacaa agtaaggatt      60

c                                                                      61


<210>  16
<211>  3301
<212>  DNA
<213>  artificial sequence

<220>
<223>  PITCh_1G4 vector cassette

<400>  16
   gatgcaattt cctcatttta ttaggaaagg acagtgggag tggcaccttc cagggtcaag      60
   gaaggcacgg gggaggggca aacaacagat ggctggcaac tagaaggcac agtcgaggct     120
   gatttttacg tagcggccgc tcagctgctc cacagtctca gggtcatcag caggttgaag     180
   ccggccacct tcagcagcag gattctcagc acgatcacca gcaggttctg gaagttcagg     240
   ttggtgtcgg tctcgaagct cttctccacc agcttcacgt cgcagctgct ctcggggctg     300
   gggaagaagg tgtcctcggg gatgatgctg ttgttgaagg cgttggcgca ggcgaagtcg     360
   ctcttgttgc tccaggccac ggcgctgttg ctcttgaagt ccatgcttct catgtccagc     420
   acgcacttgt cggtgatgta cacgtcgctg tccttgctct ggctcacgtt ggtctggctg     480
   tcgaagtcgg tgaataggca gacagacttg tcactggatt tagagtctct cagctggtac     540
   acggcggggt cggggttctg gatgtagggg tgcacgatca ggctggtgcc tctgccaaag     600
   gtggggatat agctgccgcc gctggttggt ctcacggcac acaggtaggt ggcgctatcg     660
   cctggctggc tggcggcgat atacagggtg cttctgccgc tgctcttgtc caggctggcg     720
   ttcagtctgc cgctggtctg ctctctctgg ctgctctgga tcagcagcag gctggtcagg     780
   cccttgccgg ggtcctgtct gaaccactgc aggttgtaga tggcgctgtc ggtgaagctg     840
   cagttcagca ccagattctc gccttcaggc acgctcaggg cggcaggaat ctgggtcacc     900
   tcctgcttgc tgctcaccca ctgcagctgc agccacagga tcagcaggcc caagagggtc 960
   tccataggtc cagggttctc ctccacgtct ccagcctgct tcagcaggct gaagttagta 1020
   gctccgcttc cgaagtcctt tctcttcacc atggccatca gcaccagggc gctcaccagc 1080
   acggcgtaca gggtggcctt gcccagcagg atctcgtaca ggatggtggc gctcagcacg 1140
   ccctgctggt agctcacgct ggtgaagccg cagtcggctc tgccccaggc ctcggcgctc 1200
   acgatctggg tcacgggctt ggctctgtcc tgggtccact cgtcgttctc gctcaggccg 1260
   tagaactgca cctggcatct gaagtggttt ctggggttct gccagaaggt ggcgctcact 1320
   ctcagtctgc tgctcaggca gtatctgctg tcgttcaggg cgggctgctc cttcaggggc 1380
   tgggggtcgg tgcacacgcc gctgtgcacc tccttgccgt tcacccacca gctcagctcc 1440
   acgtggtcgg ggaagaagcc tgtggccagg cacaccagtg tggccttttg ggtgtggctg 1500
   atctcggcct cgcttggctc aaacacggcc acctcaggag ggaacacgtt cttcaggtcc 1560
   tccagcacgg tcagtctgct gccctcgccg aagaacagct cgccggtgtt gcccacgtag 1620
   ctgctggcgc aaaagtacac gctggtctgg cttggggcgg cagacagcag tctcagtggg 1680
   aaatcctcgg tggtgcttct gctcacgttg tagccgtttg gcacctcgcc ctggtctgtg 1740
   attccggcgc ccacgctgta gtggatcagt ctcaggccca tgccggggtc ctgtctgtac 1800
   cagctcatgt actcgtggtt catgtcctgg gcgcactgca gggtcatgct ctggccggtc 1860
   ttcagcacct ggaacttagg ggtctgtgtc acgccggcat tcactggtcc agcccacagc 1920
   agagacaggg cggcacaaca cagcaggccg atgctcatgg tggcctcgag gccaaagttg 1980
   agcgtttatt ctgagcttct gcaaaaagaa caagaagctt ccctatagtg agtcgtatta 2040
   atttcgataa gccagtaagc agtgggttct ctagttagcc agagggccgc ttcacgacac 2100
   ctgaaatgga agaaaaaaac tttgaaccac tgtctgaggc ttgagaatga accaagatcc 2160
   aaactcaaaa agggcaaatt ccaaggagaa ttacatcaag tgccaagctg gcctaacttc 2220
   agtctccacc cactcagtgt ggggaaactc catcgcataa aacccctccc cccaacctaa 2280
   agacgacgta ctccaaaagc tcgagaacta atcgaggtgc ctggacggcg cccggtactc 2340
   cgtggagtca catgaagcga cggctgagga cggaaaggcc cttttccttt gtgtgggtga 2400
   ctcacccgcc cgctctcccg agcgccgcgt cctccatttt gagctccctg cagcagggcc 2460
   gggaagcggc catctttccg ctcacgcaac tggtgccgac cgggccagcc ttgccgccca 2520
   gggcggggcg atacacggcg gcgcgaggcc aggcaccaga gcaggccggc cagcttgaga 2580
   ctacccccgt ccgattctcg gtggccgcgc tcgcaggccc cgcctcgccg aacatgtgcg 2640
   ctgggacgca cgggccccgt cgccgcccgc ggccccaaaa accgaaatac cagtgtgcag 2700
   atcttggccc gcatttacaa gactatcttg ccagaaaaaa agcgtcgcag caggtcatca 2760
   aaaattttaa atggctagag acttatcgaa agcagcgaga caggcgcgaa ggtgccacca 2820
   gattcgcacg cggcggcccc agcgcccagg ccaggcctca actcaagcac gaggcgaagg 2880
   ggctccttaa gcgcaaggcc tcgaactctc ccacccactt ccaacccgaa gctcgggatc 2940
   aagaatcacg tactgcagcc aggggcgtgg aagtaattca aggcacgcaa gggccataac 3000
   ccgtaaagag gccaggcccg cgggaaccac acacggcact tacctgtgtt ctggcggcaa 3060
   acccgttgcg aaaaagaacg ttcacggcga ctactgcact tatatacggt tctcccccac 3120
   cctcgggaaa aaggcggagc cagtacacga catcactttc ccagtttacc ccgcgccacc 3180
   ttctctaggc accggttcaa ttgccgaccc ctccccccaa cttctcgggg actgtgggcg 3240
   atgtgcgctc tgcccactga cgggcaccgg agcctcacga ctagtcaata atcaatgtca 3300
   a 3301

<210> 17
<211> 3203
<212> DNA
<213> artificial sequence

<220>
<223> PITCh_1G4 modified vector cassette

<400> 17
gatgcaattt cctcatttta ttaggaaagg acagtgggag tggcaccttc cagggtcaag 60

gaaggcacgg gggaggggca aacaacagat ggctggcaac tagaaggcac agcccgggta 120

ccgagctcgg ctgctccaca gtctcagggt catcagcagg ttgaagccgg ccaccttcag 180

cagcaggatt ctcagcacga tcaccagcag gttctggaag ttcaggttgg tgtcggtctc 240

gaagctcttc tccaccagct tcacgtcgca gctgctctcg ggctggggga agaaggtgtc 300

ctcggggatg atgctgttgt tgaaggcgtt ggcgcaggcg aagtcgctct tgttgctcca 360

ggccacggcg ctgttgctct tgaagtccat gcttctcatg tccagcacgc acttgtcggt 420

gatgtacacg tcgctgtcct tgctctggct cacgttggtc tggctgtcga agtcggtgaa 480

taggcagaca gacttgtcac tggatttaga gtctctcagc tggtacacgg cggggtcggg 540

gttctggatg taggggtgca cgatcaggct ggtgcctctg ccaaaggtgg ggatatagct 600

gccgccgctg gttggtctca cggcacacag gtaggtggcg ctatcgcctg gctggctggc 660

ggcgatatac agggtgcttc tgccgctgct cttgtccagg ctggcgttca gtctgccgct 720

ggtctgctct ctctggctgc tctggatcag cagcaggctg gtcaggccct tgccggggtc 780

ctgtctgaac cactgcaggt tgtagatggc gctgtcggtg aagctgcagt tcagcaccag 840

attctcgcct tcaggcacgc tcagggcggc aggaatctgg gtcacctcct gcttgctgct 900

cacccactgc agctgcagcc acaggatcag caggcccaag agggtctcca taggtccagg 960

gttctcctcc acgtctccag cctgcttcag caggctgaag ttagtagctc cgcttccgaa 1020

gtcctttctc ttcaccatgg ccatcagcac cagggcgctc accagcacgg cgtacagggt 1080

ggccttgccc agcaggatct cgtacaggat ggtggcgctc agcacgccct gctggtagct 1140

cacgctggtg aagccgcagt cggctctgcc ccaggcctcg gcgctcacga tctgggtcac 1200

gggcttggct ctgtcctggg tccactcgtc gttctcgctc aggccgtaga actgcacctg 1260

gcatctgaag tggtttctgg ggttctgcca gaaggtggcg ctcactctca gtctgctgct 1320

caggcagtat ctgctgtcgt tcagggcggg ctgctccttc aggggctggg ggtcggtgca 1380

cacgccgctg tgcacctcct tgccgttcac ccaccagctc agctccacgt ggtcggggaa 1440

gaagcctgtg gccaggcaca ccagtgtggc cttttgggtg tggctgatct cggcctcgct 1500

tggctcaaac acggccacct caggagggaa cacgttcttc aggtcctcca gcacggtcag 1560

tctgctgccc tcgccgaaga acagctcgcc ggtgttgccc acgtagctgc tggcgcaaaa 1620

gtacacgctg gtctggcttg gggcggcaga cagcagtctc agtgggaaat cctcggtggt 1680

gcttctgctc acgttgtagc cgtttggcac ctcgccctgg tctgtgattc cggcgcccac 1740

gctgtagtgg atcagtctca ggcccatgcc ggggtcctgt ctgtaccagc tcatgtactc 1800

gtggttcatg tcctgggcgc actgcagggt catgctctgg ccggtcttca gcacctggaa 1860

cttaggggtc tgtgtcacgc cggcattcac tggtccagcc cacagcagag acagggcggc 1920

acaacacagc aggccgatgc tcatgaattc ccaaagttga gcgtttattc tgagcttctg 1980

caaaaagaac aagtcacgac acctgaaatg gaagaaaaaa actttgaacc actgtctgag 2040

gcttgagaat gaaccaagat ccaaactcaa aaagggcaaa ttccaaggag aattacatca 2100

agtgccaagc tggcctaact tcagtctcca cccactcagt gtggggaaac tccatcgcat 2160

aaaacccctc cccccaacct aaagacgacg tactccaaaa gctcgagaac taatcgaggt 2220

gcctggacgg cgcccggtac tccgtggagt cacatgaagc gacggctgag gacggaaagg 2280

cccttttcct ttgtgtgggt gactcacccg cccgctctcc cgagcgccgc gtcctccatt 2340

ttgagctccc tgcagcaggg ccgggaagcg gccatctttc cgctcacgca actggtgccg 2400

accgggccag ccttgccgcc cagggcgggg cgatacacgg cggcgcgagg ccaggcacca 2460

gagcaggccg gccagcttga gactaccccc gtccgattct cggtggccgc gctcgcaggc 2520

cccgcctcgc cgaacatgtg cgctgggacg cacgggcccc gtcgccgccc gcggccccaa 2580

aaaccgaaat accagtgtgc agatcttggc ccgcatttac aagactatct tgccagaaaa 2640

aaagcgtcgc agcaggtcat caaaaatttt aaatggctag agacttatcg aaagcagcga 2700

gacaggcgcg aaggtgccac cagattcgca cgcggcggcc ccagcgccca ggccaggcct 2760

caactcaagc acgaggcgaa ggggctcctt aagcgcaagg cctcgaactc tcccacccac 2820

ttccaacccg aagctcggga tcaagaatca cgtactgcag ccaggggcgt ggaagtaatt 2880

caaggcacgc aagggccata acccgtaaag aggccaggcc cgcgggaacc acacacggca 2940

cttacctgtg ttctggcggc aaacccgttg cgaaaaagaa cgttcacggc gactactgca 3000

cttatatacg gttctccccc accctcggga aaaaggcgga gccagtacac gacatcactt 3060

tcccagttta ccccgcgcca ccttctctag gcaccggttc aattgccgac ccctcccccc 3120

aacttctcgg ggactgtggg cgatgtgcgc tctgcccact gacgggcacc ggagcctcac 3180

gactagtcaa taatcaatgt caa 3203

<210> 18
<211> 3193
<212> DNA
<213> artificial sequence

<220>
<223> PITCh_NLV48 modified vector cassette

<220>
<221> misc_feature
<222> (918)..(918)
<223> n is a, c, g, t or u

<220>
<221> misc_feature
<222> (1482)..(1482)
<223> n is a, c, g, t or u

<220>
<221> misc_feature
<222> (1512)..(1512)
<223> n is a, c, g, t or u

<400> 18
gatgcaattt cctcatttta ttaggaaagg acagtgggag tggcaccttc cagggtcaag 60

gaaggcacgg gggaggggca aacaacagat ggctggcaac tagaaggcac agcccgggta 120

ccgagctcgc agctgctcca cagtctcagg gtcatcagca ggttgaagcc ggccaccttc 180

agcagcagga ttctcagcac gatcaccagc aggttctgga agttcaggtt ggtgtcggtc 240

tcgaagctct tctccaccag cttcacgtcg cagctgctct cggggctggg gaagaaggtg 300

tcctcgggga tgatgctgtt gttgaaggcg ttggcgcagg cgaagtcgct cttgttgctc 360

caggccacgg cgctgttgct cttgaagtcc atgcttctca tgtccagcac gcacttgtcg 420

gtgatgtaca cgtcgctgtc cttgctctgg ctcacgttgg tctggctgtc gaagtcggtg 480

aataggcaga cagacttgtc actggattta gagtctctca gctggtacac ggcagggtca 540

gggttctgga tatagggcag cacggacaat ctggttccgg gaccaaagac aaaattctga 600

ccataatagt cagcacagaa gtacaaagcg gagtcgctca caagggcaga tggtttcttc 660

aggtggaagg aggtttggct cttgttaaat tcagcttcaa agccatagct gcctttaacc 720

aggttatccc ctgtgatgta tttcagaagg aactggaggc ctcggttggg gtattgaaca 780

taccaaaaaa gataagggtt tccagagact gaataggtgc atttcacagt cagaggattc 840

ccttcagcaa cgttgacctg atcttccggc tgagccactg actgagctct cagcccactc 900

aatgtgaaga gcatcgcnag catcgagatg ggtgcagagg ccataggtcc agggttctcc 960

tccacgtctc cagcctgctt cagcaggctg aagttagtag ctccgcttcc gaagtccttt 1020

ctcttcacca tggccatcag caccagggcg ctcaccagca cggcgtacag ggtggccttg 1080

cccagcagga tctcgtacag gatggtggcg ctcagcacgc cctgctggta gctcacgctg 1140

gtgaagccgc agtcggctct gccccaggcc tcggcgctca cgatctgggt cacgggcttg 1200

gctctgtcct gggtccactc gtcgttctcg ctcaggccgt agaactgcac ctggcatctg 1260

aagtggtttc tggggttctg ccagaaggtg gcgctcactc tcagtctgct gctcaggcag 1320

tatctgctgt cgttcagggc gggctgctcc ttcaggggct gggggtcggt gcacacgccg 1380

ctgtgcacct ccttgccgtt cacccaccag ctcagctcca cgtggtcagg gaagaagcct 1440

gtggccaggc acaccagtgt ggccttttgg gtgtgggaga tntctgcttc tgatggctca 1500

aacacagcga cntcgggtgg gaacaccttg ttcaggtcct ctacaactgt gagtctggtg 1560

ccttgtccaa agaaagcttc agtgaacccc tggaaactgc tggcacagag gtacatagat 1620

gtctggttgg tgctggcgga ctccagaatc agggagaagc gctccttctt ctctctagag 1680

acactgtacc cctcaggaat atctcctttt tctttcattt taacatcata tgagaaatag 1740

atccgccgta gccccagacc tgggtcttgt cgataccaga acatattttc atggtccata 1800

tcctggacac attccagaaa aactttctct cccgtccttt tgactagata tctcgagctc 1860

tgggttactt tcacatctac gaggcctaca gccaggaaac aaaaggccac acgacagagg 1920

agcctggtgc ccatgaattc ccaaagttga gcgtttattc tgagcttctg caaaaagaac 1980

aagtcacgac acctgaaatg gaagaaaaaa actttgaacc actgtctgag gcttgagaat 2040

gaaccaagat ccaaactcaa aaagggcaaa ttccaaggag aattacatca agtgccaagc 2100

tggcctaact tcagtctcca cccactcagt gtggggaaac tccatcgcat aaaacccctc 2160

cccccaacct aaagacgacg tactccaaaa gctcgagaac taatcgaggt gcctggacgg 2220

cgcccggtac tccgtggagt cacatgaagc gacggctgag gacggaaagg cccttttcct 2280

ttgtgtgggt gactcacccg cccgctctcc cgagcgccgc gtcctccatt ttgagctccc 2340

tgcagcaggg ccgggaagcg gccatctttc cgctcacgca actggtgccg accgggccag 2400

ccttgccgcc cagggcgggg cgatacacgg cggcgcgagg ccaggcacca gagcaggccg 2460

gccagcttga gactaccccc gtccgattct cggtggccgc gctcgcaggc cccgcctcgc 2520

cgaacatgtg cgctgggacg cacggccccc gtcgccgccc gcggccccaa aaaccgaaat 2580

accagtgtgc agatcttggc ccgcatttac aagactatct tgccagaaaa aaagcgtcgc 2640

agcaggtcat caaaaatttt aaatggctag agacttatcg aaagcagcga gacaggcgcg 2700

aaggtgccac cagattcgca cgcggcggcc ccagcgccca ggccaggcct caactcaagc 2760

acgaggcgaa ggggctcctt aagcgcaagg cctcgaactc tcccacccac ttccaacccg 2820

aagctcggga tcaagaatca cgtactgcag ccaggggcgt ggaagtaatt caaggcacgc 2880

aagggccata acccgtaaag aggccaggcc cgcgggaacc acacacggca cttacctgtg 2940

ttctggcggc aaacccgttg cgaaaaagaa cgttcacggc gactactgca cttatatacg 3000

gttctccccc accctcggga aaaaggcgga gccagtacac gacatcactt tcccagttta 3060

ccccgcgcca ccttctctag gcaccggttc aattgccgac ccctcccccc aacttctcgg 3120

ggactgtggg cgatgtgcgc tctgcccact gacgggcacc ggagcctcac gactagtcaa 3180

taatcaatgt caa 3193

<210> 19
<211> 112
<212> DNA
<213> artificial sequence

<220>
<223> bGH poly(A) signal

<400> 19
gatgcaattt cctcatttta ttaggaaagg acagtgggag tggcaccttc cagggtcaag 60

gaaggcacgg gggaggggca aacaacagat ggctggcaac tagaaggcac ag 112

<210> 20
<211> 58
<212> DNA
<213> artificial sequence

<220>
<223> P2A sequence

<400> 20
taggtccagg gttctcctcc acgtctccag cctgcttcag caggctgaag ttagtagc 58

<210> 21
<211> 43
<212> DNA
<213> artificial sequence

<220>
<223> Xenopus globin 5'-UTR

<400> 21
ccaaagttga gcgtttattc tgagcttctg caaaaagaac aag 43

<210> 22
<211> 19
<212> DNA
<213> artificial sequence

<220>
<223>  T7 promoter

<400>  22
cctatagtga gtcgtatta                                                    19


<210>  23
<211>  1182
<212>  DNA
<213>  artificial sequence

<220>
<223>  EF-1a promoter

<400>  23
tcacgacacc tgaaatggaa gaaaaaaact ttgaaccact gtctgaggct tgagaatgaa      60

ccaagatcca aactcaaaaa gggcaaattc caaggagaat tacatcaagt gccaagctgg     120

cctaacttca gtctccaccc actcagtgtg gggaaactcc atcgcataaa acccctcccc     180

ccaacctaaa gacgacgtac tccaaaagct cgagaactaa tcgaggtgcc tggacggcgc     240

ccggtactcc gtggagtcac atgaagcgac ggctgaggac ggaaaggccc ttttcctttg     300

tgtgggtgac tcacccgccc gctctcccga gcgccgcgtc ctccattttg agctccctgc     360

agcagggccg ggaagcggcc atctttccgc tcacgcaact ggtgccgacc gggccagcct     420

tgccgcccag ggcggggcga tacacggcgg cgcgaggcca ggcaccagag caggccggcc     480

agcttgagac tacccccgtc cgattctcgg tggccgcgct cgcaggcccc gcctcgccga     540

acatgtgcgc tgggacgcac gggccccgtc gccgcccgcg gccccaaaaa ccgaaatacc     600

agtgtgcaga tcttggcccg catttacaag actatcttgc cagaaaaaaa gcgtcgcagc     660

aggtcatcaa aaattttaaa tggctagaga cttatcgaaa gcagcgagac aggcgcgaag     720

gtgccaccag attcgcacgc ggcggcccca gcgcccaggc caggcctcaa ctcaagcacg     780

aggcgaaggg gctccttaag cgcaaggcct cgaactctcc cacccacttc caacccgaag     840

ctcgggatca agaatcacgt actgcagcca ggggcgtgga agtaattcaa ggcacgcaag     900

ggccataacc cgtaaagagg ccaggcccgc gggaaccaca cacggcactt acctgtgttc     960

tggcggcaaa cccgttgcga aaaagaacgt tcacggcgac tactgcactt atatacggtt    1020

ctcccccacc ctcgggaaaa aggcggagcc agtacacgac atcactttcc cagtttaccc    1080

cgcgccacct tctctaggca ccggttcaat tgccgacccc tccccccaac ttctcgggga    1140

ctgtgggcga tgtgcgctct gcccactgac gggcaccgga gc                        1182


<210>  24
<211>  26
<212>  DNA
<213>  artificial sequence

<220>
<223>  Primer sequence

<400>  24
ctctgcatga ctcactagca ctctat                                          26


<210>  25
<211>  20
<212>  DNA
<213>  artificial sequence

<220>
<223>  Primer sequence

<400>  25
agaccgacac caacctgaac                                                 20


<210>  26
<211>  20
<212>  DNA
<213>  artificial sequence

<220>
<223>  Primer sequence

<400>  26
tggctaggaa ggtggatgag                                                 20


<210>  27
<211>  20
<212>  DNA
<213>  artificial sequence

<220>
<223>  Primer sequence

<400>  27
atcgtgctga gaatcctgct                                                 20


<210>  28
<211>  20
<212>  DNA
<213>  artificial sequence

<220>
<223>  Primer sequence

<400>  28
cacggcgact actgcactta                                                 20


<210>  29
<211>  26
<212>  DNA
<213>  artificial sequence

<220>
<223>  Primer sequence

<400>  29
gactgactta gtgagctggg aaagat                                          26


<210>  30
<211>  20
<212>  DNA
<213>  artificial sequence

<220>
<223>  Primer sequence

<400>  30
ggagccagta cacgacatca                                                 20


<210>  31
<211>  20
<212>  DNA
<213>  artificial sequence

<220>
<223>  Primer sequence

<400>  31
tttggcagac agggagaaat                                                 20


<210>  32
<211>  18
<212>  DNA
<213>  Artificial Sequence

<220>
<223>  5' vector overlap sequence

<400>  32
taaacgctca actttggt                                                   18


<210>  33
<211>  933
<212>  DNA
<213>  Artificial Sequence

<220>
<223>  CMV specific TCR beta


<220>
<221>  misc_feature
<222>  (423)..(423)
<223>  n is a, c, g, or t

<220>
<221>  misc_feature
<222>  (453)..(453)
<223>  n is a, c, g, or t

<400>  33
atgggcacca ggctcctctg tcgtgtggcc ttttgtttcc tggctgtagg cctcgtagat      60

gtgaaagtaa cccagagctc gagatatcta gtcaaaagga cgggagagaa agtttttctg     120

gaatgtgtcc aggatatgga ccatgaaaat atgttctggt atcgacaaga cccaggtctg     180

gggctacggc ggatctattt ctcatatgat gttaaaatga aagaaaaagg agatattcct     240

gaggggtaca gtgtctctag agagaagaag gagcgcttct ccctgattct ggagtccgcc     300

agcaccaacc agacatctat gtacctctgt gccagcagtt tccaggggtt cactgaagct     360

ttctttggac aaggcaccag actcacagtt gtagaggacc tgaacaaggt gttcccaccc     420

gangtcgctg tgtttgagcc atcagaagca ganatctccc acacccaaaa ggccacactg     480

gtgtgcctgg ccacaggctt cttccctgac cacgtggagc tgagctggtg ggtgaacggc     540

aaggaggtgc acagcggcgt gtgcaccgac ccccagcccc tgaaggagca gcccgccctg     600

aacgacagca gatactgcct gagcagcaga ctgagagtga gcgccacctt ctggcagaac     660

cccagaaacc acttcagatg ccaggtgcag ttctacggcc tgagcgagaa cgacgagtgg     720

acccaggaca gagccaagcc cgtgacccag atcgtgagcg ccgaggcctg gggcagagcc     780

gactgcggct tcaccagcgt gagctaccag cagggcgtgc tgagcgccac catcctgtac     840

gagatcctgc tgggcaaggc caccctgtac gccgtgctgg tgagcgccct ggtgctgatg     900

gccatggtga agagaaagga cttcggaagc gga                                  933

<210>  34
<211>  66
<212>  DNA
<213>  Artificial Sequence

<220>
<223>  GSG P2A

<400>  34
ggaagcggag ctactaactt cagcctgctg aagcaggctg gagacgtgga ggagaaccct      60

ggacct                                                                 66

<210>  35
<211>  816
<212>  DNA
<213>  Artificial Sequence

<220>
<223>  CMV specific TCR alpha

<220>
<221>  misc_feature
<222>  (27)..(27)
<223>  n is a, c, g, or t

<400>  35
atggcctctg cacccatctc gatgctngcg atgctcttca cattgagtgg gctgagagct      60

cagtcagtgg ctcagccgga agatcaggtc aacgttgctg aagggaatcc tctgactgtg     120

aaatgcacct attcagtctc tggaaaccct tatctttttt ggtatgttca ataccccaac 180

cgaggcctcc agttccttct gaaatacatc acaggggata acctggttaa aggcagctat 240

ggctttgaag ctgaatttaa caagagccaa acctccttcc acctgaagaa accatctgcc 300

cttgtgagcg actccgcttt gtacttctgt gctgactatt atggtcagaa ttttgtcttt 360

ggtcccggaa ccagattgtc cgtgctgccc tatatccaga accctgaccc tgccgtgtac 420

cagctgagag actctaaatc cagtgacaag tctgtctgcc tattcaccga cttcgacagc 480

cagaccaacg tgagccagag caaggacagc gacgtgtaca tcaccgacaa gtgcgtgctg 540

gacatgagaa gcatggactt caagagcaac agcgccgtgg cctggagcaa caagagcgac 600

ttcgcctgcg ccaacgcctt caacaacagc atcatccccg aggacacctt cttccccagc 660

cccgagagca gctgcgacgt gaagctggtg gagaagagct tcgagaccga caccaacctg 720

aacttccaga acctgctggt gatcgtgctg agaatcctgc tgctgaaggt ggccggcttc 780

aacctgctga tgaccctgag actgtggagc agctga 816

<210> 36
<211> 18
<212> DNA
<213> Artificial Sequence

<220>
<223> 3′ vector overlap sequence

<400> 36
gcaactagaa ggcacagt 18

<210> 37
<211> 18
<212> DNA
<213> Artificial Sequence

<220>
<223> 5′ vector overlap sequence

<400> 37
taaacgctca actttggt 18

<210> 38
<211> 1530
<212> DNA
<213> Artificial Sequence

<220>
<223> human CD19 specific chimeric antigen receptor

<400> 38
atgacccaga ccacaagcag cctgtctgcc agcctgggcg atagagtgac catcagctgt 60

agagccagcc aggacatcag caagtacctg aactggtatc agcagaaacc cgacggcacc 120

gtgaagctgc tgatctacca caccagcaga ctgcacagcg gcgtgccaag cagattttct 180

ggcagcggct ctggcaccga ctacagcctg acaatcagca acctggaaca agaggatatc 240

gctacctact tctgccagca aggcaacacc ctgccttaca cctttggcgg aggcaccaag 300

ctggaaatca ccggctctac aagcggcagc ggcaaacctg gatctggcga gggatctacc 360

aagggcgaag tgaaactgca agagtctggc cctggactgg tggccccatc tcagtctctg 420

agcgtgacct gtacagtcag cggagtgtcc ctgcctgatt acggcgtgtc ctggatcaga 480

cagcctcctc ggaaaggcct ggaatggctg ggagtgatct ggggcagcga gacaacctac 540

tacaacagcg ccctgaagtc ccggctgacc atcatcaagg acaactccaa gagccaggtg 600

ttcctgaaga tgaacagcct gcagaccgac gacaccgcca tctactattg cgccaagcac 660

tactactacg gcggcagcta cgccatggat tattggggcc agggcaccag cgtgaccgtt 720

tcttctacca cgacgccagc gccgcgacca ccaacaccgg cgcccaccat cgcgtcgcag 780

cccctgtccc tgcgcccaga agcgtgccgg ccagcggcgg ggggcgcagt gcacacgagg 840

gggctggact tcgcctgtga tttctgggtg ctggtcgttg tgggcggcgt gctggcctgc 900

tacagcctgc tggtgacagt ggccttcatc atcttttggg tgaggagcaa gcggagcaga 960

ctgctgcaca gcgactacat gaacatgacc ccccggaggc ctggccccac ccggaagcac 1020

taccagccct acgcccctcc cagggatttc gccgcctacc ggagcaaacg gggcagaaag 1080

aaactcctgt atatattcaa acaaccattt atgagaccag tacaaactac tcaagaggaa 1140

gatggctgta gctgccgatt tccagaagaa gaagaaggag gatgtgaact gagagtgaag 1200

ttcagcagga gcgcagacgc ccccgcgtac aagcagggcc agaaccagct ctataacgag 1260

ctcaatctag gacgaagaga ggagtacgat gttttggaca agagacgtgg ccgggaccct 1320

gagatggggg gaaagccgag aaggaagaac cctcaggaag gcctgtacaa tgaactgcag 1380

aaagataaga tggcggaggc ctacagtgag attgggatga aaggcgagcg ccggaggggc 1440

aaggggcacg atggccttta ccagggtctc agtacagcca ccaaggacac ctacgacgcc 1500

cttcacatgc aggccctgcc ccctcgctaa 1530

<210> 39
<211> 18
<212> DNA
<213> Artificial Sequence

<220>
<223> 3′ vector overlap sequence

<400> 39
gcaactagaa ggcacagt 18

## Description

### [Technical Field]

The present disclosure relates to biotechnological manipulation of immune cells. The present disclosure particularly relates to a technology for introducing a desired antigen receptor into an immune cell.

### [Background Art]

Currently, development of chimeric antigen receptor (CAR) introduced T cells (CAR-T) and T cell receptor introduced T cells (TCR-T) is rapidly progressing as novel therapeutic means for malignant tumor. A viral vector or a transposon vector is used for the creation thereof. Such technologies are unable to identify a donor gene insertion region and thus, safety concerns remain. Meanwhile, a technology for creating genetically modified T cells against malignant tumor with a circular DNA vector has not been established. The viability ratio of cells upon introduction of a circular double stranded DNA vector using a conventional method is very low.

### [Summary of Invention]

### [Solution to Problem]

The present disclosure provides a technology that enables insertion of any antigen receptor (e.g., human T cell receptor (TCR) α chain and β chain genes or chimeric antigen receptor (CAR)) into a desired region on a genome of an immune cell by using a circular DNA vector in concomitant use with a nuclease. The present disclosure also provides a technology for creating a genome edited T cell for medical use having a desired antigen specific gene inserted therein by using said technology.

The present disclosure can also provide a circular double stranded DNA vector, which can achieve the following actions by cleaving a target region on a genome of a T cell with an artificial nuclease and accurately inserting a sequence between cleaved sequences upon DNA repair: 1) sustainably expressing a desired TCR complex comprising a TCRα chain and a TCRβ chain or a chimeric antigen receptor (CAR) in a cell introduced with the vector; and 2) substitution into any TCRα chain/TCRβ chain sequence or a chimeric antigen receptor (CAR) that is desired to be expressed only by processing with a specific restriction enzyme.

The present disclosure can provide a method of introducing a nucleic acid encoding an antigen receptor into a given site in a nucleic acid contained in an immune cell of a target organism, comprising the step of introducing a vector comprising a nucleic acid encoding the antigen receptor and a nuclease into the immune cell. The nuclease can cleave a vector and a genome sequence of an immune cell. The vector can be configured to produce, when cleaved by the nuclease, a sequence encoding the antigen receptor, which is introduced into a portion cleaved by the nuclease of the genome sequence of the immune cell by microhomology-mediated end joining (MMEJ). In one aspect of the present disclosure, a vector that enables introduction of a gene by MMEJ can be provided. In one aspect of the present disclosure, a nuclease that is suitable for use in such a technology can be provided.

Embodiments of the present disclosure include, for example, the following items.

### (Item 1)

A method of introducing a nucleic acid encoding an antigen receptor into a given site in a nucleic acid contained in an immune cell of a target organism, comprising the step of:
introducing a vector comprising the nucleic acid encoding the antigen receptor and a nuclease into the immune cell;
wherein the nuclease cleaves the vector and a genome sequence of the immune cell, and
wherein the vector is configured to produce, when cleaved by the nuclease, a sequence encoding the antigen receptor, which is introduced into a portion cleaved by the nuclease of the genome sequence of the immune cell by microhomology-mediated end joining (MMEJ).

### (Item 2)

The method of the preceding item, wherein the nuclease cleaves a locus of an endogenous antigen receptor of the immune cell.

### (Item 3)

The method of any of the preceding items, wherein knockout of an endogenous antigen receptor gene of the immune cell occurs concurrently with introduction of the antigen receptor.

### (Item 4)

The method of any of the preceding items, wherein the endogenous antigen receptor gene is a TRA gene.

### (Item 5)

The method of any of the preceding items, wherein the endogenous antigen receptor gene is a TRB gene.

### (Item 6)

The method of any of the preceding items, wherein the nucleic acid encoding the antigen receptor encodes an α chain and a β chain of the antigen receptor.

### (Item 7)

The method of any of the preceding items, wherein the introducing step is performed using electroporation.

### (Item 8)

The method of any of the preceding items, wherein the introducing step is performed between about 48 and about 72 hours after stimulating an immune cell with CD3/CD28 beads.

### (Item 9)

The method of any of the preceding items, wherein the antigen receptor is selected from the group consisting of a T cell receptor (TCR), a B cell receptor (BCR), and a chimeric antigen receptor (CAR).

### (Item 10)

The method of any of the preceding items, wherein the nuclease cleaves inside of a constant region of a TRA gene.

### (Item 11)

The method of any of the preceding items, wherein the nuclease cleaves inside of a constant region of a TRB gene.

### (Item 12)

The method of any of the preceding items, wherein the nuclease is a Cas9 nuclease.

### (Item 13)

The method of any of the preceding items, wherein the nuclease is a homodimer nuclease, and the vector is a circular vector.

### (Item 14)

The method of any of the preceding items, wherein the nuclease is a TALEN.

### (Item 15)

The method of any of the preceding items, wherein the nuclease is a Platinum TALEN.

### (Item 16)

The method of any of the preceding items, wherein
the nuclease comprises a DNA binding domain,
the DNA binding domain comprises a plurality of DNA binding modules consecutively from the N-terminus side,
   a combination of the xth amino acid and the yth amino acid in the 4n-3th DNA binding module from the N-terminus being identical for any n,
   a combination of the xth amino acid and the yth amino acid in the 4n-2th DNA binding module from the N-terminus being identical for any n,
   a combination of the xth amino acid and the yth amino acid in the 4n-1th DNA binding module from the N-terminus being identical for any n, and
   a combination of the xth amino acid and the yth amino acid in the 4nth DNA binding module from the N-terminus being identical for any n,
the combination of the xth amino acid and the yth amino acid in the 4n-3th DNA binding module from the N-terminus, the combination of the xth amino acid and the yth amino acid in the 4n-2th DNA binding module from the N-terminus, the combination of the xth amino acid and the yth amino acid in the 4n-1th DNA binding module from the N-terminus, and the combination of the xth amino acid and the yth amino acid in the 4nth DNA binding module from the N-terminus are different from one another, and
n is a natural number from 1 to 10, x is a natural number from 1 to 40, y is a natural number from 1 to 40, and x and y are different natural numbers.

### (Item 17)

The method of any of the preceding items, wherein
the vector produces a nucleic acid fragment comprising a region consisting of a first nucleotide sequence, the desired nucleic acid, and a region consisting of a second nucleotide sequence, in order, in a direction from the 5' end to the 3' end by the nuclease,
the nucleic acid contained in the cell comprises a region consisting of a first nucleotide sequence, the given site, and a region consisting of a second nucleotide sequence, in order, in a direction from the 5' end to the 3' end, and
the first nucleotide sequence in the nucleic acid contained in the cell and the first nucleotide sequence in the vector are linked by microhomology-mediated end joining, and the second nucleotide sequence in the nucleic acid contained in the cell and the second nucleotide sequence in the vector are linked by microhomology-mediated end joining, whereby the desired nucleic acid is inserted.

### (Item 18)

The method of any of the preceding items, wherein the vector comprises a plurality of introduction cassettes comprising the nucleic acid encoding the antigen receptor.

### (Item 19)

A vector for inserting a nucleic acid encoding an antigen receptor at a given site in a nucleic acid contained in a cell in combination with a nuclease, comprising:
an introduction cassette comprising the nucleic acid encoding the antigen receptor; and
a microhomology sequence for producing microhomology-mediated end joining (MMEJ) for introducing a sequence encoding the antigen receptor into a site of cleavage by the nuclease of a genome sequence of the cell;
wherein the vector produces a nucleic acid fragment comprising a region consisting of a first nucleotide sequence, the nucleic acid encoding the antigen receptor, and a region consisting of a second nucleotide sequence, in order, in a direction from the 5' end to the 3' end by the nuclease, and
the first nucleotide sequence in the nucleic acid contained in the cell and the first nucleotide sequence in the vector are linked by microhomology-mediated end joining, and the second nucleotide sequence in the nucleic acid contained in the cell and the second nucleotide sequence in the vector are linked by microhomology-mediated end joining, whereby the sequence encoding the antigen receptor is inserted.

### (Item 19A)

A vector for inserting a desired nucleic acid into a given site in a nucleic acid contained in a cell in combination with a nuclease, wherein
the nucleic acid contained in the cell comprises a region consisting of a first nucleotide sequence, the given site, and a region consisting of a second nucleotide sequence, in order, in a direction from the 5' end to the 3' end, the nuclease specifically cleave the given site contained in the cell,
the vector produces a nucleic acid fragment comprising a region consisting of a first nucleotide sequence, the desired nucleic acid, and a region consisting of a second nucleotide sequence, in order, in a direction from the 5' end to the 3' end by the nuclease,
the first nucleotide sequence in the nucleic acid contained in the cell and the first nucleotide sequence in the vector are linked by microhomology-mediated end joining, and the second nucleotide sequence in the nucleic acid contained in the cell and the second nucleotide sequence in the vector are linked by microhomology-mediated end joining, whereby the desired nucleic acid is inserted, and
the desired nucleic acid comprises a nucleic acid encoding an antigen receptor.

### (Item 19B)

The vector of the preceding item, comprising a feature of one or more of the preceding items.

### (Item 20)

The vector of any of the preceding items, wherein
the given site is present in an endogenous antigen receptor gene of the cell, and
the nucleic acid encoding the antigen receptor comprises a synonymous substitution in a sequence corresponding to a targeted site by the nuclease contained in the cell.

### (Item 21)

The vector of any of the preceding items, wherein the nucleic acid encoding the antigen receptor comprises an EF-1α promoter that is operably linked to the antigen receptor.

### (Item 22)

The vector of any of the preceding items, wherein the nucleic acid encoding the antigen receptor comprises a leader sequence for enhancing translation efficiency upstream of the antigen receptor.

### (Item 23)

The vector of any of the preceding items, wherein the leader sequence is a 5'-UTR sequence of a β globin gene.

### (Item 24)

The vector of any of the preceding items, wherein the leader sequence is a Xenopus globin 5'-UTR.

### (Item 25)

The vector of any of the preceding items, wherein the nucleic acid encoding the antigen receptor encodes an α chain and a β chain of the antigen receptor.

### (Item 26)

The vector of any of the preceding items, wherein the antigen receptor is selected from the group consisting of a T cell receptor (TCR), a B cell receptor (BCR), and a chimeric antigen receptor (CAR).

### (Item 27)

The vector of any of the preceding items, wherein the desired site is inside of a constant region of a TRA gene.

### (Item 28)

The vector of any of the preceding items, wherein the desired site is inside of a constant region of a TRB gene.

### (Item 29)

The vector of any of the preceding items, wherein the nuclease is a Cas9 nuclease.

### (Item 30)

The vector of any of the preceding items, wherein the nuclease is a homodimer nuclease, and the vector is a circular vector.

### (Item 31)

The vector of any of the preceding items, wherein the nuclease is a TALEN.

### (Item 32)

The vector of any of the preceding items, wherein the nuclease is a Platinum TALEN.

### (Item 33)

The vector of any of the preceding items, wherein
the nuclease comprises a DNA binding domain,
the DNA binding domain comprises a plurality of DNA binding modules consecutively from the N-terminus side,
   a combination of the xth amino acid and the yth amino acid in the 4n-3th DNA binding module from the N-terminus being identical for any n,
   a combination of the xth amino acid and the yth amino acid in the 4n-2th DNA binding module from the N-terminus being identical for any n,
   a combination of the xth amino acid and the yth amino acid in the 4n-1th DNA binding module from the N-terminus being identical for any n, and
   a combination of the xth amino acid and the yth amino acid in the 4nth DNA binding module from the N-terminus being identical for any n,
the combination of the xth amino acid and the yth amino acid in the 4n-3th DNA binding module from the N-terminus, the combination of the xth amino acid and the yth amino acid in the 4n-2th DNA binding module from the N-terminus, the combination of the xth amino acid and the yth amino acid in the 4n-1th DNA binding module from the N-terminus, and the combination of the xth amino acid and the yth amino acid in the 4nth DNA binding module from the N-terminus are different from one another, and
n is a natural number from 1 to 10, x is a natural number from 1 to 40, y is a natural number from 1 to 40, and x and y are different natural numbers.

### (Item 34)

The vector of any of the preceding items, wherein the vector comprises a nucleotide sequence recognized by a gRNA target sequence or a first DNA binding domain on the 5' side, a region consisting of a first nucleotide sequence, the desired nucleic acid, a second nucleotide sequence, and a region consisting of a nucleotide sequence recognized by a gRNA target sequence or a second DNA binding domain on the 3' side, in order, in a direction from the 5' end to the 3' end.

### (Item 35)

The vector of any of the preceding items, wherein the first nucleotide sequence is 3 to 10 bases long, and/or the second nucleotide sequence is 3 to 10 bases long.

### (Item 36)

The vector of any of the preceding items, wherein the vector comprises a plurality of introduction cassettes comprising the nucleic acid encoding the antigen receptor.

### (Item 37)

A vector comprising:
a nucleic acid of SEQ ID NO: 4 and a nucleic acid of SEQ ID NO: 5, a nucleic acid of SEQ ID NO: 6 and a nucleic acid of SEQ ID NO: 7, or a nucleic acid of SEQ ID NO: 8 and a nucleic acid of SEQ ID NO: 9,
a nucleic acid of SEQ ID NO: 10 and a nucleic acid of SEQ ID NO: 11, a nucleic acid of SEQ ID NO: 12 and a nucleic acid of SEQ ID NO: 13, or a nucleic acid of SEQ ID NO: 14 and a nucleic acid of SEQ ID NO: 15, and
an introduction cassette comprising a nucleic acid encoding an antigen receptor.

### (Item 37A)

The vector of the preceding item, comprising a feature of one or more of the preceding items.

### (Item 38)

The vector of any of the preceding items, wherein the introduction cassette comprises a sequence encoding an α chain of an antigen receptor and a sequence encoding a β chain of an antigen receptor.

### (Item 39)

The vector of any of the preceding items, wherein the introduction cassette comprises a polyA addition sequence, a leader sequence, and/or one or more promoter sequences.

### (Item 40)

The vector of any of the preceding items, wherein the introduction cassette comprises a P2A sequence between the sequence encoding an α chain of an antigen receptor and the sequence encoding a β chain of an antigen receptor.

### (Item 41)

The vector of any of the preceding items, wherein the vector comprises a plurality of introduction cassettes.

### (Item 42)

A kit for inserting a nucleic acid encoding an antigen receptor into a given site in a nucleic acid contained in a cell, comprising the vector of any of the preceding items and a vector for expressing the nuclease.

### (Item 42A)

The kit of the preceding item, comprising a feature of one or more of the preceding items.

### (Item 43)

A method of inserting a nucleic acid encoding an antigen receptor into a given site in a nucleic acid contained in an immune cell in vitro or in vivo (in vivo method of inserting into a non-human organism in one embodiment), comprising the step of introducing the vector of any of the preceding items and a vector for expressing the nuclease into an immune cell.

### (Item 43A)

The method of the preceding item, comprising a feature of one or more of the preceding items.

### (Item 44)

A method of manufacturing an immune cell that expresses an antigen receptor, comprising the step of inserting a nucleic acid encoding the antigen receptor into a given site in a nucleic acid contained in the immune cell by the method of any of the preceding items.

### (Item 45)

A method of administering immunotherapy to a subject, comprising the steps of:
(A) obtaining blood from the subject;
(B) knocking out a TRB of mononuclear cells of the blood (PBMC);
(C) concentrating the mononuclear cells for CD3 negative;
(D) knocking out a TRA of the mononuclear cells and introducing an antigen receptor (e.g., a T cell receptor (TCR), a B cell receptor (BCR), and a chimeric antigen receptor (CAR));
(E) concentrating the mononuclear cells for CD3 positive; and
(G) optionally subjecting the mononuclear cells to expansion culture.

### (Item 46)

A method of manufacturing a cell used in immunotherapy, comprising the steps of:
(A) knocking out a TRB of mononuclear cells of blood (PBMC) obtained from a subject;
(B) concentrating the mononuclear cells for CD3 negative;
(C) knocking out a TRA of the mononuclear cells and introducing an antigen receptor;
(D) concentrating the mononuclear cells for CD3 positive; and
(E) optionally subjecting the mononuclear cells to expansion culture.

### (Item 46A)

The method of the preceding item, comprising a feature of one or more of the preceding items.

### (Item 47)

The method of any of the preceding items, wherein the TRA and/or TRB is cleaved by using a Platinum TALEN.

### (Item 48)

The method of any of the preceding items, wherein the immunotherapy comprises TCR-T therapy or CAR-T therapy.

### (Item 49)

A composition for use in the method of any of the preceding items, comprising a Platinum TALEN.

### (Item 49A)

The composition of the preceding item, comprising a feature of one or more of the preceding items.

### (Item 50)

The method, vector, or composition of the preceding items, wherein the cell comprises a human cell.

The present disclosure is intended so that one or more of the features described above can be provided not only as the explicitly disclosed combinations, but also as other combinations thereof. Additional embodiments and advantages of the present disclosure are recognized by those skilled in the art by reading and understanding the following detailed description as needed.

### [Advantageous Effects of Invention]

The technology of the present disclosure enables modification of an immune cell without using a viral vector and particularly enables introduction of a desired antigen receptor. Since a master cell for stably producing a virus would not be required, the technology enables reduction in the cost of processing cells. The technology of the present disclosure can also introduce a gene to be introduced into an accurate position on a genome. If, for example, the same sequence of a paternal allele and a maternal allele is targeted, one or two copies of TCR genes can be introduced into a desired genome region per cell, resulting in a drastic improvement in safety. Further, an antigen receptor introduced by the technology of the present disclosure can be stably expressed for a long period of time.

### [Brief Description of Drawings]

[Figure 1] Figure 1 is a diagram showing results of introducing an NY-ESO-1 antigen specific TCR into a Jurkat cell (human T cell leukemia strain).
[Figure 2] Figure 2 is a diagram showing stable expression of a desired TCR introduced by the method of the present disclosure. The state of expression of a TCR of each cell at one month after transfection is shown.
[Figure 3] Figure 3 shows results of analyzing expressed TCR of a β null Jurkat cell introduced with 1G4 TCR and a Jurkat cell (control) when an antibody cocktail for Vβ chain repertoire analysis was added. It can be clearly understood from Figure 3 that the 1G4 expressing Jurkat cell created by the present method has lost expression of a Vβ8 chain endogenous prior to genome editing, and expresses a Vβ13.1 chain that matches 1G4-TCR.
[Figure 4] Figure 4 is a diagram showing results of TCR Vβ repertoire analysis in a β null Jurkat cell introduced with 1G4 TCR by the method of the present disclosure. Figure 4 shows that a β null Jurkat cell introduced with 1G4 TCR by the method of the present disclosure does not express a V chain other than a Vβ13.1 chain that matches 1G4-TCR, i.e., a novel Vβ chain derived from a non-reconstituted allele is not expressed after genome editing.
[Figure 5] Figure 5 is a diagram showing results of checking on-target insertion of a TCR sequence to be introduced into the genome.
[Figure 6] Figure 6 is a diagram showing an example of a process of immunotherapy using the method of the present disclosure.
[Figure 7] Figure 7 is a diagram showing an example of a configuration of the vector of the present disclosure.
[Figure 8] Figure 8 is a schematic diagram showing an example of inserting DNA components together and an example of inserting DNA components separately in two groups when inserting a DNA into the vector of the present disclosure.
[Figure 9] Figure 9 is a diagram showing an exemplary design of a DNA to be inserted of a chimeric antigen receptor (CAR) gene.
[Figure 10] Figure **10** is a diagram showing results of comparison between a conventional PITCh vector and an improved PITCh vector introduced with 1G4 in β null Jurkat cells.
[Figure 11] Figure **11** is a diagram showing results of checking introduction of 1G4 into β null Jurkat cells through electroporation by using an improved PITCh vector.
[Figure 12] Figure **12** is a diagram showing results of introducing 1G4 when using a human T cell.

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

### (1. Explanation of definitions and basic technologies)

The definitions of the terms and/or details of the basic technologies that are especially used herein are explained hereinafter as appropriate.

As used herein, "antigen receptor" refers to any molecule that specifically binds to a target antigen and performs signaling within a cell expressing the receptor. An antigen receptor may be a naturally-occurring or artificial molecule herein. Examples thereof include chimeric antigen receptors (CAR) in addition to T cell receptors (TCR) and B cell receptors (BCR).

As used herein, "T cell receptor (TCR)" refers to a receptor that is present in a T cell. A TCR is a heterodimer receptor molecule consisting of two TCR polypeptide chains. There are αβ TCRs expressed by normal T cells and γδ TCRs with a special function. α and β chain TCR molecules form a complex with a plurality of CD3 molecules (CD3ζ chain, CD3ε chain, CD3γ chain, and CD3δ chain), transmit an intracellular signal after antigen recognition, and initiate various immune responses. Endogenous antigens such as a viral antigen proliferated within a cell due to a viral infection or a cancer antigen derived from a cancer cell are presented as an antigen peptide on an MHC class I molecule. Further, an antigen derived from an exogenous microorganism is taken up by an antigen-presenting cell by endocytosis and processed, and then presented on an MHC class II molecule. Such antigens are recognized by TCRs expressed by each of CD8+ T cells and CD4+ T cells. It is also known that a costimulatory molecule such as a CD28, ICOS, or OX40 molecule is important for stimulation via a TCR molecule. For αβ TCRs, which are one of the primary objectives herein, a gene product of each of α and β is understood to express specificity by a unique combination.

As used herein, "chimeric antigen receptor (CAR)" refers to a modified receptor that imparts antigen specificity to immune system cells (e.g., T cell such as naive T cells, central memory T cells, effector memory T cells, or a combination thereof, NK cells, macrophage, etc.) A CAR can comprise, for example, an antigen-specific targeting region, an extracellular domain, a transmembrane domain, a costimulatory domain, and/or an intracellular signaling domain. CARs can include those that use a plurality (mostly two) of antigen specific targeting regions (bispecific CAR)

As used herein, "microhomology-mediated end joining (MMEJ)" refers to a mechanism for recognizing and repairing a short homologous sequence (microhomology) of 5 to 30 base pairs when a DNA double strand is cleaved in a cell of a eukaryote.

As used herein, "immune cell" includes lymphocytes, macrophages, dendritic cells, etc., referring to any effector cell that can damage a target cell. Examples thereof include CD8+ T cells, CD4+ T cells, NK cells, γδ T cells, NKT cells, B cells, bone marrow cells, and the like. These cells include genetically modified cells thereof such as viruses and cancer cell specific CD8+ T cells that are converted into iPS cells, naive CD8+ T cells with an antigen receptor (T cell antigen receptor: TCR) gene incorporated therein or a gene of a chimeric antibody (chimeric antigen receptor: CAR) that recognizes a cancer cell surface expressed molecule incorporated therein.

### (2. PITCh vector)

One aspect of the present disclosure can provide a vector for inserting a desired nucleic acid into a given site in a nucleic acid contained in a cell in combination with a nuclease. A desired nucleic acid can comprise a nucleic acid encoding an antigen receptor. In this regard, the nucleic acid contained in a cell can comprise a region consisting of a first nucleotide sequence, the given site, and a region consisting of a second nucleotide sequence, in order, in a direction from the 5' end to the 3' end, and the nuclease can specifically cleave a given site contained in a cell. The vector can produce a nucleic acid fragment comprising a region consisting of a first nucleotide sequence, a desired nucleic acid, and a region consisting of a second nucleotide sequence, in order, in a direction from the 5' end to the 3' end by a nuclease. In addition, the vector can be configured so that the first nucleotide sequence in the nucleic acid contained in the cell and the first nucleotide sequence in the vector are linked by microhomology-mediated end joining, and the second nucleotide sequence in the nucleic acid contained in the cell and the second nucleotide sequence in the vector are linked by microhomology-mediated end joining, whereby the desired nucleic acid is inserted. Such vectors that enable introduction of a desired nucleic acid via MMEJ in combination with a nuclease are described, for example, in Japanese Patent No. 5900942, which is incorporated herein by reference in its entirety. The vector of the present disclosure can have, for example, the feature described below.

The vectors used in the present disclosure can comprise a region consisting of a first nucleotide sequence, a desired nucleic acid to be inserted, and a region consisting of a second nucleotide sequence, in order, in a direction from the 5' end to the 3' end. In this regard, the vector can be used in combination with a nuclease, which specifically cleaves a portion consisting of a region consisting of a first nucleotide sequence and a region of a nucleic acid within a cell consisting of a second nucleotide sequence, sandwich a given site where a nucleic acid is intended to be inserted. The vector can be introduced into a cell, the nuclease can be acted with the cell to produce a cleavage in the given site in a nucleic acid within the cell while also allowing the nuclease to act on the vector to produce a nucleic acid fragment comprising a region consisting of a first nucleotide sequence, the desired nucleic acid, and a region consisting of second nucleotide sequence, in order, in a direction from the 5' end to the 3' end. This can link the first nucleotide sequence in the nucleic acid within the cell with the first nucleotide sequence in the vector by microhomology-mediated end joining (MMEJ), and link the second nucleotide sequence in the nucleic acid within the cell with the second nucleotide sequence in the vector by MMEJ within the cell. This enables a desired nucleic acid to be inserted accurately into a given site of a nucleic acid of a cell. Insertion is possible for nucleic acids with a relatively long chain of several kb or longer such as an antigen receptor gene sequence. A nuclease may specifically cleave a portion consisting of a region consisting of a first nucleotide sequence and a region consisting of a second nucleotide sequence contained in a nucleic acid within a cell before insertion, but a nucleic acid after linking can lose a part of said portion by insertion of the desired nucleic acid. This can lead to no more cleavage by a nuclease that is present within a cell and stable retention, and insertion of a desire nucleic acid at a high frequency.

Since such a vector and a method of using the same are for linking through microhomology-mediated end joining that functions in many cells, a desired nucleic acid can be inserted accurately at a high frequency even in cells in a development stage with low homologous recombination efficiency, so that the vector and the method can be applied to a broad range of cell types. The vector and method of using the same can be readily used because a vector for introducing a nuclease and a vector for inserting a nucleic acid can be simultaneously inserted into a cell. Furthermore, the vector and method of using the same can prevent repeated cleavage of a nucleic acid after insertion by a change in a portion of a nucleic acid within a cell through microhomology-mediated end joining. Thus, a highly active homodimer domain can be used as a DNA cleavage domain contained in a nuclease, such that the breadth of selection of materials is broad.

The vector of the present disclosure can be a vector for inserting a desired nucleic acid into a given site in a nucleic acid contained in a cell by a nuclease, wherein the nucleic acid contained in the cell comprises a region consisting of a first nucleotide sequence, the given site, and a region consisting of a second nucleotide sequence, in order, in a direction from the 5' end to the 3' end, wherein the nuclease specifically cleaves a portion consisting of the region consisting of a first nucleotide sequence and the region consisting of a second nucleotide sequence contained in the cell, and the vector comprises a region consisting of a first nucleotide sequence, the desired nucleic acid, and a region consisting of a second nucleotide sequence, in order, in a direction from the 5' end to the 3' end.

The vector of the present disclosure can also be a vector for inserting a desired nucleic acid into a given site in a nucleic acid contained in a cell by a nuclease comprising a first DNA binding domain and a second DNA binding domain, wherein the nucleic acid contained in the cell comprises a region consisting of a first nucleotide sequence, the given site, and a region consisting of a second nucleotide sequence, in order, in a direction from the 5' end to the 3' end, wherein the region consisting of a first nucleotide sequence, the given site, and the region consisting of a second nucleotide in the nucleic acid contained in the cell are each between a region consisting of a nucleotide sequence recognized by the first DNA binding domain and a region consisting of a nucleotide sequence recognized by the second DNA binding domain, wherein the vector comprises a region consisting of a first nucleotide sequence, the given nucleic acid, and a region consisting of a second nucleotide sequence, in order, in a direction from the 5' end to the 3' end, wherein the region consisting of a first nucleotide sequence and the region consisting of a second nucleotide sequence are each between a region consisting of a nucleotide sequence recognized by the first DNA binding domain and a region consisting of a nucleotide sequence recognized by the second DNA binding domain in the vector, and the vector produces a nucleic acid fragment comprising a region consisting of a first nucleotide sequence, the desired nucleic acid, and a region consisting of a second nucleotide sequence, in order, in a direction from the 5' end to the 3' end by the nuclease.

In the vector of the present disclosure, the first nucleotide sequence in the nucleic acid contained in the cell and the first nucleotide sequence in the vector are linked by microhomology-mediated end joining (MMEJ), and the second nucleotide sequence in the nucleic acid contained in the cell and the second nucleotide sequence in the vector are linked by MMEJ, whereby the desired nucleic acid can be inserted.

In the present disclosure, a nuclease may be a homodimer nuclease, and a vector may be a circular vector.

The present disclosure can provide a kit for inserting a desired nucleic acid into a given site in a nucleic acid contained in a cell, comprising the vector of the present disclosure and a vector for expressing a nuclease. The present disclosure can provide a method of inserting a desired nucleic acid into a given site in a nucleic acid contained in a cell, comprising the step of introducing the vector of the present disclosure and a vector for expressing a nuclease into a cell.

If the vector of the present disclosure is used, a desired nucleic acid can be inserted into a human immune system cell accurately and readily without requiring a complex step such as preparation of a vector of a long chain without resulting in a frame shift, regardless of the homologous recombination efficiency, and a nucleic acid with a relatively long chain of several kb or longer can be inserted. A method of inserting a nucleic acid using the vector of the present disclosure can use a nuclease comprising a homodimer DNA cleavage domain having high nuclease activity in combination. Alternatively, a method of inserting a nucleic acid using a vector of the present disclosure can use an RNA guided nuclease such as a CRISPR/Cas system in combination.

A cell to which the vector of the present disclosure is inserted comprises a nucleic acid comprising a region consisting of a first nucleotide sequence, a given site to which the nucleic acid is to be inserted, and a region consisting of a second nucleotide sequence, in order, in a direction from the 5' end to the 3' end. First nucleotide sequence and second nucleotide sequence are descriptions of convenience for showing the relationship with sequences contained in a vector to be inserted. A first nucleotide sequence and a second nucleotide sequence may be directly adjacent to the given site or adjacent with a region consisting of a specific base sequence interposed therebetween. When adjacent with a region consisting of a specific base sequence interposed therebetween, the specific base sequence is preferably 1 to 7 bases long, and more preferably 1 to 3 bases long. A first nucleotide sequence is preferably 3 to 10 bases long, more preferably 4 to 8 bases long, and still more preferably 5 to 7 bases long. A second nucleotide sequence is preferably 3 to 10 bases long, more preferably 4 to 8 bases long, and still more preferably 5 to 7 bases long.

The vector of the present disclosure can be a vector for inserting a desired nucleic acid into a given site in a nucleic acid contained in a cell by a nuclease. In the present disclosure, the nuclease can be a nuclease for specifically cleaving a portion consisting of a region consisting of the first nucleotide sequence and a region consisting of the second nucleotide sequence contained in the cell. Examples of such a nuclease include a nuclease comprising a first DNA binding domain and a second DNA binding domain. Examples of other nucleases for specific cleavage include RNA guided nucleases such as a nuclease in a CRISPR/Cas system. A portion known as PAM in a CRISPR/Cas system is essential for double strand cleavage by a Cas9 nuclease. Examples of Cas9 nucleases include Streptococcus pyogenes derived SpCas9 and Streptococcus thermophilus derived StCas9. PAM of SpCas9 is a "5'-NGG-3'" sequence (N is any nucleotide), and the portion where a double strand cleavage occurs is located three bases upstream (on the 5' end side) of PAM. A guide RNA (gRNA) in a CRISPR/Cas system recognizes a base sequence located on the 5' side of the portion where a double strand cleavage occurs. The portion where a double strand cleavage occurs in a CRISPR/Cas system corresponds to the given site to which insertion of a desired nucleic acid is intended in a nucleic acid contained in a cell. A region consisting of a first nucleotide sequence and a region consisting of a second nucleotide sequence are present at both ends of the given site. Thus, a CRISPR/Cas system using a gRNA that recognizes a base sequence located towards the 5' end side relative to PAM contained in a nucleic acid in a cell can specifically cleave a portion consisting of region consisting of a first nucleotide sequence and a region consisting of a second nucleotide sequence.

The vector of the present disclosure can comprise a region consisting of a first nucleotide sequence, a desired nucleic acid which is intended to be inserted into a cell, and a region consisting of a second nucleotide sequence, in order, in a direction from the 5' end to the 3' end. A region consisting of a first nucleotide sequence contained in a vector may be identical to a region consisting of a first nucleotide sequence in a nucleic acid contained in the cell. A region consisting of a second nucleotide sequence contained in a vector may be identical to a region consisting of a second nucleotide sequence in a nucleic acid contained in the cell.

The vector of the present disclosure can be preferably a circular vector. If a vector is a circular vector, the 3' end of a second nucleotide sequence and the 5' end of a first nucleotide sequence contained in the vector of the present disclosure are preferably adjacent or directly linked. If the vector of the present disclosure is a circular vector, and a second nucleotide sequence and a first nucleotide sequence are adjacent, the 3' end of the second nucleotide sequence and the 5' end of the first nucleotide sequence are separated by a region consisting of a base sequence that is preferably 1 to 7 bases long, more preferably 1 to 5 bases long, and still more preferably 1 to 3 bases long.

In a nucleic acid contained in a cell to be inserted by the vector of the present disclosure, a region consisting of a first nucleotide sequence may be between a region consisting of a nucleotide sequence recognized by a first DNA binding domain and a region consisting of a nucleotide sequence recognized by a second DNA binding domain. A region consisting of a second nucleotide sequence may be between a region consisting of a nucleotide sequence recognized by a first DNA binding domain and a region consisting of a nucleotide sequence recognized by a second DNA binding domain. The given site may be between a region consisting of a nucleotide sequence recognized by a first DNA binding domain and a region consisting of a nucleotide sequence recognized by a second DNA binding domain. In said nucleic acid, a combination of two nucleotide sequences recognized by a DNA binding domain surrounding a region consisting of a first nucleotide sequence may be different from a combination of two nucleotide sequences recognized by a DNA binding domain surrounding a region consisting of a second nucleotide sequence. In such a case, different nucleases are used for a nuclease for cleaving the surroundings of the region consisting of a first nucleotide sequence and a nuclease for cleaving the surroundings of the region consisting of a second nucleotide sequence. In a nucleic acid contained in the cell, a region consisting of a nucleotide sequence recognized by a first DNA binding domain and a region consisting of a nucleotide sequence recognized by a second DNA binding domain are separated by a region consisting of a nucleotide sequence that is preferably 5 to 40 bases long, more preferably 10 to 30 bases long, and still more preferably 12 to 20 bases long. The base length of a region separating the two regions may be identical to or different from the total of the base length of the first nucleotide sequence and the base length of the second nucleotide sequence. For example, if the conditions, i.e., the 3' end of a first nucleotide sequence and the 5' end of a second nucleotide sequence are in direct contact; there is no overlap between a nucleotide sequence recognized by a first DNA binding domain and a first nucleotide sequence; and there is no overlap between a second nucleotide sequence and a nucleotide sequence recognized by a second DNA binding domain, in a nucleic acid contained in the cell are met, the base length of a region separating the two sequences is identical to the total of the base length of the first nucleotide sequence and the base length of the second nucleotide sequence. However, if one or more of these conditions are not met, the base length of a region separating the two is different from the total of the base length of the first nucleotide sequence and the base length of the second nucleotide sequence. In a nucleic acid contained in a cell, a region consisting of a first nucleotide sequence may partially overlap with a region consisting of a nucleotide sequence recognized by a first DNA binding domain. In a nucleic acid contained in a cell, a region consisting of a second nucleotide sequence may partially overlap with a region consisting of a nucleotide sequence recognized by a second DNA binding domain. When there is a partial overlap, the overlapping portion consists of a nucleotide sequence that is preferably 1 to 6 bases long, more preferably 1 to 5 bases long, and still more preferably 2 to 4 bases long. If there is a partial overlap, the length of a portion separating two regions recognized by a DNA binding domain, which includes a region consisting of a first nucleotide sequence and a region consisting of a second nucleotide, would decrease more significantly by a linkage through microhomology-mediated end joining. Thus, it is less likely to be cleaved again after linkage, and the inserted nucleic acid is more stably retained, which is preferable.

In the vector of the present disclosure, a region consisting of a first nucleotide sequence and a region consisting of a second nucleotide sequence may each be between a region consisting of a nucleotide sequence recognized by a first DNA binding domain and a region consisting of a nucleotide sequence recognized by a second DNA binding domain. In said vector, a combination of two nucleotide sequences recognized by a DNA binding domain surrounding a region consisting of a first nucleotide sequence may be different from a combination of two nucleotide sequences recognized by a DNA binding domain surrounding a region consisting of a second nucleotide sequence. In such a case, different nucleases are used for a nuclease for cleaving the surroundings of a region consisting of a first nucleotide sequence and a nuclease for cleaving the surroundings of a region consisting of second nucleotide sequence. In a vector, a region consisting of a nucleotide sequence recognized by a first DNA binding domain may be towards the 5' end side or the 3' end side relative to a region consisting of a nucleotide sequence recognized by a second DNA binding domain. However, a nucleotide sequence, which is recognized by a first DNA binding domain or a second DNA binding domain and is located on the 3' end side of a first nuclease sequence, in a vector, is preferably different from a sequence, which is recognized by a first DNA binding domain or a second DNA binding domain and is located on the 3' end side of a second nucleotide in a nucleic acid contained in a cell. Further, a nucleotide sequence, which is recognized by a first DNA binding domain or a second DNA binding domain and is located on the 5' end side of a second nuclease sequence, in a vector, is preferably different from a sequence, which is recognized by a first DNA binding domain or a second DNA binding domain and is located on the 5' end side of a first nucleotide in a nucleic acid contained in a cell. In such cases, the frequency of repeated cleavage occurring after insertion of a desired nucleic acid can be further reduced by combining and using a nuclease comprising a heterodimer DNA cleavage domain. Further, a vector may be a vector resulting one cleavage site or two or more cleavage sites due to one or more nucleases comprising a first DNA binding domain and a second DNA binding domain. Examples of vectors resulting in two cleavage sites include vectors comprising a region consisting of a nucleotide sequence recognized by a first DNA binding domain, a region consisting of a first nucleotide sequence, a region consisting of a nucleotide sequence recognized by a second DNA binding domain, a desired nucleic acid intended to be inserted into a cell, a region consisting of a nucleotide sequence recognized by a first DNA binding domain, a region consisting of a second nucleotide sequence, and a region consisting of a nucleotide sequence recognized by a second DNA binding domain, in order, in a direction from the 5' end to the 3' end. When using a vector resulting in two cleavage sites, an unnecessary nucleic acid contained in the vector can be removed by cleavage with a nuclease, so that a desired cell that is free of an unnecessary nucleic acid can be more safely obtained.

In the vector of the present disclosure, a region separating a region consisting of a nucleotide sequence recognized by a first DNA binding domain and a region consisting of a nucleotide sequence recognized by a second DNA binding domain, comprising a region consisting of a first nucleotide sequence or a region consisting of a second nucleotide sequence, consists of a nucleotide sequence that is preferably 5 to 40 bases long, more preferably 10 to 30 bases long, and still more preferably 12 to 20 bases long. If a region separating a region consisting of a nucleotide sequence recognized by a first DNA binding domain and a region consisting of a nucleotide sequence recognized by a second DNA binding domain comprises both a first nucleotide sequence and a second nucleotide sequence, the base length of the separating region is identical or approximately identical to the total of the base length of the first nucleotide sequence and the base length of the second nucleotide sequence. As described above, a first nucleotide sequence is preferably 3 to 10 bases long, more preferably 4 to 8 bases long, and still more preferably 5 to 7 bases long. As described above, a second nucleotide sequence is preferably 3 to 10 bases long, more preferably 4 to 8 bases long, and still more preferably 5 to 7 bases long. If there is an overlap between a region consisting of a first nucleotide sequence or a second nucleotide sequence and a region consisting of a nucleotide sequence recognized by a DNA binding domain, if there is an overlap between a region consisting of a first nucleotide sequence and a region consisting of a second nucleotide sequence, or if a region consisting of a first nucleotide sequence and a region consisting of a second nucleotide sequence are not directly connected, the base length of the separating region would not be identical, just approximately identical to the total of the base length of the first nucleotide sequence and the base length of the second nucleotide sequence.

For example, in the vector of the present disclosure, the first nucleotide sequence in the nucleic acid contained in the cell and the first nucleotide sequence in the vector are linked by microhomology-mediated end joining (MMEJ), and the second nucleotide sequence in the nucleic acid contained in the cell and the second nucleotide sequence in the vector are linked by MMEJ, whereby the desired nucleic acid can be inserted into a given site in the nucleic acid contained in the cell.

A desired nucleic acid can be preferably a nucleic acid encoding an antigen receptor. An antigen receptor can be selected from the group consisting of a T cell receptor (TCR), a B cell receptor (BCR), and a chimeric antigen receptor (CAR). An antigen receptor can be a multimer consisting of two or more chains. A desired nucleic acid can be a nucleic acid encoding a plurality of chains (e.g., α chain and β chain) of an antigen receptor.

In the vector of the present disclosure, a region consisting of a first nucleotide sequence may be directly adjacent to the desired nucleic acid. Further, the desired nucleic acid may be directly adjacent to a region consisting of a second nucleotide sequence. If the desired nucleic acid comprises a functional agent such as a gene, a first nucleotide sequence and a second nucleotide sequence contained in the vector may encode a part of the functional agent.

The vector of the present disclosure may be a circular vector or a linear vector. A vector provided by the present disclosure is preferably a circular vector.

### (2-1. PITCh vector for introducing an antigen receptor)

In the present disclosure, a vector optionally comprising the aforementioned feature, configured for introducing an antigen receptor, can be used. Such a vector can comprise one or more features described below.

A vector can comprise a nucleic acid encoding an antigen receptor. In this regard, a nuclease used in combination with such a vector can be a nuclease that cleaves a locus of an endogenous antigen receptor of an immune cell. Knockout of an endogenous antigen receptor gene of an immune cell can be induced to occur concurrently with introduction of an antigen receptor. An endogenous antigen receptor gene may be a TRA gene or a TRB gene. Since there are four constant regions in a TRB gene, it can be possible to increase the amount of expression of TCR by introducing up to four copies. A nucleic acid encoding an antigen receptor can encode an α chain and a β chain of an antigen receptor together.

Synonymous substitution: If a given site is present in an endogenous antigen receptor gene of a cell, a nucleic acid encoding an antigen receptor may comprise a synonymous substitution in a sequence corresponding to a target site due to a nuclease contained in the cell. A synonymous substitution can prevent an antigen receptor sequence to be introduced to be cleaved by a nuclease while maintaining the basic structure of an antigen receptor that is expressed to materialize a high introduction efficiency. For example, a modification can be applied so that a codon of a TALEN target homologous sequence or a guide RNA that is present on a donor TCR cassette sequence is synonymously substituted.

Promoter: The technology of the present disclosure introduces a nucleic acid encoding an antigen receptor on a genome. In this regard, the nucleic acid encoding an antigen receptor may comprise a promoter operably linked to the antigen receptor, and the nucleic acid may be introduced into a genome, including the promoter. Since the technology of the present disclosure theoretically inserts only one copy of an antigen receptor within a sequence corresponding to a target site of a nuclease, it may be preferable to select a promoter that is capable of high and stable expression as the promoter. A promoter sequence can be, for example, an EF-1α promoter or a synthetic promoter developed for cells for introducing an antigen receptor gene (Colamartino A, Biol Blood Marrow Transplant, 2019; 3: Suppl S164, https://www.bbmt.org/article/S1083-8791(18)31286-2/abstract, etc.)

Leader sequence: A nucleic acid encoding an antigen receptor can comprise a leader sequence for enhancing translation efficiency upstream of the antigen receptor. Since the technology of the present disclosure theoretically inserts only one copy of an antigen receptor within a sequence corresponding to a target site of a nuclease, it can be advantageous to include a sequence for enhancing translation efficiency and introduce them together. For example, a 5'-UTR sequence of a β globin gene can be used as a leader sequence. In particular, a Xenopus globin 5'-UTR sequence is reported to have the best translation efficiency (https://www.ncbi.nlm.nih.gov/pubmed/2017171). Thus, it is understood to be suitable for use in the vector of the present disclosure.

Fluorescent dye: It can be desirable for the vector of the present disclosure to be free of a fluorescent dye sequence such as EGFP or mKATE in order to create a cell that can be clinically used.

Cassette length: It can be preferable for a sequence to be introduced (antigen receptor cassette) of the vector of the present disclosure to have the minimum length in order to facilitate incorporation into a site cleaved by a nuclease. Examples of the length of an antigen receptor cassette of the vector of the present disclosure include less than about 5000 base pairs, less than about 4900 base pairs, less than about 4800 base pairs, less than about 4700 base pairs, less than about 4600 base pairs, less than about 4500 base pairs, less than about 4400 base pairs, less than about 4300 base pairs, less than about 4200 base pairs, less than about 4100 base pairs, less than about 4000 base pairs, less than about 3900 base pairs, less than about 3800 base pairs, less than about 3700 base pairs, less than about 3600 base pairs, or less than about 3500 base pairs such as about 3400 base pairs. Since plasmids with a vector size exceeding 10 kb reduce the efficiency of introduction/efficiency of migration into the nucleus, the size of a core vector that has not been inserted with a cassette comprising multiple cloning sites can be, for example, less than about 6500 base pairs, less than about 6400 base pairs, less than about 6300 base pairs, less than about 6200 base pairs, less than about 6100 base pairs, less than about 6000 base pairs, less than about 5900 base pairs, less than about 5800 base pairs, less than about 5700 base pairs, less than about 5600 base pairs, less than about 5500 base pairs, less than about 5400 base pairs, less than about 5300 base pairs, less than about 5200 base pairs, less than about 5100 base pairs, less than about 5000 base pairs, less than about 4900 base pairs, less than about 4800 base pairs, less than about 4700 base pairs, less than about 4600 base pairs, less than about 4500 base pairs, or less than about 4400 base pairs such as about 4300 base pairs. If an antigen receptor cassette is large (e.g., if a plurality of antigen receptors (such as a plurality of TCRs and/or CARs) are simultaneously expressed using an IRES sequence, etc.), a small core vector may be used.

Vector amount: The vector of the present disclosure can be introduced at an amount of about 1 to about 5 µg per 2 x 10⁶ to 10⁷ cells. Toxicity of plasmids can be avoided for introduction at a high viability rate by using such an amount.

The vector that can be used in the present disclosure can be, for example, a vector having the sequence set forth in SEQ ID NO: 1, 2, or 3, or a sequence with an appropriate modification thereto. Those skilled in the art understand that a portion encoding an antigen receptor can be used after replacement thereof with a portion encoding the desired antigen receptor.

The vector that can be used in the present disclosure can comprise a cassette portion introduced into a genome of a cell, a sequence recognized by a nuclease, and a vector backbone. The vector backbone portion can be, for example, SEQ ID NO: 4 and SEQ ID NO: 5, SEQ ID NO: 6 and SEQ ID NO: 7, SEQ ID NO: 8 and SEQ ID NO: 9, etc. The sequence recognized by a nuclease can be a sequence selected from SEQ ID NOs: 10 to 15. Examples of the cassette portion include sequences set forth in SEQ ID NOs: 16 to 18. An element can be substituted, added, or deleted as needed from such sequences to be used as the cassette portion.

A cassette portion can comprise, for example, a polyA addition sequence, a leader sequence, and one or more promoter sequences in addition to a sequence encoding an antigen receptor. Examples of polyA addition sequences include the sequence of SEQ ID NO: 19. Examples of leader sequences include the sequence of SEQ ID NO: 21. Examples of promoter sequences include the sequences of SEQ ID NOs: 22 and 23. In the present disclosure, a promoter sequence, a leader sequence, and a polyA addition sequence may be deleted, modified, or substituted with a different sequence having the same effect as needed in a vector for use, as long as the desired expression is materialized.

It should be noted that the sequences specifically disclosed can be modified for use in the present disclosure, as long as the desired activity is maintained. For example, a sequence having identity of at least about 70%, at least about 75%, at least about 80%, at least about 85%, at least about 90%, at least about 95%, at least about 96%, at least about 97%, at least about 98%, or at least about 99% with respect to a sequence specifically disclosed can be used in the present disclosure. The nucleic acid sequences that are specifically disclosed can be used by adding one or more (preferably 1 or several, or 1, 2, 3, or 4) mutations (substitutions, insertions, or deletions) in the present disclosure, as long as the desired activity is maintained.

A modified amino acid sequence may be an amino acid sequence with one or a plurality (preferably one or several, or 1, 2, 3, or 4) conservative substitutions. As used herein, "conservative substitution" refers to substitution of one or a plurality of amino acid residues with other chemically similar amino acid residues in a manner that does not substantially modify the function of a protein. Examples thereof include a substitution of a hydrophobic residue with another hydrophobic residue, a substitution of a polar residue with another polar residue having the same charge, and the like. Functionally similar amino acids that can be substituted in such a manner are known in the art for each amino acid. Specific examples of nonpolar (hydrophobic) amino acids include alanine, valine, isoleucine, leucine, proline, tryptophan, phenylalanine, methionine, etc. Examples of polar (neutral) amino acids include glycine, serine, threonine, tyrosine, glutamine, asparagine, cysteine, etc. Examples of positively charged (basic) amino acids include arginine, histidine, lysine, etc. Examples of negatively-charged (acidic) amino acids include aspartic acid, glutamic acid, etc.

Since the same amino acid can be encoded by a plurality of gene codons, there can be a plurality of nucleic acid sequences encoding the same amino acid sequence. A different nucleic acid sequence can be utilized, as long as the purpose is to encode an amino acid sequence.

### (3. Nuclease)

A nuclease for inserting a desired nucleic acid into a given site in a nucleic acid contained in a cell in combination with a vector and use thereof herein are described. In the present disclosure, an immune cell may have a modification (e.g., knockdown) in an endogenous antigen receptor gene thereof, or this may be performed using a nuclease and subsequently introducing an antigen receptor via MMEJ using a vector with a similar nuclease. Knockdown of an endogenous antigen receptor can be performed with a nuclease or by using an antisense approach, RNAi, or the like. An endogenous antigen receptor can also be removed by knocking out an endogenous TCR gene. An endogenous TCR gene can be modified by, for example, deletion of all or part of a coding region, introduction of a mutation into a regulatory region, introduction of a nonsense or missense mutation, etc.

Preferably, modification of an endogenous TCR gene and/or introduction of a nucleic acid encoding an antigen receptor can be performed using a genome editing technology. Genome editing is a technology for modifying a target gene by utilizing a site specific nuclease. Examples of genome editing technologies include ZFN, TALEN, CRISPR/Cas9, etc. They each have a binding domain for materializing DNA sequence specific binding to a desired sequence and a cleavage domain for cleaving a DNA at a desired site of a sequence.

ZFN is an artificial restriction enzyme comprising a zinc finger domain and a DNA cleavage domain. A zinc finger domain can be modified to recognize any DNA base sequence, whereby a zinc finger nuclease can target a single sequence in a complex genome.

CRISPR (Clustered Regularly Interspaced Short Palindromic Repeats)/Cas9 (Crispr associated protein 9) system comprises two separate molecules, i.e., guide RNA and Cas9, whereas ZFN and TALEN are basically used as a single protein. A guide RNA can specifically bind to a target site by including a sequence that is complementary to a target site of DNA in the guide RNA. The guide RNA then binds to a Cas9 protein so as to cover the DNA, and the DNA is cleaved. Since Cas9 itself can be reused and it is sufficient to create only a guide RNA depending on a target site, multiplexing is facilitated.

TALEN (transcription activator-like effector nuclease) is an artificial enzyme made by fusing a DNA binding domain of a TALE protein secreted from phytopathogenic bacteria Xanthomonas, with a restriction enzyme FokI as a DNA cleavage domain. A DNA binding domain of a TALE protein has a repeat configuration of about 34 amino acids. The repeat unit is known as a module. In particular, the 12^{th} and 13^{th} amino acids are variable portions that bind to a target sequence and are known as "repeat variable diresidues" (RVD). TALEN uses molecules that bind to opposing chains of a target DNA as a pair of L TALEN and R TALEN. For FokI to exhibit cleaving activity, it is necessary for TALEN to maintain a suitable distance and form a dimer. Mismatch tolerance and off-target activity are hardly reported in TALEN. TALEN is characterized by high specificity. It is preferable to use TALEN with high specificity in the present disclosure because an unexpected adverse action can be induced if modified by off-targeting when modifying a T cell.

Various modified TALENs have been made in addition to conventional TALEN. Modification of an endogenous TCR gene with such a TALEN is preferable for its high specificity and high modification efficiency. The Examples herein demonstrate that complete removal of an endogenous TCR of a T cell was able to be materialized by using a modified TALEN.

A vector used in the present disclosure may be a vector for inserting a desired nucleic acid with any nuclease described herein. The nuclease may comprise a first DNA binding domain and a second DNA binding domain.

Examples of the origin of a DNA binding domain include TALE (Transcription Activator-Like Effector) of phytopathogenic bacteria Xanthomonas, zinc finger, etc. A DNA binding domain can preferably comprise one or more DNA binding modules that specifically recognize a base pair consecutively from the N-terminus side. One DNA binding module specifically recognizes one base pair. Thus, a first DNA binding domain and a second DNA binding domain recognize a respective region consisting of a specific nucleotide sequence. The nucleotide sequence recognized by a first DNA binding domain and the nucleotide sequence recognized by a second DNA binding domain may be the same or different. The number of DNA binding modules contained in a DNA binding domain is preferably 8 to 40, more preferably 12 to 25, and still more preferably 15 to 20 from the viewpoint of achieving both high nuclease activity and high DNA sequence recognition specificity of a DNA cleavage domain. Examples of a DNA binding module include a TAL effector repeat, etc. Examples of the length of one DNA binding module include 20 to 45, 30 to 38, 32 to 36, 34, and the like. The length of a DNA binding module contained in a DNA binding domain is preferably the same for all DNA binding modules. A first DNA binding domain and a second DNA binding domain preferably have the same origin, property, etc.

If an RNA guided Fokl nuclease (Fokl-dCas9) is used, Fokl-dCas9 forming a complex with gRNA can correspond to a nuclease comprising a DNA binding domain. dCas9 is Cas9 with catalytic activity thereof that is inactivated. dCas9 is guided by gRNA that recognizes a base sequence located in the vicinity of a location where a double strand cleavage occurs and binds to a nucleic acid. Specifically, dCas9 forming a complex with gRNA can correspond to a DNA binding domain.

A nuclease comprising a first DNA binding domain and a second DNA binding domain preferably comprises a first nuclease subunit comprising a first DNA binding domain and a first DNA cleavage domain and a second nuclease subunit comprising a second DNA binding domain and a second DNA cleavage domain.

Preferably, a first DNA cleavage domain and a second DNA cleavage domain, after a first DNA binding domain and a second DNA binding domain each bind to a DNA, approach each other to form a multimer to attain an improved nuclease activity. Examples of such a DNA cleavage domain include those derived from a restriction enzyme Fokl, etc. A DNA cleavage domain may be a heterodimer DNA cleavage domain or a homodimer DNA cleavage domain. If a first DNA cleavage domain and a second DNA cleavage domain approach each other, a multimer is formed to attain an improved nuclease activity. Meanwhile, even when a first DNA cleavage domain and a first DNA cleavage domain approach each other, a multimer is not formed to attain improved nuclease activity, and even when a second DNA cleavage domain and a second DNA cleavage domain approach each other, a multimer is not formed to attain improved nuclease activity. In such a case, the first DNA cleavage domain and the second DNA cleave domain are heterodimer DNA cleavage domains. If a first DNA cleavage domain and a first DNA cleavage domain approach each other and form a multimer to attain an improved nuclease activity, the first DNA cleavage domains are homodimer DNA cleavage domains. If a homodimer DNA cleavage domain is used, high nuclease activity is generally attained. A first DNA cleavage domain and a second DNA cleavage domain preferably have the same origin, properties, etc.

When a TALEN is used, a first DNA binding domain and a first DNA cleavage domain are connected through a polypeptide consisting of 20 to 70, 25 to 65, or 30 to 60, preferably 35 to 55, more preferably 40 to 50, still more preferably 45 to 49, and most preferably 47 amino acids in a first nuclease subunit. When ZFN is used, a first DNA binding domain and a first DNA cleavage domain are connected through a polypeptide consisting of 0 to 20 or 2 to 10, preferably 3 to 9, more preferably 4 to 8, and still more preferably 5 to 7 amino acids in a first nuclease subunit. When Fokl-dCas9 is used, dCas9 and FokI are connected through a polypeptide consisting of 1 to 20, 1 to 15, or 1 to 10, preferably 2 to 8, more preferably 3 to 7, still more preferably 4 to 6, and most preferably 5 amino acids in a first nuclease subunit. The same applies to a second nuclease subunit. Since a first nuclease subunit connected through a polypeptide with such a length has high specificity to a length of a portion consisting of a region consisting of a first nucleotide sequence and a region consisting of a second nuclease sequence, and specifically cleaves a spacer region with a specific length, the frequency of a nucleic acid being inserted into an unintended site due to nonspecific cleavage and the frequency of repeated cleavage of a nucleic acid after being linked through microhomology-mediated end joining described below are lower. Thus, such a subunit is preferable.

In the vector of the present disclosure, a nuclease may be combined with, for example, an RNA guided nuclease such as a nuclease from a CRISPR/Cas system. For example, a nuclease may be a Cas9 nuclease.

In the vector of the present disclosure, a nuclease is ZFN, TALEN, or Fokl-dCas9, and preferably TALEN. ZFN, TALEN, or Fokl-dCas9 may be a homodimer or a heterodimer nuclease. The nuclease is preferably a homodimer ZFN, TALEN, or Fokl-dCas9 and more preferably a homodimer TALEN.

The aforementioned nucleases also include mutants thereof. Such a mutant may be any mutant, as long as the activity of the nuclease is exhibited. Examples of such a mutant include nucleases comprising substitution, deletion, and/or addition of several, such as 1, 2, 3, 4, 5, 6, 7, 8, 9, 10, 15, 20, 25, 30, 35, 40, 45, or 50 amino acids in the amino acid sequence of the nuclease described above.

A nuclease can cleave a locus of an endogenous antigen receptor of an immune cell. Knockout of an endogenous antigen receptor gene of the immune cell can be induced to occur concurrently with introduction of the antigen receptor. An endogenous antigen receptor gene may be a TRA gene or a TRB gene. A constant region of a TRA gene or a TRB gene may be targeted.

### (3-1. Modified TALEN)

In one aspect of the present disclosure, a nuclease is preferably a TALEN and more preferably a Platinum TALEN. As is well known in the art, TALEN is a genome editing technology using a transcription activator-like (TAL) protein (see, for example, US Patent No. 8,470,973 and US Patent No. 8,586,363). Representative examples of a Platinum TALEN include those with more elevated activity than Voytas TALEN due to a cyclical arrangement with variation in the 4th and 32nd amino acids among the 34 amino acids contained in a DNA binding repeat of a TALEN (Sakuma et al., Sci Rep, 2013). The method of the present disclosure preferably can edit an endogenous TCR gene and/or introduce an antigen receptor by using a Platinum TALEN. Platinum TALEN is described in Japanese Laid-Open Publication No. 2016-175922, which is incorporated herein by reference in its entirety. The modified TALEN of the present disclosure can have, for example, a feature described below. In one embodiment of the present disclosure, a high function due to a functional domain and high specificity for recognizing a DNA sequence can be both achieved to safely attain a desired function at a high probability. Moreover, modification of an endogenous TCR gene and/or introduction of an antigen receptor is performed using a polypeptide that can be prepared by a simple operation or a nucleic acid encoding the same.

A polypeptide, wherein a DNA binding domain and a functional domain are connected by a polypeptide consisting of 35 to 55 amino acids, and amino acids at two specific positions in a DNA binding module contained in the DNA binding domain exhibit different repeat forms for each of the four DNA binding modules, can have both high functionality by a functional domain and high recognition specificity to a DNA sequence. A vector for expressing said polypeptide can be readily manufactured by using a vector set with a specific feature and a vector library with a specific feature.

In one embodiment of the present disclosure, the present disclosure can utilize a polypeptide comprising a DNA binding domain and a functional domain. The polypeptide wherein the DNA binding domain and the functional domain are connected by a polypeptide consisting of 35 to 55 amino acids, the DNA binding domain comprises a plurality of DNA binding modules consecutively from the N-terminus side, a combination of the xth amino acid and the yth amino acid in the 4n-3th DNA binding module from the N-terminus being identical for any n, a combination of the xth amino acid and the yth amino acid in the 4n-2th DNA binding module from the N-terminus being identical for any n, a combination of the xth amino acid and the yth amino acid in the 4n-1th DNA binding module from the N-terminus being identical for any n, and a combination of the xth amino acid and the yth amino acid in the 4nth DNA binding module from the N-terminus being identical for any n, the combination of the xth amino acid and the yth amino acid in the 4n-3th DNA binding module from the N-terminus, the combination of the xth amino acid and the yth amino acid in the 4n-2th DNA binding module from the N-terminus, the combination of the xth amino acid and the yth amino acid in the 4n-1th DNA binding module from the N-terminus, and the combination of the xth amino acid and the yth amino acid in the 4nth DNA binding module from the N-terminus are different from one another, and n is a natural number from 1 to 10, x is a natural number from 1 to 40, y is a natural number from 1 to 40, and x and y are different natural numbers, or a nucleic acid encoding the same can be used. The functional domain can be a DNA cleavage domain. Polynucleotides encoding polypeptides are included thereby.

The present invention can also utilize a vector library for manufacturing a vector comprising a polynucleotide encoding the polypeptide described above, wherein the vector library is comprised of a plurality of vectors having, in order from the 5' terminus, a first restriction enzyme cleavage site, a polypeptide encoding four DNA binding modules, and a second restriction enzyme cleavage site, wherein the combination of the first restriction enzyme cleavage site and the second restriction enzyme cleavage site is a combination of a type A restriction enzyme cleavage site and a type B restriction enzyme cleavage site, a combination of a type A restriction enzyme cleavage site and a type C restriction enzyme cleavage site, a combination of a type A restriction enzyme cleavage site and a type D restriction enzyme cleavage site, a combination of a type A restriction enzyme cleavage site and a type E restriction enzyme cleavage site, a combination of a type B restriction enzyme cleavage site and a type C restriction enzyme cleavage site, a combination of a type C restriction enzyme cleavage site and a type D restriction enzyme cleavage site, or a combination of a type D restriction enzyme cleavage site and a type E restriction enzyme cleavage site, wherein the type A restriction enzyme cleavage site to type E restriction enzyme cleavage site each result in different cleavage ends from one another by cleavage with the same restriction enzyme, and in the four DNA binding modules, a combination of the xth amino acid and the yth amino acid in the 1st DNA binding module from the 5' end being identical for any vector, a combination of the xth amino acid and the yth amino acid in the 2nd DNA binding module from the 5' end being identical for any vector, a combination of the xth amino acid and the yth amino acid in the 3rd DNA binding module from the 5' end being identical for any vector, and a combination of the xth amino acid and the yth amino acid in the 4th DNA binding module from the 5' end being identical for any vector, the combination of the xth amino acid and the yth amino acid in the 1st DNA binding module from the 5' end, the combination of the xth amino acid and the yth amino acid in the 2nd DNA binding module from the 5' end, the combination of the xth amino acid and the yth amino acid in the 3rd DNA binding module from the 5' end, and the combination of the xth amino acid and the yth amino acid in the 4th DNA binding module from the 5' end are different from one another, and x is a natural number from 1 to 40, y is a natural number from 1 to 40, and x and y are different natural numbers.

The present disclosure can also utilize a vector set for manufacturing the vector library described above. In this regard, the vector set comprises a plurality of vectors comprising, in order from the 5' end, a first restriction enzyme cleavage site, a DNA binding module, and a second restriction enzyme cleavage site, the first restriction enzyme cleavage site and the second restriction enzyme cleavage site resulting in different cleavage ends from each other by cleaving with the same restriction enzyme, a combination of the xth amino acid and the yth amino acid in the DNA binding module being one of four different combinations, wherein x is a natural number from 1 to 40, y is a natural number from 1 to 40, and x and y are different natural numbers.

Since the polypeptide described above materializes high functionality due to a functional domain and high recognition specificity to a DNA sequence, a modification of a desired TCR gene and/or introduction of an antigen receptor can be materialized safely and at a high probability by introducing a vector comprising a polynucleotide encoding the polypeptide described above into a cell. If the vector library described above is used, a vector for expressing a polypeptide achieving both high functionality due to a functional domain and high recognition specificity to a DNA sequence can be prepared readily and quickly.

Examples of origins of a DNA binding domain include the phytopathogenic bacteria Xanthomonas TALE (Transcription Activator-Like Effector), Zinc finger, etc.

Examples of functional domains include domains encoding enzymes, transcription regulatory factors, reporter proteins, etc. Examples of enzymes include DNA modifying enzymes such as a recombinase, nuclease, ligase, kinase, and phosphatase, and other enzymes such as lactamase. As used herein, a domain encoding a nuclease is referred to as a DNA cleavage domain. Examples of transcription regulatory factors include activators, repressors, etc. Examples of reporter proteins include fluorescent proteins such as a green fluorescent protein (GFP), humanized Renilla reniformis green fluorescent protein (hrGFP), enhanced green fluorescent protein (eGFP), enhanced blue fluorescent protein (eBFP), enhanced cyan fluorescent protein (eCFP), enhanced yellow fluorescent protein (eYFP), red fluorescent protein (RFP or DsRed), and mCherry; bioluminescent proteins such as firefly luciferase and Renilla luciferase; enzymes converting a chemiluminescent substrate such as alkaline phosphatase, peroxidase, chloramphenicol acetyltransferase, and β-galactosidase, etc. A DNA cleavage domain preferably approaches another DNA cleavage domain to form a multimer, and attains improved nuclease activity. Examples of such a DNA cleavage domain include those derived from FokI, etc.

A DNA binding domain and a functional domain are connected by a polypeptide consisting of 35 to 55, preferably 40 to 50, more preferably 45 to 49, and most preferably 47 amino acids.

A DNA binding domain can comprise a plurality of DNA binding modules consecutively from the N-terminus side. One DNA binding module specifically recognizes one base pair. The number of DNA binding modules contained in a DNA binding domain is preferably 8 to 40, more preferably 12 to 25, and still more preferably 15 to 20, from the viewpoint of achieving both high functionality of a functional domain and high DNA sequence recognition specificity. Examples of DNA binding modules include TAL effector repeat, etc. Examples of the length of a single DNA binding module include 20 to 45, 30 to 38, 32 to 36, 34, etc. The length of a DNA binding module contained in a DNA binding domain is preferably the same for all DNA binding modules.

A combination of the xth amino acid and the yth amino acid in the 4n-3th DNA binding module from the N-terminus can be identical for any n. Further, a combination of the xth amino acid and the yth amino acid in the 4n-2th DNA binding module from the N-terminus can be identical for any n. Further, a combination of the xth amino acid and the yth amino acid in the 4n-1th DNA binding module from the N-terminus can be identical for any n. Further, a combination of the xth amino acid and the yth amino acid in the 4nth DNA binding module from the N-terminus can be identical for any n. In this regard, n is a natural number from 1 to 10, preferably a natural number from 1 to 7, and more preferably a natural number from 1 to 5. n is preferably a natural number that is sufficient to indicate all DNA binding modules contained in a DNA binding domain, x is a natural number from 1 to 40, preferably a natural number from 1 to 10, more preferably a natural number from 2 to 6, still more preferably a natural number from 3 to 5, and most preferably the natural number 4. y is a natural number from 1 to 40, preferably a natural number from 25 to 40, more preferably a natural number from 30 to 36, still more preferably a natural number from 31 to 33, and most preferably the natural number 32. x and y are different natural numbers. The values of x and y can be different depending on the length of the DNA binding modules used. x is preferably a numerical value indicating a position corresponding to the 4th amino acid in a DNA binding module consisting of 34 amino acids. y is preferably a numerical value indicating a position corresponding to the 32nd amino acid in a DNA binding module consisting of 34 amino acids.

A combination of the xth amino acid and the yth amino acid in the 4n-3th DNA binding module from the N-terminus, a combination of the xth amino acid and the yth amino acid in the 4n-2th DNA binding module from the N-terminus, a combination of the xth amino acid and the yth amino acid in the 4n-1th DNA binding module from the N-terminus, and a combination of the xth amino acid and the yth amino acid in the 4nth DNA binding module from the N-terminus can be different from one another, wherein n is a natural number from 1 to 10, preferably a natural number from 1 to 7, and more preferably a natural number from 1 to 5. n is preferably a natural number that is sufficient to indicate all DNA binding modules contained in a DNA binding domain. x is a natural number from 1 to 40, preferably a natural number from 1 to 10, more preferably a natural number from 2 to 6, still more preferably a natural number from 3 to 5, and most preferably the natural number 4. y is a natural number from 1 to 40, preferably a natural number from 25 to 40, more preferably a natural number from 30 to 36, still more preferably a natural number from 31 to 33, and most preferably the natural number 32. x and y are different natural numbers. Preferably, a combination of the xth amino acid and the yth amino acid in the 4n-3th DNA binding module from the N-terminus, a combination of the xth amino acid and the yth amino acid in the 4n-2th DNA binding module from the N-terminus, and a combination of the xth amino acid and the yth amino acid in the 4nth DNA binding module from the N-terminus can each be selected from the group consisting of, in order of x and y, a combination of D and D, a combination of E and A, a combination of D and A, and a combination of A and D.

Examples of vectors that can be used include plasmid vectors, cosmid vectors, viral vectors, artificial chromosome vectors, etc. Examples of artificial chromosome vectors include yeast artificial chromosome vectors (YAC), bacterial artificial chromosome vectors (BAC), P1 artificial chromosome vectors (PAC), mouse artificial chromosome vectors (MAC), human artificial chromosome vectors (HAC), etc. Examples of vector components include nucleic acids such as DNA and RNA, nucleic acid analogs such as GNA, LNA, BNA, PNA, and TNA, etc. Vectors may be modified with a component other than a nucleic acid such as saccharides.

The polypeptide described above can be prepared by introducing a vector into a cell or the like to cause the expression thereof. A desired function corresponding to the functional domain e.g., DNA modification such as DNA recombination or DNA cleavage, expression of other enzymatic activity such as transcription regulation, labeling of a DNA region with a reporter protein, and/or introduction of an antigen receptor via MMEJ can be exerted in a cell by introducing a vector into a cell or the like to cause expression thereof. In one aspect of the present disclosure, a composition for use in any method of the present disclosure, comprising a Platinum TALEN can be provided.

### (4. TCR modification)

In the present disclosure, a method of introducing a nucleic acid encoding an antigen receptor into a given site in a nucleic acid contained in an immune cell of a target organism can be provided. The method can comprise the step of introducing a vector comprising the nucleic acid encoding an antigen receptor and a nuclease into the immune cell. The nuclease cleaves the vector and a genome sequence of the immune cell, and the vector can be configured to produce, when cleaved by the nuclease, a sequence encoding the antigen receptor, which is introduced into a portion cleaved by the nuclease of the genome sequence of the immune cell by microhomology-mediated end joining (MMEJ).

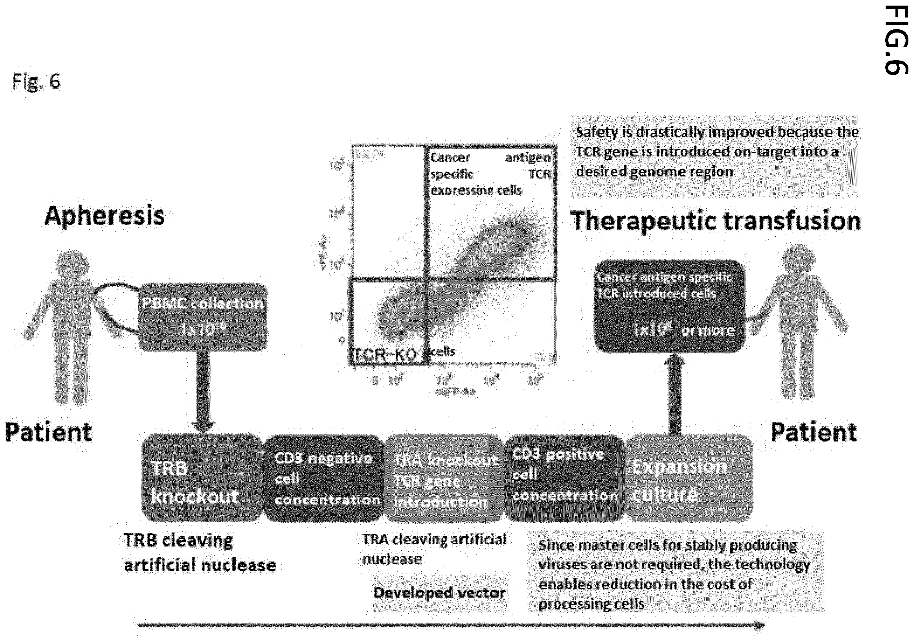

Although not intended to be limiting, the technology described herein can be used, for example, in an immunotherapy process shown in Figure **6****.**

In one aspect, the present disclosure provides a method of administering immunotherapy to a subject, comprising the steps of: (A) obtaining blood from the subject; (B) knocking out a TRB of mononuclear cells of the blood (PBMC); (C) concentrating the mononuclear cells for CD3 negative; (D) knocking out a TRA of the mononuclear cells and introducing an antigen receptor (a TCR or CAR-T); (E) concentrating the mononuclear cells for CD3 positive; and (G) optionally subjecting the mononuclear cells to expansion culture.

(A) the step of obtaining blood from the subject is characterized by the following.

About 1 × 10¹⁰ peripheral blood mononuclear cells (PBMC) are collected using a medically approved continuous blood component separator such as Spectra Optia^{®} (TERUMO BCT) or COM.TEC^{®} (FRESENIUS KABI Japan), which is already used in blood component donation, collection of peripheral blood stem cells, etc. The collection rate is 60 ml/min or less in order to avoid onset of an adverse event, such as hypocalcemia, during collection as much as possible.

(B) the step of knocking out the first antigen receptor locus (e.g., TRB) of mononuclear cells of the blood (PBMC) is characterized by the following.

A vector of a nuclease having a TRB constant region as a target sequence is introduced into a T cell in the growth phase by using electroporation during the optimal period within 48 to 72 hours after activation/growth of T cells by using GMP grade Dynabeads^{®} (Thermo Fisher) CD3/CD28. More preferably, this process is performed by purifying the CD3 positive cell fraction from PBMCs by using GMP grade CD3 positive cell separation beads such as MACS GMP CD3 pure^{®}

### (Miltenyi Biotec).

(C) the step of concentrating the mononuclear cells for CD3 negative is characterized by the following.

Since T cells that are introduced with a nuclease and deficient of TCRβ chain expression simultaneously lose expression of CD3, a CD3 negative cell fraction is purified and concentrated using GMP grade CD3 positive cell separation beads such as MACS GMP CD3 pure^{®} (Miltenyi Biotec).

(D) the step of knocking out a second antigen receptor locus (e.g., TRA) of the mononuclear cells and introducing an antigen receptor (TCR or CAR-T) is characterized by the following.

From the step of (C), T cells deficient of TCRβ chain expression are grown in the presence of IL-2, and a circular DNA vector according to the present disclosure for introducing a desired TCR is introduced by a second electroporation, simultaneously with a vector of a nuclease for cleaving a TCRα chain at an optimal timing of about 48 to 72 hours from the first electroporation.

It is also possible to omit the process of C), and introduce a circular DNA vector according to the present disclosure for introducing a desired CAR simultaneously with a vector of a nuclease for cleaving a TCRα chain by electroporation and purify a CD3 negative cell fraction to concentrate T cells having a cleaved TCRα chain and expressing a CAR.

(E) the step of concentrating the mononuclear cells for CD3 positive is characterized by the following.

If a TCR is introduced, T cells of which TCR expression has recovered due to introduction of a vector also recovers expression of CD3. Thus, a CD3 positive cell fraction is purified and concentrated again by using GMP grade CD3 positive cell separation beads such as MACS GMP CD3 pure^{®}

### (Miltenyi Biotec).

(G) optionally subjecting the mononuclear cells to expansion culture is characterized by the following.

When expanding T cells, which have been purified and concentrated by step (E), expressing a desired TCR (or T cells deficient of TCR and expressing CAR), IL-2 is added at a suitable concentration, a medium is added when appropriate to maintain a cell concentration of about 1 × 10⁶ cells/ml, and a bag is optionally exchanged with a cell culture bag with a large size.

In a preferred embodiment, the TRA and/or TRB of the present disclosure may be cleaved by using a Platinum TALEN.

In a specific embodiment, the immunotherapy comprises TCR-T therapy or CAR-T therapy. As used herein, "TCR-T therapy" refers to a cell therapy utilizing modification of a T cell receptor (TCR) such as those used in cancer therapy. As used herein, "CAR-T therapy" refers to gene/cell therapy method of transfecting a chimeric antigen receptor (CAR) (e.g., genetically engineered to overcome the tumor immune escape mechanism) into patient T cells, amplifying and culturing the T cells ex vivo, and transfusing the cells into a patient.

The present disclosure provides a composition comprising a Platinum TALEN for use in a method of administering immunotherapy to a subject, comprising the steps of: (A) obtaining blood from the subject; (B) knocking out a TRB of mononuclear cells of the blood (PBMC); (C) concentrating the mononuclear cells for CD3 negative; (D) knocking out a TRA of the mononuclear cells and introducing an antigen receptor (TCR or CAR-T); (E) concentrating the mononuclear cells for CD3 positive; and (G) optionally subjecting the mononuclear cells to expansion culture.

### (4-1. Introduction step)

In the present disclosure, the step of introducing a vector can be performed by using electroporation. In the present disclosure, introduction of a nuclease can be performed concurrently with a vector comprising a nucleic acid encoding an antigen receptor by introducing a nucleic acid (vector) encoding the nuclease.

It may be preferable to perform electroporation within a flow chamber. While cells may die in some cases due to the high temperature of buffer in electroporation, buffer can be maintained at a low temperature within a chamber (flow chamber) installed in a channel to reduce damage to cells and prevent death of the cells due to excessive heat. A buffer that facilitates survival of cells after electroporation may also be used. Electroporation can be performed with MaxCyte Flow Electroporation^{™}, NEPA21^{®}, NEON^{®}, CliniMACS Electroporator^{®}, or the like.

In the present disclosure, it can be advantageous to check the status of cells for introduction at the optimal timing. For example, a nuclease and a vector may be introduced by electroporation between about 48 hours to about 72 hours after stimulation of immune cells with CD3/CD28 beads. Introduction may be performed at the optimal time between about 48 to about 72 hours after stimulation. Those skilled in the art can find the optimal timing upon performing this step. Prior to the introduction step, it can be advantageous to check for any instance of swelling or clustering of cells following activation/growth of the cells with an optical microscope and to check for an increase in the values of both forward scatter and side scatter relative to cells in the resting phase by using flow cytometry.

In the present disclosure, the amount of DNA of a PITCh vector to be introduced can be about 1 to 5 µg.

### (5. Preferred embodiment)

Preferred embodiments of the present disclosure are described below. Embodiments provided below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is understood that the following embodiments of the present disclosure can be used alone or in combination.

Each of the embodiments described below provides a comprehensive or specific example. The numerical values, shapes, materials, constituent elements, positions of arrangement and connection modes of the constituent elements, steps, order of steps, and the like in the following embodiments are one example, which is not intended to limit the Claims. Further, the constituent elements in the following embodiments that are not recited in the independent claims showing the most superordinate concept are described as an optional constituent element.

### (5-1. Kit)

The present disclosure can provide a kit for inserting a nucleic acid encoding an antigen receptor into a given site in a nucleic acid contained in a cell, comprising the vector described herein and a vector for expressing the nuclease described herein.

### (5-2. Method)

The present disclosure can provide a method of inserting a nucleic acid encoding an antigen receptor into a given site in a nucleic acid contained in an immune cell in vitro or in vivo (in vivo method of inserting into a non-human organism in one embodiment), comprising the step of introducing the vector described herein and a vector for expressing the nuclease described herein into an immune cell. A method of manufacturing an immune cell that expresses an antigen receptor, comprising the step of inserting a nucleic acid encoding the antigen receptor into a given site in a nucleic acid contained in the immune cell by the method can be provided.

### (5-3. Immunotherapy)

The method, kit, cell created thereby, etc. of the present disclosure can be used in immunotherapy. It is understood that immunotherapy is effective on diseases resulting in a lesion with antigenicity (e.g., cancer) and diseases in which an abnormal immune response to a specific antigen is involved in the onset or progression of a pathological condition.

Cancer immunotherapy is a method of treating cancer by using the biological defense mechanism of an organism. Cancer immunotherapy is roughly classified into cancer immunotherapy through strengthening the immunological function against cancer and cancer immunotherapy through inhibition of the cancer immune escape mechanism. Furthermore, cancer immunotherapy includes active immunotherapy that activates the immunological function within the body and passive immunotherapy through returning immune cells, which have been grown or induced to activate the immunological function outside the body, into the body. Examples of cancer immunotherapy include non-specific immunopotentiators, cytokine therapy, cancer vaccine therapy, dendritic cell therapy, adoptive immunotherapy, non-specific lymphocyte therapy, cancer antigen specific T cell therapy, antibody therapy, immune checkpoint inhibition therapy, etc. It is understood that cells created by the method of the present disclosure are effective for adoptive cell transfer.

Cells created by the method of the present disclosure can be combined with another cancer therapy when appropriate and can be used as a combination therapy. Typically, such cells can be administered in combination with one or more additional agents. Alternatively, combination therapy may be a combination with radiation therapy. One or more additional agents may be any chemotherapeutic drug, or contain an immune checkpoint inhibitor. Examples of another cancer therapy used in combination therapy include, but are not limited to, other cancer immunotherapy (e.g., immune checkpoint inhibitor), hyperthermia therapy, surgical procedure, etc.

Examples of cancer targeted by the present disclosure include, but are not limited to, melanoma (malignant melanoma), non-small cell lung cancer, renal cell cancer, malignant lymphoma (Hodgkin's or non-Hodgkin's lymphoma), head and neck cancer, urological cancer (bladder cancer, urothelial cancer, and prostate cancer), small cell lung cancer, thymic carcinoma, gastric cancer, esophageal cancer, esophagogastric junction cancer, liver cancer (hepatocellular carcinoma and intrahepatic cholangiocarcinoma), primary brain tumor (glioblastoma and primary central nervous system lymphoma), malignant pleural mesothelioma, gynecologic cancer (ovarian cancer, cervical cancer, and uterine cancer), soft tissue sarcoma, cholangiocarcinoma, multiple myeloma, breast cancer, colon cancer, and the like.

Immune cells created in the present disclosure can be used in the treatment, therapy, or prevention of an autoimmune disease, allergic disease, or graft-versus-host disease (GVHD), rejection, or graft failure in transplantation. Examples of autoimmune diseases include, but are not limited to, rheumatoid arthritis (RA), Sjogren's syndrome, systemic lupus erythematosus (SLE), antiphospholipid syndrome, polymyositis/dermatomyositis, systemic sclerosis, mixed connective tissue disease, vasculitis syndrome, type I diabetes, Graves' disease, Hashimoto Disease, idiopathic Addison's disease, autoimmune hepatitis, Goodpasture syndrome, glomerulonephritis, autoimmune hemolytic anemia (AIHA), autoimmune thrombocytopenic purpura, autoimmune neutropenia, myasthenia gravis, pemphigus, vitiligo, idiopathic azoospermia, and the like. Examples of allergic diseases include, but are not limited to, hay fever, allergic rhinitis, bronchial asthma, atopic dermatitis, and the like. In addition, treatment or prevention utilizing the antigen specific regulatory T cell of the present disclosure can be used for diseases in which an abnormal immune response to a specific antigen is involved in the onset or progression of a pathological condition.

### (5-4. Cells)

One embodiment of the present disclosure can provide a cell or a population of cells comprising an exogenous antigen receptor of interest that is modified by the method, vector, etc. of the present disclosure. The cell population has a reduced ratio of cells comprising an antigen receptor other than the exogenous antigen receptor of interest. The ratio of cells comprising an antigen receptor other than the exogenous antigen receptor of interest is, for example, less than about 20%, about 15%, about 12%, about 10%, about 7%, about 5%, about 3%, about 2%, or about 1%, or the cell population of the present disclosure is substantially free of cells comprising an antigen receptor other than the exogenous antigen receptor of interest. The exogenous antigen receptor of interest may be a T cell receptor or chimeric antigen receptor for a desired antigen.

The cell population of the present disclosure can comprise cells with reduced or substantially no introduction of a mutation into a site other than an endogenous antigen receptor locus upon introduction of an antigen receptor. The ratio of cells introduced with a mutation into a site other than an endogenous antigen receptor locus in the cell population of the present disclosure is, for example, less than about 20%, about 15%, about 12%, about 10%, about 7%, about 5%, about 3%, about 2%, or about 1%, or the cell population of the present disclosure is substantially free of cells introduced with a mutation into a site other than an endogenous antigen receptor locus.

### (General technology)

The molecular biological methodology, biochemical methodology, and microbiological methodology used herein are well known and conventionally used in the art.

### (Note)

As used herein, "or" is used when "at least one or more" of the listed matters in the sentence can be employed. When explicitly described herein as "within the range" of "two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

### [Examples]

The Examples are described hereinafter. The subjects used in the following Examples were handled, when needed, in compliance with the ethical guidelines for human genomic gene/analysis studies specified by the national government, ethical guidelines for medical studies involving humans, and the standards stipulated by the Hiroshima University. Even where it is not explicitly stated, experiments were conducted in accordance with relevant laws and regulations.

### (Example 1-1: Introduction of 1G4 TCR into β null Jurkat cells using MaxCyte by TAL-PITCh)

### (Materials and Methods)

1G4 TCRs were introduced into β null Jurkat cells by TAL-PITCh in accordance with the following protocol.

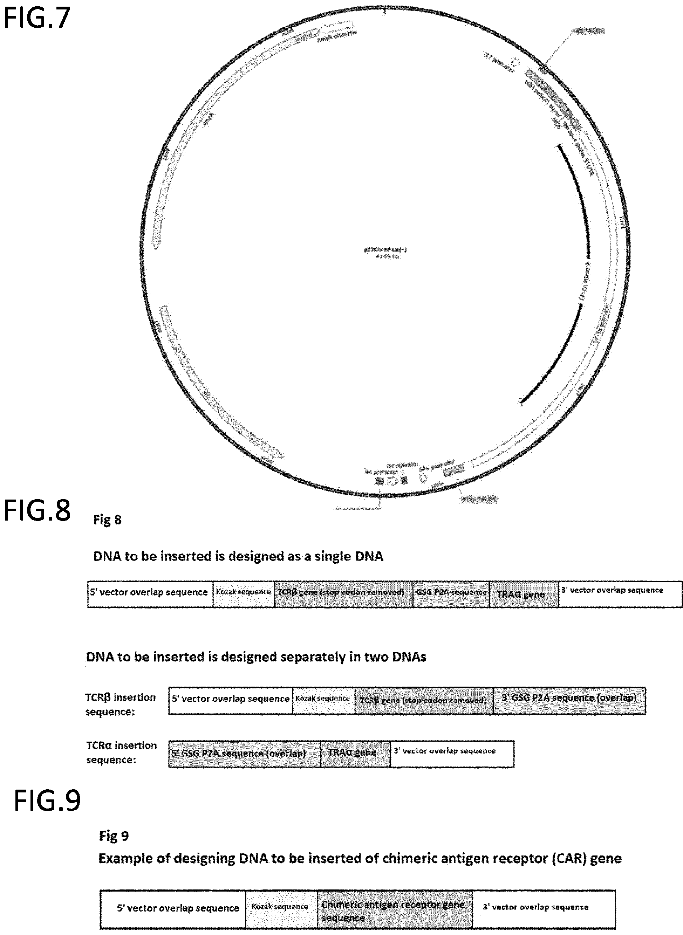

1) Platinum TALEN mRNA targeting TRB C region exon 1 is introduced by electroporation, then a CD3 negative fraction is sorted to create β null Jurkat cells that do not express a TCRβ chain.
2) The β null Jurkat cells are counted.
3) 2 × 10⁶ β null Jurkat cells/sample are centrifuged (400 G, 10 minutes, room temperature) to prepare cell pellets.
4) The supernatant is discarded, PBS is added, and the samples are centrifuged (400 G, 10 minutes, room temperature) to prepare cell pellets.
5) The supernatant is discarded, MaxCyte buffer is added, and the samples are centrifuged (400 G, 10 minutes, room temperature) to prepare cell pellets.
6) The solution in the following Table 1 is prepared with TCRα2 TALEN mRNA and 1G4 TCR TAL-PITCh vector (SEQ ID NO: 1, Figure **7****),** and the cells pellets are suspended.
7) The cell suspension is transferred to a cuvette (SOC-25) and subjected to electroporation by using MaxCyte (program: Jurkat).
8) The cell suspension is transferred to a plate and left standing for 40 minutes in a CO₂ incubator.
9) A medium (RPMI 1640 + 10% FBS + 2 mmol/l L-Glutamine + 1% penicillin/streptomycin) is added, and culture is started in an incubator (37°C, 5% CO₂).
10) After 96 hours, the culture is analyzed by FACS.

**[Table 1]**

| [Condition settings] | | | | | | |
|---|---|---|---|---|---|---|
| Sample# | Cell Type | EP Protocol | DNA (ug/25ul) | α2TALEN (ug/25ul) | Cell (Cells/25ul) | Processing Assembly |
| 1 | *β* null Jurkat | No EP | 0 | 0 | 1.00E+07 | |
| 2 | *β* null Jurkat | Jurkat | 0 | 0 | 1.00E+07 | OC-25×3 |
| 3 | *β* null Jurkat | Jurkat | 0 | 10 | 1.00E+07 | OC-25×3 |
| 4 | *β* null Jurkat | Jurkat | 5 | 10 | 1.00E+07 | OC-25×3 |

Following the above experiment, the stability of expression was tested by the following protocol.

1) A medium for culturing Jurkat cells (RPMI 1640 + 10% FBS + 2 mmol/l L-Glutamine + 1% penicillin/streptomycin) is added to 2 × 10⁶ Jurkat cells (Control Jurkat cells) and β-null Jurkat cells used in the above experiment and 1G4 expressing Jurkat cells (αβ-null Jurkat cells) created in the above experiment. The cell groups were cultured in parallel in a normal Co₂ incubator (37°C, 5% CO₂).
2) On day 32 after starting the culture, expression levels of CD3, TCRαβ, and 1G4-TCR in each cell group were analyzed by using flow cytometry.

### (Results)

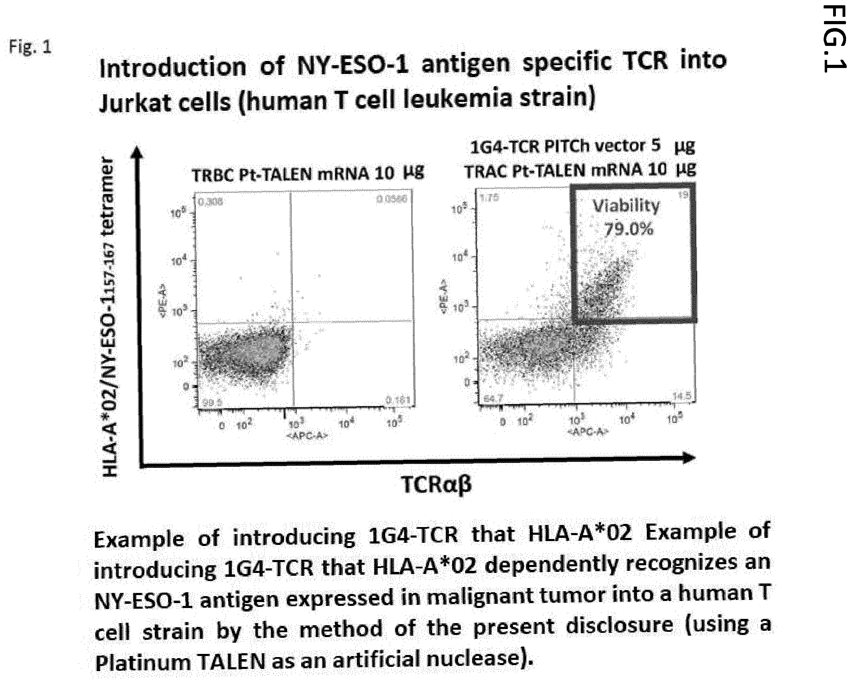

The results are shown in Figure **1****.** This shows that 1G4-TCR, which HLA-A*02 dependently recognizes an NY-ESO-1 antigen expressed in malignant tumor, can be introduced into human T cell strains without using a viral vector by the method of the present disclosure.

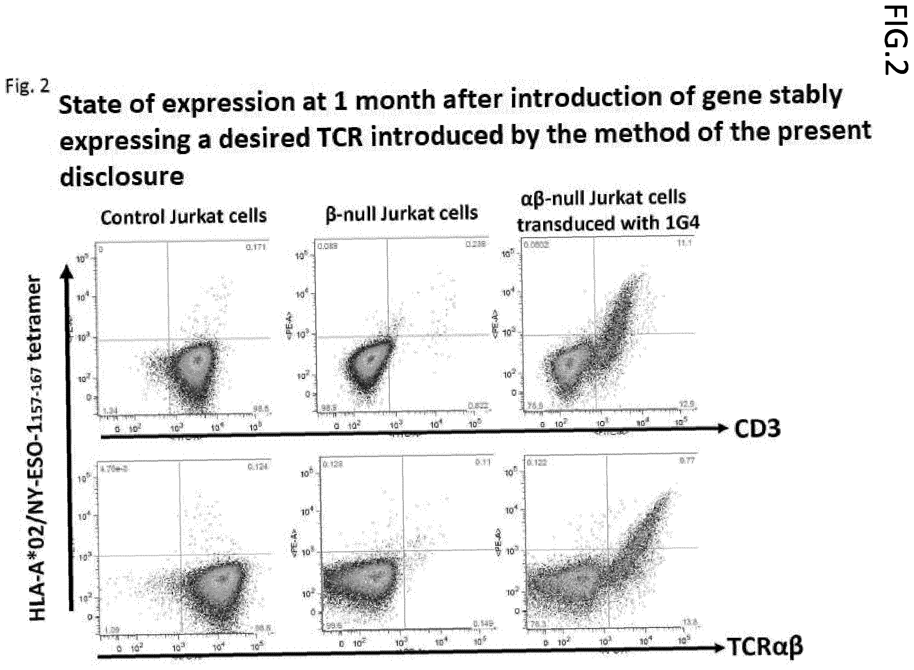

The stability of expression after transfection is demonstrated in that 1G4-TCR expressing αβ-null Jurkat cells created by the present method stably express 1G4-TCR over 30 days or longer (Figure **2****).**

### (Example 1-2: TCR Vβ repertoire analysis)

### (Materials and Methods)

Expression of receptors other than TCR introduced into T cells were studied by the following protocol.

1. Prepare eight 5 ml tubes of 1 × 10⁶ β null Jurkat cells introduced with 1G4 TCR by the method described in Example 1-1 and eight 5 ml tubes of 1 × 10⁶ Jurkat cells used as a control.
2. First washing: Fill the tubes until reaching 3 ml with PBS and centrifuge the tubes for 10 minutes (400 g, 4°C). Remove the supernatant.
3. Second washing in the same manner as 2.
4. Add 10 µl of each of antibody cocktails A to H for flow cytometry of IOTest Beta Mark (Beckman Coulter) (see the following Table: from Beckman Coulter "IOTest^{®} Beta Mark" Manufacturer's Instruction) to each tube and stir.
5. Leaving the tubes standing for 20 minutes at room temperature away from light.
6. First washing in the same manner as 2.
7. Second washing in the same manner as 2.
8. Add 500 µl of 0.5% BSA PBS buffer to each tube and pipette the resultant.
9. Analyze the sample by FACS Canto II.

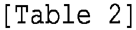

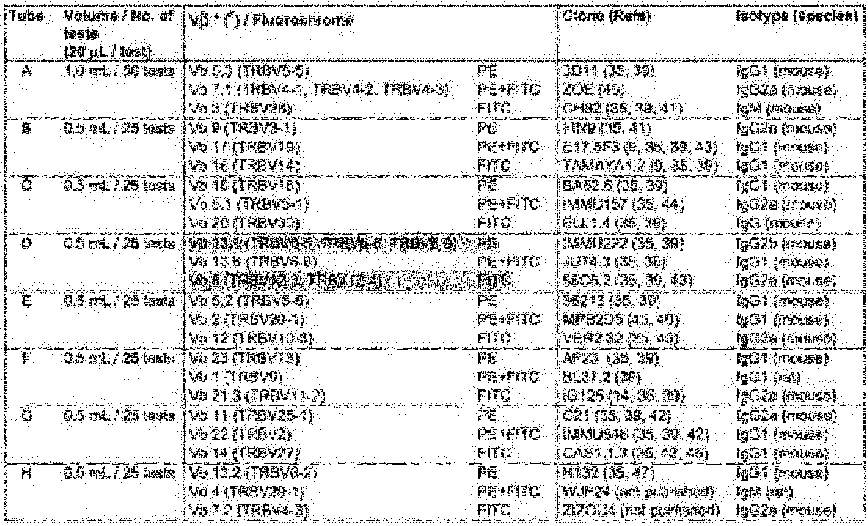

### (Results)

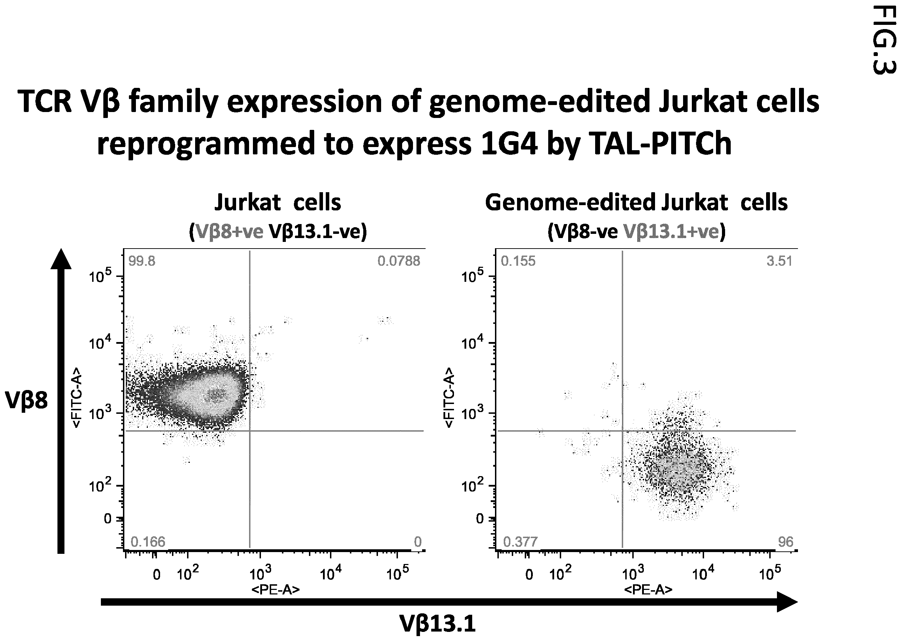

Figure **3** shows results of analyzing expressed TCR of β null Jurkat cells introduced with 1G4 TCR and Jurkat cells (control) when antibody cocktail D was added. It can be clearly understood from Figure **3** that 1G4 expressing Jurkat cells created by the present method have lost expression of a Vβ8 chain that was present before genome editing and express a Vβ13.1 chain that matches 1G4-TCR.

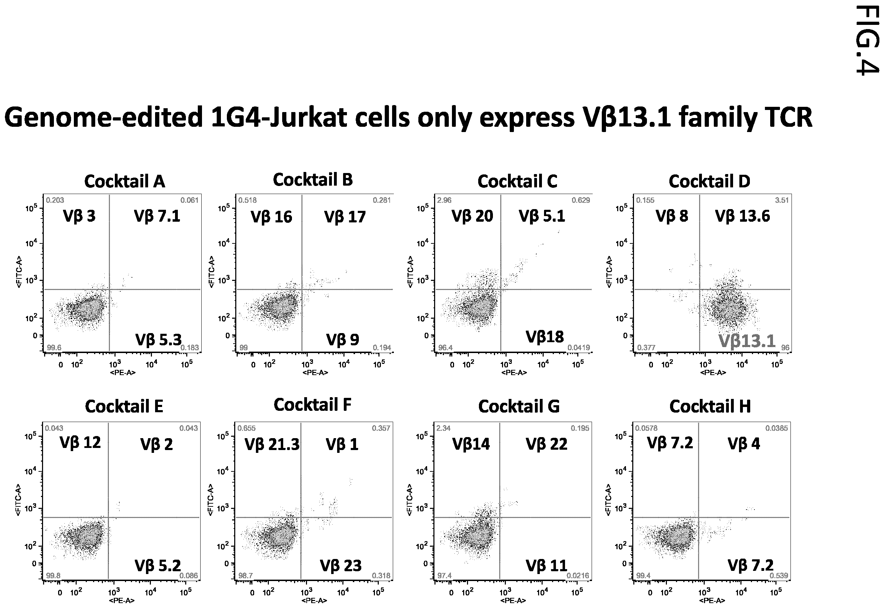

Figure **4** shows that β null Jurkat cells introduced with 1G4 TCR do not express a V chain other than a Vβ13.1 chain that matches 1G4-TCR, i.e., a new Vβ chain derived from a non-reconstituted allele is not expressed after genome editing.

### (Example 1-3: Checking integration of 1G4 TCR cassette by nested PCR)

### (Materials and Methods)

A test was conducted to check whether an introduced TCR cassette is correctly incorporated into a target by the following protocol.

1. Prepare cell pellets by centrifuging (400 G, 10 minutes, room temperature) 1 × 10⁶ β null Jurkat cells introduced with 1G4 TCR by the method described in Example 1-1 and 1 × 10⁶ β null Jurkat cells used as a control.
2. Extract a genomic DNA from each cell with a QIAamp DNA blood kit (Qiagen).
3. Design a primer as follows so that a junction (junction of 1G4 TCR cassette and TRCA) formed when a 1G4 TCR cassette is transfected into a target site (exon 1 of a TRCA gene) is amplified by nested PCR.
   Primer pair for 1^{st} PCR for amplifying a genetic sequence comprising a junction on the 5' side 5'-CTCTGCATGACTCACTAGCACTCTAT-3' (SEQ ID NO: 24) 5'-AGACCGACACCAACCTGAAC-3' (SEQ ID NO: 25)
   Primer pair for 2^{nd} PCR for amplifying a genetic sequence comprising a junction on the 5' side by using the 1^{st} PCR product as a template DNA 5'-TGGCTAGGAAGGTGGATGAG-3' (SEQ ID NO: 26) 5'-ATCGTGCTGAGAATCCTGCT-3' (SEQ ID NO: 27)
   Primer pair for 1^{st} PCR for amplifying a genetic sequence comprising a junction on the 3' side 5'-CACGGCGACTACTGCACTTA-3' (SEQ ID NO: 28) 5'-GACTGACTTAGTGAGCTGGGAAAGAT-3' (SEQ ID NO: 29)
   Primer pair for 2^{nd} PCR for amplifying a genetic sequence comprising a junction on the 3' side by using the 1^{st} PCR product as a template DNA 5'-GGAGCCAGTACACGACATCA-3' (SEQ ID NO: 30) 5'-TTTGGCAGACAGGGAGAAAT-3' (SEQ ID NO: 31)
4. Amplify genetic sequences comprising junctions on the 5' side and 3' side by nested PCR by using Kapa HiFi HotStart Ready Mix (KAPA Biosystems). The PCR condition settings are the following. 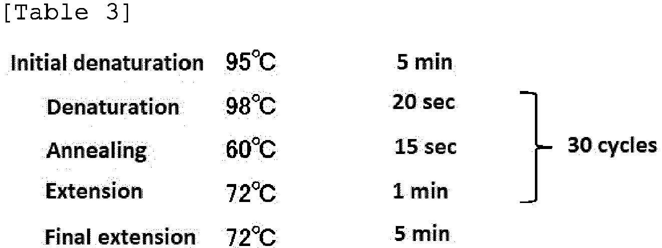
5. Check bands of PCR products by electrophoresis.
6. Check genetic sequences of the PCR products by sequencing.

### (Results)

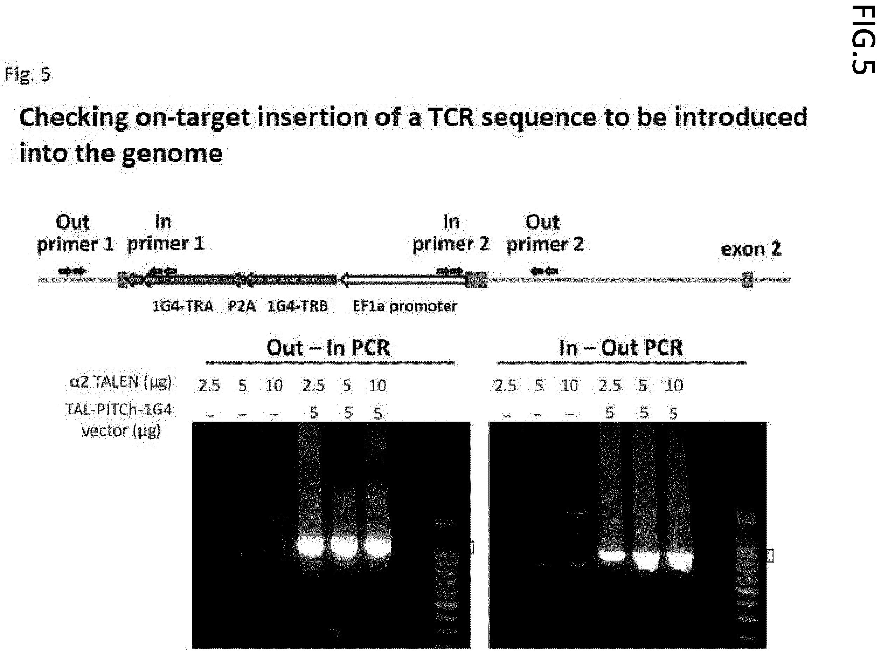

The results are shown in Figure **5.** Figure **5** shows that a sequence for expressing 1G4-TCR contained in a vector is accurately inserted in a region matching exon 1 in the C region of a TRA gene on a genome of 1G4 expressing Jurkat cells by the present method. Out primer 1 and In primer 1 are set in a 5' side untranslated region of exon 1 of C region in an endogenous TRA gene and a TRA coding region of a 1G4-TCR cassette, and the sequence length when accurately inserted is 1006 bp. Meanwhile, In primer 2 and Out primer 2 are set in an EF1a promoter region and within an intron on the downstream side of exon 1 in C region of a TRA gene, and the sequence length when accurately inserted is 742 bp. It is apparent in view of Figure **5** that only one PCR product with an expected length is amplified in each primer combination.

### (Example 2: Checking insertion of accurate number of copies of vector sequences into a desired region using a human T cell strain)

1) 1G4-TCR expressing human Jurkat cells are created and cloned in accordance with a known method by using a conventional retroviral vector (e.g., by a protocol described in Morgan Science 2006; 314: 126-129, Zhao Y, J Immunol 2005; 174: 4415-4423, Robbins PF, CLin Cancer Res 2015; 21: 1019-27).
2) Genome-edited 1G4-TCR expressing Jurkat cells are created using a DNA vector described herein.
3) The full genome sequences of 1G4-TCR expressing Jurkat cells created with a retrovirus in step 1) and 1G4-TCR expressing Jurkat cells created in step 2) are compared using Illumina's Hi-Seq or a next generation sequencer with the same performance to compare the insertion sites and the number of copies of inserted 1G4 sequences. In particular, the presence/absence of an insertion site contained in a database of genes associated with carcinogenesis such as COSMIC is also compared.

### (Example 3: Checking insertion of accurate number of copies of vector sequences into a desired region of a human T cell genome)

1) 1G4-TCR expressing human T cells are created and cloned in accordance with a known method by using a conventional retroviral vector (e.g., by a protocol described in Morgan Science 2006; 314: 126-129, Zhao Y, J Immunol 2005; 174: 4415-4423, Robbins PF, CLin Cancer Res 2015; 21: 1019-27).
2) 1G4-TCR expressing T cells are created using a DNA vector described herein and cloned.
3) The full genome sequences of 1G4-TCR expressing T cells created with a retrovirus in step 1) and 1G4-TCR expressing T cells created in step 2) are compared using Illumina's Hi-Seq or a next generation sequencer with the same performance to compare the insertion sites and the number of copies of inserted 1G4 sequences. In particular, the presence/absence of an insertion site contained in a database of genes associated with carcinogenesis such as COSMIC is also compared.

### (Example 4: Comparison of manufacturing time and cytotoxicity of genome-edited 1G4 expressing T cells created by the present method and 1G4 expressing T cells created with a viral vector)

1) 1G4-TCR expressing human T cells are created in accordance with a known method by using a conventional retroviral vector (e.g. by a protocol described in Morgan Science 2006; 314: 126-129, Zhao Y, J Immunol 2005; 174: 4415-4423, Robbins PF, CLin Cancer Res 2015; 21: 1019-27).
2) 1G4-TCR expressing human T cells are created using the present method.
3) The time (days) required from obtaining a raw material in steps 1) and 2) to creating 1G4-TCR expressing human T cells is compared.
4) Cytotoxicity of two types of 1G4-TCR expressing cells created in steps 1) and 2) are compared in terms of E:T ratio by a cytotoxicity assay described in WO 2019/073964 (mixing effectors (1G4-TCR expressing cells) and targets (epitope peptide added cells) at various ratios and evaluating cell killing activity by a Chromium-51 release assay).

### (Example 5: Flow Electroporation using MaxCyte's transfection apparatus)

The same procedure as Example 1-1 is performed using electroporation that is not Flow Electroporation. The results are compared with results from introduction in Example 1-1 to confirm that the post-introduction cell viability rate and introduction efficiency are higher with introduction in Example 1-1.

### (Example 6: State of cells/timing of electroporation)

After stimulating immune cells with CD3/CD28 beads, the introduction step is performed after passage of various periods of time. Otherwise, the test is in accordance with the procedure of Example 1-1. The introduction efficiency is confirmed to be high with introduction between 48 to 72 hours after stimulation.

Prior to the introduction step, cells are checked with an optical microscope as to whether swelling/clustering, etc. has occurred in the cells. Flow cytometry is used to check whether forward scatter and side scatter values are increased compared to cells in the resting phase. The introduction efficiency is confirmed to be high in introduction into cells, which have cell swelling or clustering and an increased forward scatter or side scatter value compared to cells in the resting phase.

### (Example 7: Synonymous substitution of codon of a TALEN target homologous sequence or a guide RNA that is present on a donor TCR cassette sequence)

Introduction is performed by the same procedure as Example 1-1 when a codon of a TALEN target homologous sequence or a guide RNA that is present on a donor TCR cassette sequence is not synonymously substituted. It is understood that if a codon is not substituted, the TCR sequence to be introduced is cleaved by CRISPR or TALEN, so that a donor TCR would not be expressed.

### (Example 8: Promoter sequence)

Introduction is performed by the same procedure as Example 1-1 with a promoter that is not an EF-1α promoter. The results are compared with results in Example 1-1 using an EF-1α promoter to confirm that the post-introduction expression level and stability of an antigen receptor in cells are higher with introduction in Example 1-1.

### (Example 9: Leader sequencer)

Introduction is performed by the same procedure as Example 1-1 with a vector free of a Xenopus globin leader. The results are compared with results in Example 1-1 using a Xenopus globin leader to confirm that the post-introduction expression level and stability of an antigen receptor in cells are higher with introduction in Example 1-1.

### (Example 10: Experiment for incorporating T cell receptor pair gene into a generic pITCh vector) (Cytomegalovirus (CMV) specific T cell receptor pair)

### (Reagent/equipment)

1. pITCh-EF1α-α2 vector
2. Xba I (Takara Bio)
3. MinElute Reaction Cleanup Kit (Qiagen)
4. NanoDrop 2000 (Thermo Fisher Scientific)
5. NEBuilder HiFi DNA Assembly Master Mix (New England Biolabs)
6. Competent Quick DH5α (Toyobo)
7. LB medium (Sigma-Aldrich)
8. LB agar medium (Sigma-Aldrich)
9. Ampicillin (Sigma-Aldrich)
10. QIAGEN Plasmid Midi Kit (Qiagen)

### (Experimental Method)

### 1. Design/synthesis of artificial DNA of a target gene

When a TCR (T cell receptor) α/β gene pair is incorporated downstream of an EF1α promoter of a generic pITCh vector, inserted DNA is designed in accordance with the following conditions.
*A TCRβ gene and a TCRα gene are linked with a GSGP2A sequence.
*The stop codon of a TCRβ gene is deleted.
*A Kozak sequence is incorporated immediately before a TCRβ gene.
*15 to 25 bp overlap sequences are added before and after the DNA to be inserted.

The designed DNA to be inserted is prepared by a method such as PCR or artificial DNA synthesis. Since artificial DNA synthesis that is currently common is about 1 kbp, a DNA to be inserted may be separated into a plurality of pieces and synthesized (Figure **8****).**

### 2. Incorporation of DNA to be inserted into a generic pITCh vector/plasmid DNA preparation

### (1) Linearization of a generic pITCh vector

A generic pITCh vector is treated with XbaI, whereby the generic pITCh vector is cleaved at a cloning site with an Xba I site and linearized. The generic pITCh vector linearized with Xba I is purified by using a MinElute Reaction Cleanup Kit, and DNA is quantified by using a DNA concentration measuring instrument such as NanoDrop.

### (2) Incorporation of DNA to be inserted into a vector

The linearized generic pITCh vector and DNA to be inserted are connected using a NEBuilder HiFi DNA Assembly Master Mix. The following reaction solution is prepared and incubated for 30 minutes at 50°C.

| | |
|---|---|
| Linearized generic pITCh vector | 50 fmole |
| DNA to be inserted | 100 fmole (twice the amount of vector) |
| NEBuilder HiFi DNA Assembly Master Mix | 10 µL |
| H₂O | Added so that the total would be 20 µL |
| Total | 20µL |

### (3) Large scale preparation of generic pITCh vector with a DNA to be inserted incorporated therein

A generic pITCh vector with a DNA to be inserted incorporated therein is transformed into a DH5α competent cell. Transformed competent cells are seeded in an LB agar medium plate containing ampicillin. A single clone of E. coli that has appeared on the plate is harvested and subjected to shake culture for 16 hours in an LB medium containing ampicillin, and the bacteria are collected. Plasmid DNA is purified from the collected E. coli by using a plasmid purification kit such as QIAGEN Plasmid Midi Kit. The full length DNA sequence of the prepared plasmid is studied to confirm that a TCRα/β pair gene of interest is incorporated into a generic pITCh vector.

5' vector overlap sequence
5'-TAAACGCTCAACTTTGGT-3' (SEQ ID NO: 32)
Kozak sequence
   5'-GCCACC-3'
CMV specific TCRβ sequence 
5’-ATG-stop codon deleted-3’
5’ATGGCACCAGGCTCCTCTGTCGTGTGGCCTTTTGTTTCCTGGCTGTAGGCCTCGTA
GATGTGAAAGTAACCCAGAGCTCGAGATATCTAGTCAAAAGGACGGAGAGAAAGTTTT
TCTGGAATGTGTCCAGGATATGGACCATGAAAATATGTTCTGGTATCGACAAGACCCAG
GTCTGGGGCTACGGCGGATCTATTTCTCATATGATGTTAAAATGAAAGAAAAAGGAGAT
ATTCCTGAGGGGTACAGTGTCTCTAGAGAGAAGAAGGAGCGCTTCTCCCTGATTCTGGA
GTCCGCCAGCACCAACCAGACATCTATGTACCTCTGTGCCAGCAGTTTCCAGGGGTTCA
CTGAAGCTTTCTTTGGACAAGGCACCAGACTCACAGTTGTAGAGGACCTGAACAAGGTG
TTCCCACCCGANGTCGCTGTGTTTGAGCCATCAGAAGCAGANATCTCCCACACCCAAAA
GGCCACACTGGTGTGCCTGGCCACAGGCTTCTTCCCTGACCACGTGGAGCTGAGCTGGT
GGGTGAACGGCAAGGAGGTGCACAGCGGCGTGTGCACCGACCCCCAGCCCCTGAAGGAG
CAGCCCGCCCTGAACGACAGCAGATACTGCCTGAGCAGCAGACTGAGAGTGAGCGCCAC
CTTCTGGCAGAACCCCAGAAACCACTTCAGATGCCAGGTGCAGTTCTACGGCCTGAGCG
AGAACGACGAGTGGACCCAGGACAGAGCCAAGCCCGTGACCCAGATCGTGAGCGCCGAG
GCCTGGGGCAGAGCCGACTGCGGCTTCACCAGCGTGAGCTACCAGCAGGGCGTGCTGAG
CGCCACCATCCTGTACGAGATCCTGCTGGGCAAGGCCACCCTGTACGCCGTGCTGGTGA
GCGCCCTGGTGCTGATGGCCATGGTGAAGAGAAAGGACTTCGGAAGCGGA-3’ (SEQ
ID NO: 33)
GSG P2A sequence 
5’-
GGAAGCGGAGCTACTAACTTCAGCCTGCTGAAGCAGGCTGGAGACGTGGAGGAGAACCC
 TGGACCT-3' (SEQ ID NO: 34)
CMV specific TCRα sequence 
5′-ATG-stop codon-3′
5′ATGGCCTCTGCACCCATCTCGATGCTNGCGATGCTCTTCACATTGAGTGGGCTGAGA
GCTCAGTCAGTGGCTCAGCCGGAAGATCAGGTCAACGTTGCTGAAGGGAATCCTCTGAC
TGTGAAATGCACCTATTCAGTCTCTGGAAACCCTTATCTTTTTGGTATGTTCAATACC
CCAACCGAGGCCTCCAGTTCCTTCTGAAATACATCACAGGGGATAACCTGGTTAAAGGC
AGCTATGGCTTTGAAGCTGAATTTAACAAGAGCCAAACCTCCTTCCACCTGAAGAAACC
ATCTGCCCTTGTGAGCGACTCCGCTTTGTACTTCTGTGCTGACTATTATGGTCAGAATT
TTGTCTTTGGTCCCGGAACCAGATTGTCCGTGCTGCCCTATATCCAGAACCCTGACCCT
GCCGTGTACCAGCTGAGAGACTCTAAATCCAGTGACAAGTCTGTCTGCCTATTCACCGA
CTTCGACAGCCAGACCAACGTGAGCCAGAGCAAGGACAGCGACGTGTACATCACCGACA
AGTGCGTGCTGGACATGAGAAGCATGGACTTCAAGAGCAACAGCGCCGTGGCCTGGAGC
AACAAGAGCGACTTCGCCTGCGCCAACGCCTTCAACAACAGCATCATCCCCGAGGACAC
CTTCTTCCCCAGCCCCGAGAGCAGCTGCGACGTGAAGCTGGTGGAGAAGAGCTTCGAGA
CCGACACCAACCTGAACTTCCAGAACCTGCTGGTGATCGTGCTGAGAATCCTGCTGCTG
AAGGTGGCCGGCTTCAACCTGCTGATGACCCTGAGACTGTGGAGCAGCTGA-3′ (SEQ
ID NO: 35)
3' vector overlap sequence
5'-GCAACTAGAAGGCACAGT-3' (SEQ ID NO: 36)

### (Example 11: Experiment for incorporating a chimeric antigen receptor (CAR) gene into a generic pITCh vector) (Human CD19 specific chimeric antigen receptor)

### (Reagent/equipment)

1. pITCh-EF1α-α2 vector
2. Xba I (Takara Bio)
3. MinElute Reaction Cleanup Kit (Qiagen)
4. NanoDrop 2000 (Thermo Fisher Scientific)
5. NEBuilder HiFi DNA Assembly Master Mix (New England Biolabs)
6. Competent Quick DH5α (Toyobo)
7. LB medium (Sigma-Aldrich)
8. LB agar medium (Sigma-Aldrich)
9. Ampicillin (Sigma-Aldrich)
10. QIAGEN Plasmid Midi Kit (Qiagen)

### (Experimental Method)

### 1. Design/synthesis of artificial DNA of a target gene

When a chimeric antigen receptor (CAR) is incorporated downstream of an EF1α promoter of a generic pITCh vector, DNA to be inserted is designed in accordance with the following conditions.
*A Kozak sequence is incorporated immediately before a CAR gene.
*15 to 25 bp vector overlap sequences are added before and after the DNA to be inserted.

The designed DNA to be inserted is prepared by a method such as PCR or artificial DNA synthesis (Figure **9****).**

### 2. Incorporation of DNA to be inserted into a generic pITCh vector/plasmid DNA preparation

### (1) Linearization of a generic pITCh vector

A generic pITCh vector is treated with XbaI, whereby the generic pITCh vector is cleaved at a cloning site with an Xba I site and linearized. The generic pITCh vector linearized with Xba I is purified by using a MinElute Reaction Cleanup Kit, and DNA is quantified by using a DNA concentration measuring instrument such as NanoDrop.

### (2) Incorporation of DNA to be inserted into a vector

The linearized generic pITCh vector and DNA to be inserted are connected using a NEBuilder HiFi DNA Assembly Master Mix. The following reaction solution is prepared and incubated for 30 minutes at 50°C.

| | |
|---|---|
| Linearized generic pITCh vector | 50 fmole |
| DNA to be inserted | 100 fmole (twice the |
| | amount of vector) |
| NEBuilder HiFi DNA Assembly Master Mix | 10 µL |
| H₂O | Added so that the total would be 20 µL |
| Total | 20µL |

### (3) Large scale preparation of generic pITCh vector with a DNA to be inserted incorporated therein

A generic pITCh vector with a DNA to be inserted incorporated therein is transformed into a DH5α competent cell. Transformed competent cells are seeded in an LB agar medium plate containing ampicillin. A single clone of E. coli that has appeared on the plate is harvested and subjected to shake culture for 16 hours in an LB medium containing ampicillin, and the bacteria are collected. Plasmid DNA is purified from the collected E. coli by using a plasmid purification kit such as QIAGEN Plasmid Midi Kit. The full length DNA sequence of the prepared plasmid is studied to confirm that a chimeric antigen receptor gene of interest is incorporated into a generic pITCh vector.

5' vector overlap sequence
5'-TAAACGCTCAACTTTGGT-3' (SEQ ID NO: 37)
Kozak sequence
   5'-GCCACC-3'
Human CD19 specific chimeric antigen receptor sequence 
5′-
ATGACCCAGACCACAAGCAGCCTGTCTGCCAGCCTGGGCGATAGAGTGACCATCAGCTG
TAGAGCCAGCCAGGACATCAGCAAGTACCTGAACTGGTATCAGCAGAAACCCGACGGCA
CCGTGAAGCTGCTGATCTACCACACCAGCAGACTGCACAGCGGCGTGCCAAGCAGATTT
TCTGGCAGCGGCTCTGGCACCGACTACAGCCTGACAATCAGCAACCTGGAACAAGAGGA
TATCGCTACCTACTTCTGCCAGCAAGGCAACACCCTGCCTTACACCTTTGGCGGAGGCA
CCAAGCTGGAAATCACCGGCTCTACAAGCGGCAGCGGCAAACCTGGATCTGGCGAGGGA
 
TCTACCAAGGGCGAAGTGAAACTGCAAGAGTCTGGCCCTGGACTGGTGGCCCCATCTCA
GTCTCTGAGCGTGACCTGTACAGTCAGCGGAGTGTCCCTGCCTGATTACGGCGTGTCCT
GGATCAGACAGCCTCCTCGGAAAGGCCTGGAATGGCTGGGAGTGATCTGGGGCAGCGAG
ACAACCTACTACAACAGCGCCCTGAAGTCCCGGCTGACCATCATCAAGGACAACTCCAA
GAGCCAGGTGTTCCTGAAGATGAACAGCCTGCAGACCGACGACACCGCCATCTACTATT
GCGCCAAGCACTACTACTACGGCGGCAGCTACGCCATGGATTATTGGGGCCAGGGCACC
AGCGTGACCGTTTCTTCTACCACGACGCCAGCGCCGCGACCACCAACACCGGCGCCCAC
CATCGCGTCGCAGCCCCTGTCCCTGCGCCCAGAAGCGTGCCGGCCAGCGGCGGGGGGCG
CAGTGCACACGAGGGGGCTGGACTTCGCCTGTGATTTCTGGGTGCTGGTCGTTGTGGGC
GGCGTGCTGGCCTGCTACAGCCTGCTGGTGACAGTGGCCTTCATCATCTTTTGGGTGAG
GAGCAAGCGGAGCAGACTGCTGCACAGCGACTACATGAACATGACCCCCCGGAGGCCTG
GCCCCACCCGGAAGCACTACCAGCCCTACGCCCCTCCCAGGGATTTCGCCGCCTACCGG
AGCAAACGGGGCAGAAAGAAACTCCTGTATATATTCAAACAACCATTTATGAGACCAGT
ACAAACTACTCAAGAGGAAGATGGCTGTAGCTGCCGATTTCCAGAAGAAGAAGAAGGAG
GATGTGAACTGAGAGTGAAGTTCAGCAGGAGCGCAGACGCCCCCGCGTACAAGCAGGGC
CAGAACCAGCTCTATAACGAGCTCAATCTAGGACGAAGAGAGGAGTACGATGTTTTGGA
CAAGAGACGTGGCCGGGACCCTGAGATGGGGGGAAAGCCGAGAAGGAAGAACCCTCAGG
AAGGCCTGTACAATGAACTGCAGAAAGATAAGATGGCGGAGGCCTACAGTGAGATTGGG
ATGAAAGGCGAGCGCCGGAGGGGCAAGGGGCACGATGGCCTTTACCAGGGTCTCAGTAC
AGCCACCAAGGACACCTACGACGCCCTTCACATGCAGGCCCTGCCCCCTCGCTAA-3'
(SEQ ID NO: 38)
3' vector overlap sequence
   5'-GCAACTAGAAGGCACAGT-3' (SEQ ID NO: 39)

### (Example 12: Experiment for introducing a chimeric antigen receptor gene (CAR) into a T cell derived from a healthy individual by using a generic pITCh vector)

### (Experimental Method)

### 1. Separation of CD3 positive cells from peripheral blood of a healthy individual

### (Reagent/equipment)

1. RPMI 1640 (Thermo Fisher Scientific)
2. Density gradient medium, Lymphoprep (Abbott Diagnostics Technologies AS)
3. PBS (pH7.2)
4. MACS Buffer (Miltenyi Biotec)
5. CD3 MicroBeads (Miltenyi Biotec)
6. MACS column (Miltenyi Biotec)
7. MACS separator (Miltenyi Biotec)
8. Hemocytometer

(1) On the day of the T cell separation experiment, about 50 mL of whole blood is collected from a healthy individual. The collected whole blood of a healthy individual is diluted with an equal amount of RPMI 1640, and the mixture is stored at 37°C.
(2) 15 mL of a density gradient medium, Lymphoprep, is added to a 50 mL centrifuge tube, and 30 mL of the diluted whole blood is layered onto the density gradient medium. A plurality of samples is prepared by this step to use the entire diluted whole blood.
(3) The centrifuge tubes filled with the density gradient medium and whole blood are centrifuged for 30 minutes under room temperature at 800 x g.
(4) 15 mL of the intermediate layer comprising white blood cells formed by the centrifugation is collected in a centrifuge tube by using a Pasteur pipette.
(5) PBS (pH 7.2) is added to the collected intermediate layer until the total amount reaches 14 mL. The centrifuge tube is gently vortexed to suspend the cells.
(6) The cell suspension is centrifuged for 10 minutes at 4°C at 250 x g.
(7) The suspension is checked to confirm that cell pellets are formed by the centrifugation. The supernatant is removed while leaving 1 mL. The centrifuge tube is gently vortexed to suspend the cells. The entire sample is collected in a single 15 mL centrifuge tube, and PBS (pH 7.2) is added to the collected intermediate layer until the total amount reaches 14 mL. The centrifuge tube is gently vortexed to suspend the cells.
(8) The cell suspension is centrifuged for 10 minutes at 4°C at 250 x g.
(9) The supernatant is removed while leaving 2 mL. The centrifuge tube is gently vortexed to suspend the cells.
(10) The cells are counted by using a hemocytometer.
(11) The cell suspension is centrifuged for 10 minutes at 4°C at 300 x g. The supernatant is completely removed.
(12) MACS buffer is added at 80 µL per 1 × 10⁷ cells.
(13) CD3 MicroBeads are added at 20 µL per 1 × 10⁷ cells.
(14) The cell suspension is thoroughly stirred and incubated for 15 minutes at 4 to 8°C.
(15) MACS buffer is added at 1 to 2 mL per 1 × 10⁷ cells. The cell suspension is gently stirred, and the cells are washed.
(16) The cell suspension is centrifuged for 10 minutes at 4°C at 300 x g. The supernatant is completely removed.
(17) MACS buffer is added at 500 µL per 1 × 10⁸ cells. The centrifuge tube is gently vortexed to suspend the cells.
(18) A MACS column is set in a MACS separator.
(19) The MACS column is rinsed with MACS buffer.
(20) The cell suspension is added to the column.
(21) Unlabeled cells that pass through the MACS column are collected. Subsequently, the column is washed three times with MACS buffer.
(22) The MACS column is detached from the MACS separator and set in a collection tube.
(23) 5 mL of MACS buffer is added to the MACS column, and a plunger is set in the MACS column.
(24) The cells retained in the column are collected in a 15 mL centrifuge tube by firmly pushing the plunger. The collected CD3 positive cells are subsequently used in activation of T cells using CD3/CD28 immobilized beads.

### 2. Activation of T cells with CD3/CD28 immobilized beads (Reagent/equipment)

1. CTL-medium (X-VIVO 20 (Lonza) with 10 % AB serum (Gibco), 2 mmol/l L-glutamine (Gibco), and 1% penicillin/streptomycin (Sigma)
2. Dynabeads Human T-Activator CD3/CD28 (Thermo Fisher Scientific)
3. Magnetic stand
4. Human IL-2 (R&D Systems)

(1) 1 mL of CTL-medium is fractionated into a 50 mL centrifuge tube for 1 × 10⁶ CD3 positive cells and warmed to 37°C.
(2) The collected CD3 positive cells are centrifuged for 10 minutes at 4°C at 300 x g. The supernatant is completely removed. The cells are preserved on ice.
(3) 25 µL of suspended Dynabeads Human T-Activator CD3/CD28 is fractionated into a 1.5 mL microtube for 1 × 10⁶ CD3 positive cells.
(4) An amount equal to the fractionated beads or at least 1 mL of PBS is added. The mixture is vortexted to suspend the cells for 5 seconds.
(5) The microtube is set on the magnetic stand and left standing for 1 minute or longer. The beads are separated, and the supernatant is removed.
(6) A CTL-medium at an amount equal to the beads fractionated at first is added to the beads to suspend the beads.
(7) The suspended beads are added to the CTL-medium warmed to 37°C.
(8) Human IL-2 is added to the CTL-medium warned to 37°C to reach 30 U/mL.
(9) The CTL-medium added with beads and human IL-2 is added to the CD3 positive cell preserved on ice so that the concentration would be 1 × 10⁶ cells/mL. The cells are gently stirred and suspended.
(10) The suspended CD3 positive cells are seeded on a 24 well plate at 1 mL/well.
(11) The seeded cells are cultured for 72 hours under the condition of 37°C, 5% CO₂.

### 2. Knockdown of TCRβ

### (Reagent/equipment)

1. Hemocytometer
2. Magnetic stand
3. Opti-MEM (Thermo Fisher Scientific)
4. TALEN RNA (β3-TALEN-L/β3-TALEN-R)
5. CTL-medium
6. Human IL-2
7. NEPA21 electroporator (Nepa Gene)
8. Cuvette electrode (Nepa Gene)
9. Cuvette electrode chamber (Nepa Gene)
10. CD3 MicroBeads (Miltenyi Biotec)
11. MACS column (Miltenyi Biotec)

(1) The cells are counted with a hemocytometer by using a magnetic stand from CD3 positive cells cultured for 72 hours with CD3/CD28 beads.
(2) The CD3 positive cells cultured and stimulated with CD3/CD28 beads for 72 hours were transferred to a 1.5 mL microtube, which is set on a magnetic stand. The cell suspension is left standing for 1 minute or longer to separate the CD3/CD28 beads. The supernatant is collected in a new 15 mL centrifuge tube.
(3) The cell suspension is centrifuged for 5 minutes at 300 x g. The supernatant is removed.
(4) Opti-MEM is added at 1 mL/tube. The tube is gently vortexed to wash the cells.
(5) The cell suspension is centrifuged for 5 minutes at 300 x g. The supernatant is removed.
(6) A small amount of Opti-MEM is added to suspend the cells, and the cells are counted by using a hemocytometer.
(7) Opti-MEM is added to achieve 1 × 10⁶ cells/90 µL.
(8) TALEN-RNA of β3-TALEN-L and β3-TALEN-R is each dispensed in a 1.5 mL microtube at 10 µg/tube.
(9) The suspended cells are added to 90 µL/tube of Opti-MEM and gently stirred.
(10) A cell/RNA suspension is added to cuvette electrodes at 100 µL each.
(11) The resistance of each cuvette electrode is measured. The resistance is adjusted to be within the range of 0.043 to 0.047. If the resistance is low, 2 to 3 µL of the cell/RNA suspension is removed from the cuvette electrodes. If the resistance is high, 2 to 3 µL of Opti-MEM is added to the cuvette electrodes.
(12) A CTL-medium including 30 U/mL of human IL-2 is dispensed in a 24-well plate at 1 mL/well in the same number of wells as samples. The plate to which the medium has been dispensed is warmed in a CO₂ incubator.
(13) The cuvette electrodes are gently tapped, and the suspension is stirred. The cuvette electrodes are set in the cuvette electrode chamber.
(14) The parameters of a NEPA21 electroporator are set as follows.
   Poring Pulse: 275V (2.5 msec -50 msec) x2, 50% Transfer Pulse: 20V (50 msec -50 msec) x5, 40%
(15) The resistance of the cuvette electrodes is measured, and the Start button is pressed for electroporation.
(16) The electroporated cell suspension is added to the medium in the warmed 24-well plate by using a pipette attached to the cuvette electrodes.
(17) The seeded cells are cultured for 72 hours under the condition of 37°C, 5% CO₂.
(18) CD3 negative cells are separated from the cells cultured for 72 hours by using CD3 MicroBeads and MACS column.
(19) The separated CD3 negative cells are seeded in a CTL-medium comprising 30 U/mL of human IL-2.
(20) The seeded cells are cultured for 48 hours under the condition of 37°C, 5% CO₂.

### 3. Knockdown of TCRα and introduction of CAR by TALEN-PITCh (Reagent/equipment)

1. CTL-medium
2. Dynabeads Human T-Activator CD3/CD28 (Thermo Fisher Scientific)
3. Human IL-2
4. Hemocytometer
5. Magnetic stand
6. Opti-MEM (Thermo Fisher Scientific)
7. TALEN RNA (α2-TALEN-L/α2-TALEN-R)
8. NEPA21 electroporator (Nepa Gene)
9. Cuvette electrode (Nepa Gene)
10. Cuvette electrode chamber (Nepa Gene)

(1) CD3 negative cells cultured for 48 hours are transferred to a CTL-medium comprising CD3/CD28 beads/30 U/mL human IL-2. The seeded cells are cultured for 72 hours under the condition of 37°C, 5% CO₂.
(2) TALEN-RNA of α2-TALEN-L and α2-TALEN-R at 10 µg/tube each, and 5 ug/tube of pITCh-EF1α-α2-FMC63 are introduced to CD3 negative cells cultured for 72 hours by electroporation. The method is the same as "2. Knockdown of TCRβ".
(3) The electroporated cells are seeded in a CTL-medium comprising 30 U/mL of human IL-2. The seeded cells are cultured for 72 hours under the condition of 37°C, 5% CO₂.
(4) FMC64-CAR positive cells are analyzed by FACSCant II.

### (Example 13: Introduction of 1G4 into a TRB deficient Jurkat cell by using a modified vector)

10 µg of TALEN mRNA for cleaving TRAC and 5 µg of PITCh vector for introducing 1G4 were prepared and introduced into β-null Jurkat cells. The cells were cultured, and subjected to FACS analysis by using a NY-ESO-1 MHC tetramer to check the 1G4 expression after 72 hours.

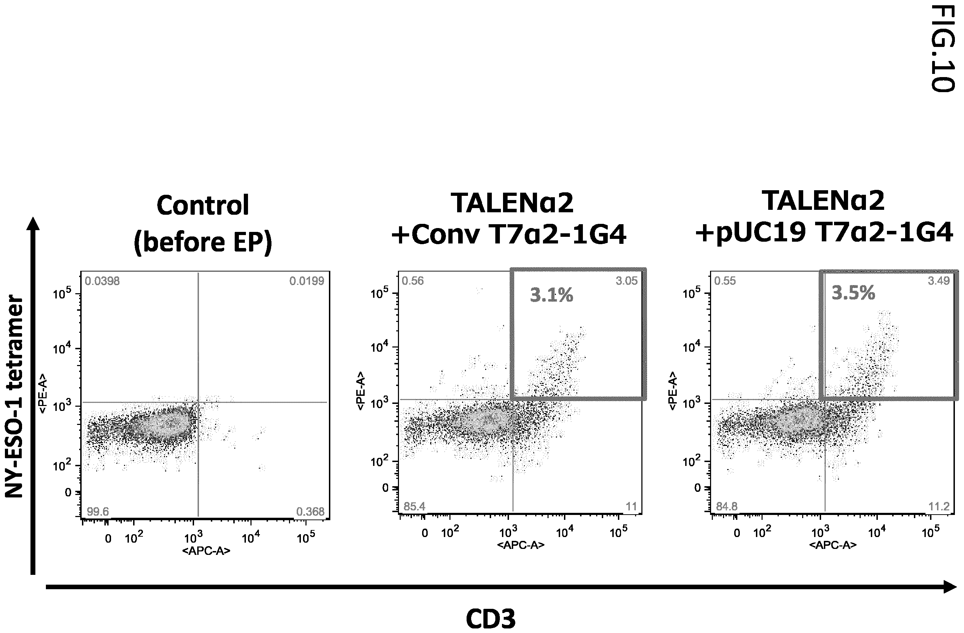

The results are shown in Figure **10****.** In Figure **10****,** Conv indicates a conventional PITCh vector, and pUC19 indicates a modified PITCh vector. As shown in Figure **10****,** the 1G4 expression efficiencies, when using a conventional PITCh vector and when using a modified PITCh vector, are 3.1% and 3.5%, respectively. It can be understood that 1G4 can be introduced, and the expression thereof was successful by using either vector.

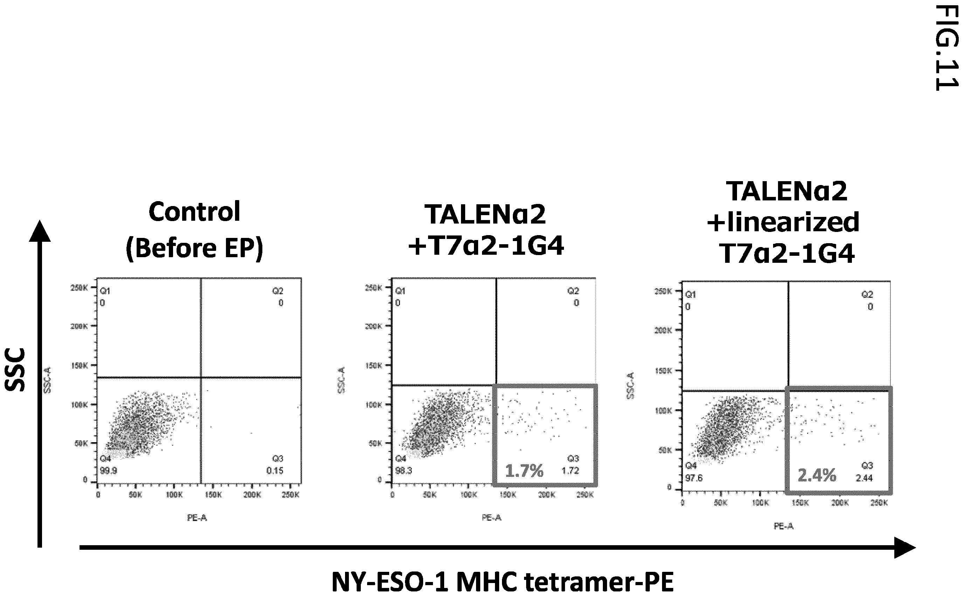

TALEN-RNA and a modified PITCh vector were prepared and introduced into β-null Jurkat cells by electroporation. The cells were cultured, and subjected to FACS analysis by using an NY-ESO-1 MHC tetramer to check the 1G4 expression after 72 hours. The results thereof are shown in Figure **11****.** As can be understood therefrom, introduction of a modified PITCh vector can be confirmed, so that expression of 1G4 is successful.

### (Example 14: Creation of 1G4-TCR-T from T cells of healthy individuals)

Experimental Method:

### (Reagent/equipment)

1. CD3(+) PBMC
2. Magnetic stand
3. 0.4 w/v% trypan blue solution
4. Opti-MEM (Gibco, 31985-070)
5. NEPA21 (Nepa Gene)
6. Cuvette electrode chamber (Nepa Gene, CU500)
7. Cuvette electrode (Nepa Gene, EC-002S)
8. TALEN RNA (α2-TALEN-L/R, β3-TALEN-L/R)
9. 1G4 PITCh vector
10. 1G4 fragment
11. Hemocytometer
12. Growth medium CTL-M (30 units/mL human IL-2)
13. IL-2 (50,000 IU/mL)
14. CD3 MicroBeads (Miltenyi Biotec, #130-050-101)
15. MACS Column
16. MACS Buffer (4°C)

### (Procedure)

### 1. KO of TRB

(1-1) CD3(+) PBMCs stimulated/cultured with CD3/CD28 beads are counted by using a hemocytometer (culture time: 72 hours).
(1-2) Cultured cells used in electroporation are transferred to a 1.5 mL tube, which is set on a magnetic stand. The CD3/CD28 beads are removed, and the supernatant is collected in a new 15 mL tube.
(1-3) Centrifugation at 300 x g for 5 min.

The supernatant is removed as much as possible by using a Pipetman.
(1-4) 1 mL/tube of Opti-MEM is added, and the tubes are vortexed to wash the cells. Centrifugation at 300 x g for 5 min.
(1-5) A Pipetman is used to remove the supernatant as much as possible.
(1-6) Number of electroporation conditions x 100 µL of Opti-MEM is added, and the cells are counted using a hemocytometer.
(1-7) Opti-MEM is added and adjusted to achieve 1 × 10⁶ cells/90 µL.
(1-8) TALEN RNA is dispensed in 1.5 mL tubes at 10 µg/tube.
(1-9) Cells suspended in 90 pL/tube of Opti-MEM are added and gently stirred.
(1-10) Cuvette electrodes are numbered, and a cell/RNA suspension is added at 100 µL/well.
(1-11) The resistance of each cuvette electrode is measured.
(1-12) The resistance of all cuvettes is adjusted to around 0.045 (0.043 to 0.047).

If the resistance is low, 2 to 3 µL of the solution is removed from the cuvette electrodes.

If the resistance is high, 2 to 3 µL of Opti-MEM is added to the cuvettes.
(1-13) Before starting electroporation, 1 mL/well of CTL-M (30 units/mL human IL-2) is dispensed into the same number of wells as the number of conditions in a 24-well plate and warmed in a CO² incubator.
(1-14) The cuvette electrodes are gently tapped and stirred, and set in a cuvette electrode chamber.
(1-15) Parameter setting
Poring Pulse: 275V (2.5 msec - 50 msec) x2, 50%
Transfer Pulse: 20V (50 msec - 50 msec) x5, 40%
(1-16) The resistance is measured, and the Start button is pressed for electroporation.
(1-17) The electroporated cell solution is added to the medium in the warmed 24-well plate by using a pipette attached to the cuvettes.
(1-18) Cells that are not electroporated are added to the medium in the 24-well plate as a control.
(1-19) The cells are cultured for 72 hours in 5% CO₂ at 37°C.
(1-20) A CD3 negative fraction is separated by using CD3 MicroBeads (Miltenyi Biotec, #130-050-101).
(1-21) The cells are cultured for 48 hours in 5% CO₂ at 37°C in CTL-M (30 units/mL human IL-2).

### 2. KO of TRA and introduction of 1G4 TCR by TAL-PITCh (2-1) Cells are stimulated/cultured with CD3/CD28 beads (culture time: 72 hours).

(2-2) 10 µg/tube of α2-TALEN RNA and 5 µg/tube of 1G4 PITCh vector are introduced into the cultured cells by electroporation. The method is the same as 1.
(2-3) The cells are cultured for 72 hours in 5% CO₂ at 37°C in CTL-M (30 units/mL human IL-2).
(2-4) The cells are analyzed by FACSCanto II.

### 3. Expansion culture

(3-1) Cells are stimulated with CD3/CD28 beads.
(3-2) The cells are cultured for 9 days in 5% CO₂ at 37°C in CTL-M (30 units/mL human IL-2).
(3-3) Cells are stimulated for the second time with CD3/CD28 beads.
(3-4) The cells are cultured for 9 days in 5% CO₂ at 37°C in CTL-M (30 units/mL human IL-2).
(3-5) The cells are analyzed by FACSCanto II.

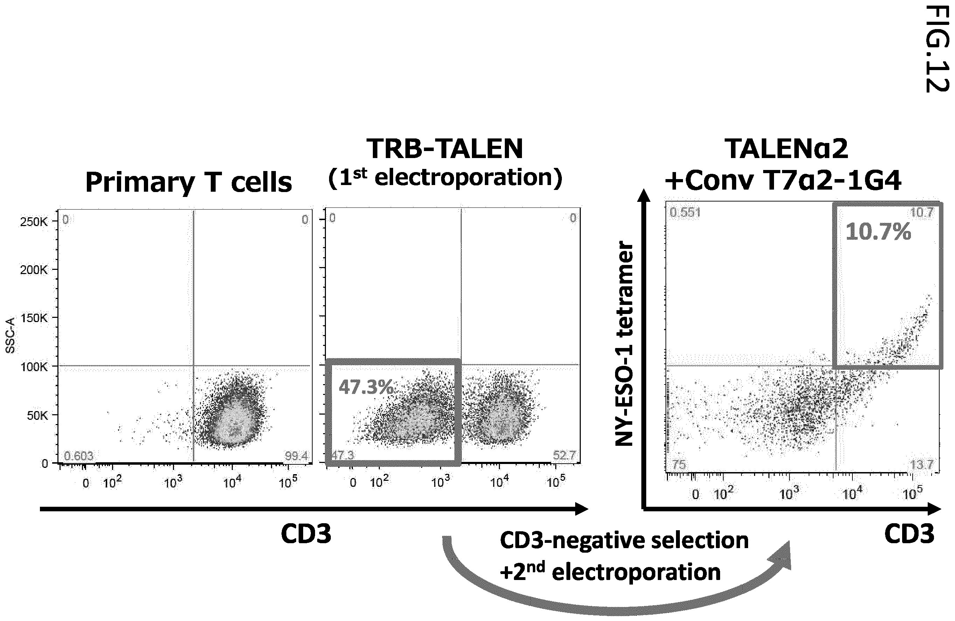

The results of introducing 1G4 when using human T cells are shown in Figure **12****.** The first electroporation knocked out TRB, and the second electroporation knocked out TRA, and 1G4 TCR is introduced by TAL-PITCh. As shown in Figure **12****,** the efficiency of 1G4 expression is 10.7% when using human T cells. It can be confirmed that a PITCh vector is introduced, and it can be understood that 1G4 is successfully expressed.

### (Note)

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein. The present application claims priority to Japanese Patent Application No. 2019-206448 filed on November 14, 2019 with the Japan Patent Office, and the entire content thereof is incorporated herein by reference in the same manner as if the contents are specifically described herein.

### [Industrial Applicability]

The present disclosure relates to biotechnological manipulation of immune cells. The present disclosure particularly relates to a technology for introducing a desired antigen receptor into an immune cell. The present disclosure can be utilized in the field of applied life science/clinical medicine. In particular, the present disclosure is understood to be useful for the preparation of modified cells for administration into a human and applicable for the treatment of autoimmune diseases or cancer.

### [Sequence Listing Free Text]

SEQ ID NO: 1: full length sequence of a PITCh_1G4 vector
SEQ ID NO: 2: full length sequence of a modified PITCh_1G4 vector
SEQ ID NO: 3: full length sequence of a modified PITCh_NLV48 vector
SEQ ID NO: 4, 5: backbone sequence of a PITCh_1G4 vector
SEQ ID NO: 6, 7: backbone sequence of a modified PITCh_1G4 vector
SEQ ID NO: 8, 9: backbone sequence of a modified PITCh_NLV48 vector
SEQ ID NO: 10 to 15: nuclease recognition sequence
SEQ ID NO: 16 to 18: introduction cassette partial sequence
SEQ ID NO: 19: polyA addition sequence
SEQ ID NO: 20: P2A sequence
SEQ ID NO: 21: Xenopus globin 5'-UTR sequence
SEQ ID NO: 22: T7 promoter sequence
SEQ ID NO: 23: EF-1α promoter sequence
SEQ ID NO: 24 to 31: promoter sequence used in the Examples
SEQ ID NO: 32: 5' vector overlap sequence used in Example 10
SEQ ID NO: 33: CMV specific TCRβ sequence
SEQ ID NO: 34: GSGP2A sequence
SEQ ID NO: 35: CMV specific TCRα sequence
SEQ ID NO: 36: 3' vector overlap sequence used in Example 10
SEQ ID NO: 37: 5' vector overlap sequence used in Example 11
SEQ ID NO: 38: human CD19 specific chimeric antigen receptor sequence
SEQ ID NO: 39: 3' vector overlap sequence used in Example 11

## Claims

1. A method of introducing a nucleic acid encoding an antigen receptor into a given site in a nucleic acid contained in an immune cell of a target organism, comprising the step of:
introducing a vector comprising the nucleic acid encoding the antigen receptor and a nuclease into the immune cell;
wherein the nuclease cleaves the vector and a genome sequence of the immune cell, and
wherein the vector is configured to produce, when cleaved by the nuclease, a sequence encoding the antigen receptor, which is introduced into a portion cleaved by the nuclease of the genome sequence of the immune cell by microhomology-mediated end joining (MMEJ).

2. The method of claim 1, wherein the nuclease cleaves a locus of an endogenous antigen receptor of the immune cell.

3. The method of claim 1, wherein knockout of an endogenous antigen receptor gene of the immune cell occurs concurrently with introduction of the antigen receptor.

4. The method of claim 2, wherein the endogenous antigen receptor gene is a TRA gene.

5. The method of claim 2, wherein the endogenous antigen receptor gene is a TRB gene.

6. The method of any one of claims 1 to 5, wherein the nucleic acid encoding the antigen receptor encodes an α chain and a β chain of the antigen receptor.

7. The method of any one of claims 1 to 6, wherein the introducing step is performed using electroporation.

8. The method of any one of claims 1 to 7, wherein the introducing step is performed between about 48 and about 72 hours after stimulating an immune cell with CD3/CD28 beads.

9. The method of any one of claims 1 to 8, wherein the antigen receptor is selected from the group consisting of a T cell receptor (TCR), a B cell receptor (BCR), and a chimeric antigen receptor (CAR).

10. The method of any one of claims 1 to 9, wherein the nuclease cleaves inside of a constant region of a TRA gene.

11. The method of any one of claims 1 to 9, wherein the nuclease cleaves inside of a constant region of a TRB gene.

12. The method of any one of claims 1 to 11, wherein the nuclease is a Cas9 nuclease.

13. The method of any one of claims 1 to 11, wherein the nuclease is a homodimer nuclease, and the vector is a circular vector.

14. The method of any one of claims 1 to 11 and 13, wherein the nuclease is a TALEN.

15. The method of claim 14, wherein the nuclease is a Platinum TALEN.

16. The method of claim 14 or 15, wherein
the nuclease comprises a DNA binding domain,
the DNA binding domain comprises a plurality of DNA binding modules consecutively from the N-terminus side,
a combination of the xth amino acid and the yth amino acid in the 4n-3th DNA binding module from the N-terminus being identical for any n,
a combination of the xth amino acid and the yth amino acid in the 4n-2th DNA binding module from the N-terminus being identical for any n,
a combination of the xth amino acid and the yth amino acid in the 4n-1th DNA binding module from the N-terminus being identical for any n, and
a combination of the xth amino acid and the yth amino acid in the 4nth DNA binding module from the N-terminus being identical for any n,
the combination of the xth amino acid and the yth amino acid in the 4n-3th DNA binding module from the N-terminus, the combination of the xth amino acid and the yth amino acid in the 4n-2th DNA binding module from the N-terminus, the combination of the xth amino acid and the yth amino acid in the 4n-1th DNA binding module from the N-terminus, and the combination of the xth amino acid and the yth amino acid in the 4nth DNA binding module from the N-terminus are different from one another, and
n is a natural number from 1 to 10, x is a natural number from 1 to 40, y is a natural number from 1 to 40, and x and y are different natural numbers.

17. The method of any one of claims 1 to 16, wherein
the vector produces a nucleic acid fragment comprising a region consisting of a first nucleotide sequence, the desired nucleic acid, and a region consisting of a second nucleotide sequence, in order, in a direction from the 5' end to the 3' end by the nuclease,
the nucleic acid contained in the cell comprises a region consisting of a first nucleotide sequence, the given site, and a region consisting of a second nucleotide sequence, in order, in a direction from the 5' end to the 3' end, and
the first nucleotide sequence in the nucleic acid contained in the cell and the first nucleotide sequence in the vector are linked by microhomology-mediated end joining, and the second nucleotide sequence in the nucleic acid contained in the cell and the second nucleotide sequence in the vector are linked by microhomology-mediated end joining, whereby the desired nucleic acid is inserted.

18. The method of any one of claims 1 to 17, wherein the vector comprises a plurality of introduction cassettes comprising the nucleic acid encoding the antigen receptor.

19. A vector for inserting a nucleic acid encoding an antigen receptor at a given site in a nucleic acid contained in a cell in combination with a nuclease, comprising:
an introduction cassette comprising the nucleic acid encoding the antigen receptor; and
a microhomology sequence for producing microhomology-mediated end joining (MMEJ) for introducing a sequence encoding the antigen receptor into a site of cleavage by the nuclease of a genome sequence of the cell;
wherein the vector produces a nucleic acid fragment comprising a region consisting of a first nucleotide sequence, the nucleic acid encoding the antigen receptor, and a region consisting of a second nucleotide sequence, in order, in a direction from the 5' end to the 3' end by the nuclease, and
the first nucleotide sequence in the nucleic acid contained in the cell and the first nucleotide sequence in the vector are linked by microhomology-mediated end joining, and the second nucleotide sequence in the nucleic acid contained in the cell and the second nucleotide sequence in the vector are linked by microhomology-mediated end joining, whereby the sequence encoding the antigen receptor is inserted.

20. The vector of claim 19, wherein
the given site is present in an endogenous antigen receptor gene of the cell, and
the nucleic acid encoding the antigen receptor comprises a synonymous substitution in a sequence corresponding to a targeted site by the nuclease contained in the cell.

21. The vector of claim 19, wherein the nucleic acid encoding the antigen receptor comprises an EF-1α promoter that is operably linked to the antigen receptor.

22. The vector of claim 19, wherein the nucleic acid encoding the antigen receptor comprises a leader sequence for enhancing translation efficiency upstream of the antigen receptor.

23. The vector of claim 22, wherein the leader sequence is a 5'-UTR sequence of a β globin gene.

24. The vector of claim 22, wherein the leader sequence is a Xenopus globin 5'-UTR.

25. The vector of any one of claims 19 to 24, wherein the nucleic acid encoding the antigen receptor encodes an α chain and a β chain of the antigen receptor.

26. The vector of any one of claims 19 to 25, wherein the antigen receptor is selected from the group consisting of a T cell receptor (TCR), a B cell receptor (BCR), and a chimeric antigen receptor (CAR).

27. The vector of any one of claims 19 to 26, wherein the desired site is inside of a constant region of a TRA gene.

28. The vector of any one of claims 19 to 26, wherein the desired site is inside of a constant region of a TRB gene.

29. The vector of any one of claims 19 to 28, wherein the nuclease is a Cas9 nuclease.

30. The vector of any one of claims 19 to 28, wherein the nuclease is a homodimer nuclease, and the vector is a circular vector.

31. The vector of any one of claims 19 to 28 and 30, wherein the nuclease is a TALEN.

32. The vector of claim 31, wherein the nuclease is a Platinum TALEN.

33. The vector of claim 30 or 32, wherein
the nuclease comprises a DNA binding domain,
the DNA binding domain comprises a plurality of DNA binding modules consecutively from the N-terminus side,
a combination of the xth amino acid and the yth amino acid in the 4n-3th DNA binding module from the N-terminus being identical for any n,
a combination of the xth amino acid and the yth amino acid in the 4n-2th DNA binding module from the N-terminus being identical for any n,
a combination of the xth amino acid and the yth amino acid in the 4n-1th DNA binding module from the N-terminus being identical for any n, and
a combination of the xth amino acid and the yth amino acid in the 4nth DNA binding module from the N-terminus being identical for any n,
the combination of the xth amino acid and the yth amino acid in the 4n-3th DNA binding module from the N-terminus, the combination of the xth amino acid and the yth amino acid in the 4n-2th DNA binding module from the N-terminus, the combination of the xth amino acid and the yth amino acid in the 4n-1th DNA binding module from the N-terminus, and the combination of the xth amino acid and the yth amino acid in the 4nth DNA binding module from the N-terminus are different from one another, and
n is a natural number from 1 to 10, x is a natural number from 1 to 40, y is a natural number from 1 to 40, and x and y are different natural numbers.

34. The vector of any one of claims 19 to 33, wherein the vector comprises a nucleotide sequence recognized by a gRNA target sequence or a first DNA binding domain on the 5' side, a region consisting of a first nucleotide sequence, the desired nucleic acid, a second nucleotide sequence, and a region consisting of a nucleotide sequence recognized by a gRNA target sequence or a second DNA binding domain on the 3' side, in order, in a direction from the 5' end to the 3' end.

35. The vector of any one of claims 19 to 34, wherein the first nucleotide sequence is 3 to 10 bases long, and/or the second nucleotide sequence is 3 to 10 bases long.

36. The vector of any one of claims 19 to 35, wherein the vector comprises a plurality of introduction cassettes comprising the nucleic acid encoding the antigen receptor.

37. A vector comprising:
a nucleic acid of SEQ ID NO: 4 and a nucleic acid of SEQ ID NO: 5, a nucleic acid of SEQ ID NO: 6 and a nucleic acid of SEQ ID NO: 7, or a nucleic acid of SEQ ID NO: 8 and a nucleic acid of SEQ ID NO: 9,
a nucleic acid of SEQ ID NO: 10 and a nucleic acid of SEQ ID NO: 11, a nucleic acid of SEQ ID NO: 12 and a nucleic acid of SEQ ID NO: 13, or a nucleic acid of SEQ ID NO: 14 and a nucleic acid of SEQ ID NO: 15, and
an introduction cassette comprising a nucleic acid encoding an antigen receptor.

38. The vector of claim 37, wherein the introduction cassette comprises a sequence encoding an α chain of an antigen receptor and a sequence encoding a β chain of an antigen receptor.

39. The vector of claim 37 or 38, wherein the introduction cassette comprises a polyA addition sequence, a leader sequence, and/or one or more promoter sequences.

40. The vector of any one of claims 37 to 39, wherein the introduction cassette comprises a P2A sequence between the sequence encoding an α chain of an antigen receptor and the sequence encoding a β chain of an antigen receptor.

41. The vector of any one of claims 37 to 40, wherein the vector comprises a plurality of introduction cassettes.

42. A kit for inserting a nucleic acid encoding an antigen receptor into a given site in a nucleic acid contained in a cell, comprising the vector of any one of claims 19 to 35 and a vector for expressing the nuclease.

43. A method of inserting a nucleic acid encoding an antigen receptor into a given site in a nucleic acid contained in an immune cell in vitro or in vivo into a non-human organism, comprising the step of introducing the vector of any one of claims 19 to 35 and a vector for expressing the nuclease into an immune cell.

44. A method of manufacturing an immune cell that expresses an antigen receptor, comprising the step of inserting a nucleic acid encoding the antigen receptor into a given site in a nucleic acid contained in the immune cell by the method of claim 43.

45. A method of administering immunotherapy to a subject, comprising the steps of:
(A) obtaining blood from the subject;
(B) knocking out a TRB of mononuclear cells of the blood (PBMC);
(C) concentrating the mononuclear cells for CD3 negative;
(D) knocking out a TRA of the mononuclear cells and introducing an antigen receptor;
(E) concentrating the mononuclear cells for CD3 positive; and
(G) optionally subjecting the mononuclear cells to expansion culture.

46. A method of manufacturing a cell used in immunotherapy, comprising the steps of:
(A) knocking out a TRB of mononuclear cells of blood (PBMC) obtained from a subject;
(B) concentrating the mononuclear cells for CD3 negative;
(C) knocking out a TRA of the mononuclear cells and introducing an antigen receptor;
(D) concentrating the mononuclear cells for CD3 positive; and
(E) optionally subjecting the mononuclear cells to expansion culture.

47. The method of claim 45 or 46, wherein the TRA and/or TRB is cleaved by using a Platinum TALEN.

48. The method of claim 45 or 46, wherein the immunotherapy comprises TCR-T therapy or CAR-T therapy.

49. A composition for use in the method of claim 45 or 46, comprising a Platinum TALEN.
